# EUROPEAN PATENT SPECIFICATION

(11) **EP 2 661 447 B1**
(45) Date of publication and mention of the grant of the patent: **23.09.2015**
(21) Application number: 12702771.2
(22) Date of filing: 06.01.2012
(51) Int. Cl.: C07K 16/24, C07K 14/00

(54) **ALPHABODIES SPECIFICALLY BINDING TO CYTOKINES OR GROWTH FACTORS AND/OR CYTOKINE OR GROWTH FACTOR RECEPTORS**
ALPHAKÖRPER ZUR SPEZIFISCHEN BINDUNG AN ZYTOKINE ODER WACHSTUMSFAKTOREN UND/ODER CYTOKIN- ODER WACHSTUMSFAKTOR-REZEPTOREN
ALPHA-CORPS SE LIANT SPÉCIFIQUEMENT À DES CYTOKINES OU À DES FACTEURS DE CROISSANCE ET/OU À DES RÉCEPTEURS DE CYTOKINES OU DE FACTEURS DE CROISSANCE

(30) Priority: 06.01.2011 WO PCT/EP2011/050138
(43) Date of publication of application: 13.11.2013
(73) Proprietor: Complix N.V., 3590 Diepenbeek (BE)
(72) Inventor: LASTERS, Ignace, B-2018 Antwerpen (BE); DESMET, Johan, B-8500 Kortrijk (BE); HENDERIKX, Maria, B-3500 Hasselt (BE); WEHNERT, Anita, B-3631 Boorsem - Maasmechelen (BE); MEERSSEMAN, Geert, B-1000 Brussel (BE)
(74) Representative: Paemen, Liesbet R.J.
(86) International application number: PCT/EP2012/050193
(87) International publication number: WO 2012/093172

(56) References cited:
- EP-A1- 2 161 278
- WO-A1-2010/066740
- Y. CHEN ET AL.: "Anti-IL-23 therapy inhibits multiple inflammatory pathways and ameliorates autoimmune encephalomyelitis.", THE JOURNAL OF CLINICAL INVESTIGATION, vol. 116, no. 5, May 2006 (2006-05), pages 1317-1326, XP002583205, U.S.A. cited in the application
- O. PILOTO ET AL.: "IMC-EB10, an anti-FLT3 monoclonal antibody, prolongs survival and reduces nonobese diabetic/severe combined immunodeficient engraftment of some acute lymphoblastic leukemia cell lines and primary leukemic samples.", CANCER RESEARCH, vol. 66, no. 9, 1 May 2006 (2006-05-01), pages 4843-4851, XP002548251, U.S.A.

## Description

### FIELD OF THE INVENTION

The present invention relates to the field of binding agents to cytokines and growth factors and/or to their receptorsand uses thereof for prophylactic, therapeutic or diagnostic purposes as well as in screening and detection.

### BACKGROUND

Growth factors are naturally occurring substances capable of stimulating cellular growth, proliferation and cellular differentiation. Cytokines are considered to be a sub-class of growth factors which function as cell-signaling protein molecules. For many growth factors the characterization as 'cytokine' or 'hormone' is debated by biochemists in the art.

Cytokines encompass a diverse group of small proteins that mediate cell signalling and communication. The major pro-inflammatory cytokines that are responsible for early responses are IL-1-alpha, IL-1-beta, IL-6, and TNF-alpha. Other pro-inflammatory mediators include LIF, IFN-gamma, OSM, CNTF, TGF-beta, GM-CSF, IL-11, IL-12, IL-23, IL-17, IL-18, IL-8 and a variety of chemotactic cytokines (chemokines) that chemoattract inflammatory cells. These cytokines either act as endogenous pyrogens (IL-1, IL-6, TNF-alpha), upregulate the synthesis of secondary mediators and pro-inflammatory cytokines by both macrophages and mesenchymal cells (including fibroblasts, epithelial and endothelial cells), stimulate the production of acute phase proteins, or attract inflammatory cells. The anti-inflammatory cytokines control the proinflammatory cytokine response. Major antiinflammatory cytokines include interleukin (IL)-1 receptor antagonist, IL-4, IL-6, IL-10, IL-11, and IL-13. Specific cytokine receptors for IL-1, tumor necrosis factor-α, and IL-18 also function as proinflammatory cytokine inhibitors. The net effect of an inflammatory response is determined by the balance between pro-inflammatory and anti-inflammatory cytokines.

The traditional cytokines can be subdivided into several groups, including the immune/hematopoietins, interferons, tumor necrosis factor (TNF)-related molecules, and the chemokines. They exert their biological functions through specific receptors expressed on the surface of target cells. These receptors can be grouped into families according to both structural and amino acid sequence similarities. The cytokine receptor superfamily is composed of the receptors for many growth factor families including interferon, TNF and haematopoietic growth factors. The largest subclass in this family is that of the type I cytokine receptors, a group characterized by the presence of a conserved extracellular region of approximately 200 amino acids containing two fibronectin type III folds. This region, known as the haematopoietin receptor module, has been shown to play an essential role in receptor/ligand binding and receptor/receptor dimerization. It is characterized by four conserved cysteine residues in the first domain and a W-S-x-W-S motif in the second domain. This canonical W-S-x-W-S motif is lacking in the class II cytokine receptors.

A number of other growth factors are often referred to as cytokines in the art because of their functional and structural overlap with cytokines. For instance, tyrosine kinase receptor ligands, such as Flt3 ligand are often classified as short chain cytokines. Flt3 ligand forms a dimer similar to the M-CSF and CSF dimers. It plays an important role in hematopoietic stem/progenitor cell survival and proliferation. Because it acts on a membrane-bound receptor it can be considered to be involved in cell signalling.

Receptor chains of the type I cytokine receptors typically form part of a multicomponent complex which includes both ligand binding and signalling subunits, of which the latter is typically a member of several receptor complexes. These characteristics account for much of the pleiotropy and redundancy amongst cytokines.

Cytokines or growth factors can be monomeric (e.g. G-CSF, IL-1alpha, Il-1beta, IL-2, IL-3, IL-4, IL-6), homodimeric (e.g. IL-5, IFN-gamma, IL-10, Flt3 ligand), or heterodimeric (e.g. IL-12, IL-23).

A well characterized family of cytokines is the superfamily of heterodimeric cytokines including IL-12, IL-23, IL-27, CLC-sCNTFR, CLC-CLF-1 and the newly identified IL-35, all of which bind heterodimeric receptors. Heterodimeric cytokines consist of two different subunits. One of these subunits comprises a four-helix bundle domain. IL-23, for example, consists of the p40 subunit, which is shared with the cytokine IL-12, and the p19 or IL-23 alpha subunit which has a 4-helix bundle structure. Also for IL-17 heterodimeric cytokine assemblies have been demonstrated, e.g. IL-17F/IL-17A is a heterodimeric cytokine (Wright et al., The Journal of Immunology, 2008, 181: 2799-2805).

The receptors of heterodimeric cytokines also consist of two or more different subunits. The IL-12 receptor, for instance, is a heterodimer of IL-12Rbeta1 and IL-12Rbeta2 and the IL-23 receptor is a heterodimer of IL-12Rbeta1 and IL-23R. IL-12Rbeta1 is used by both IL-12 and IL-23 for signalling. Targeting this receptor will lead to a blockade of both IL-12 and IL-23 signaling. IL23 activates the same signaling molecules as IL-12: JAK2, TYK2, and STAT1, STAT3, STAT4, and STAT5. STAT4 activation is substantially weaker and different DNA-binding STAT complexes form in response to IL-23 compared with IL12. IL-23R associates constitutively with JAK2 and in a ligand-dependent manner with STAT3.

Heterodimeric cytokines or growth factors often have subunits which are common to different cytokines (e.g. p40 is common to IL-12 and IL-23). This may limit the specificity of binding agents raised against these cytokines, especially in cases where the binding agent recognizes a subunit which is common to different cytokines. Also of particular importance is that sometimes an isolated subunit of a heterodimeric cytokine is found in body fluids. For example in the rheumatoid synovium, abundant expression of the interleukin (IL)23 p19 subunit has been observed (Brentano et al., Ann Rheum Dis 2008;68:143-150). Hence, if it is desired that the binding agent recognizes such subunit only in the heterodimeric context, it may be advantageous to endow this binding agent with a binding property which ensures specificity for the heterodimeric cytokine. This can be accomplished by a binding agent that recognizes simultaneously both subunits of the given heterodimeric cytokine while showing little affinity for the individual subunits. Conversely, it may be desired that the binding agent only recognizes a given subunit outside the heterodimeric context (e.g. in cases where it is desired to 'mop-up' abundantly expressed isolated cytokine subunits). This can be accomplished by e.g. a binding agent which shows affinity for the given individual subunit (e.g. IL-23(p19)) but which is sterically hindered by the other subunit (e.g. IL-23(p40)) for binding to said individual subunit in the heterodimeric context.

IL-23 plays an important role in the inflammatory response against infection. It promotes upregulation of the matrix metalloprotease MMP9, increases angiogenesis and reduces CD8+ T-cell infiltration. Recently, IL-23 has been implicated in the development of cancerous tumors. In conjunction with IL-6 and TGF-β1, IL-23 stimulates naive CD4+ T cells to differentiate into a novel subset of cells called Th17 cells, which are distinct from the classical Th1 and Th2 cells. Th17 cells produce IL-17, a proinflammatory cytokine that enhances T cell priming and stimulates the production of proinflammatory molecules such as IL-1, IL-6, TNF-alpha, NOS-2, and chemokines resulting in inflammation.

It has been shown that IL-23 is an important mediator of organ specific autoimmune diseases (Yen et al., 2006). Knockout mice deficient in either p40 or p19, or in either subunit of the IL-23 receptor (IL-23R and IL12R-β1) develop less severe symptoms of multiple sclerosis and inflammatory bowel disease. In addition, it has been demonstrated that anti-IL-23(p19) specific antibodies can inhibit EAE, a preclinical animal model of human MS (Chen et al., 2006).

The secretion of IL-12 by activated dendritic cells and phagocytes leads to a Th1 response with interferon-gamma production together with an enhanced cytotoxic, antitumor and antimicrobial response. Consequently, anti-IL12 antibodies may interfere with the Th1 pathway leading to susceptibility for infection.

Piloto et al. (2006; Cancer Research 66 (9): 4843-4851) discloses that human neutralizing monoclonal antibody IMC-EB10 against Flt3 reduces leukemic engraftment and may be useful in the treatment of acute lymphoblastic leukemia (ALL).

The therapeutic antibodies Ustekinumab (previously also known under the experimental name CNTO 1275 and now approved for the treatment of plaque psoriasis) and Briakinumab (in phase III clinical trial for the treatment of plaque psoriasis and in phase II trial for MS) are human monoclonal antibodies that neutralize both IL-12 as well as IL-23. There is a concern about adverse effects, more in particular the risk for infections, resulting from the IL23/IL-12 cross-reactivity. While Lima et al. (2009) indicated that no cases of tuberculosis or other opportunistic infections were reported in clinical studies using Ustekinumab, the medication guide for STELARA™ states that 'Some people have serious infections while taking STELARA™, including tuberculosis (TB), and infections caused by bacteria, fungi, or viruses. Some people have to be hospitalized for treatment of their infection'. In addition, the side effects reported for Ustekinumab at the Drug.com web site (Drug Information Online) show up to 5% upper respiratory tract infections.

Based on the above, it is clear that growth factors and more particularly cytokines and their receptors constitute attractive targets for the development of pharmaceuticals for the prevention or treatment of the diseases or disorders in which they are involved. More particularly, for heterodimeric cytokines or growth factors a compound blocking specifically one subunit of the cytokine may be advantageous over a compound that does not discriminate between cytokines sharing common subunits.

Moreover, there remains a need for alternative and/or improved active compounds or agents that can be used for the prevention or treatment of cytokine or growth factor or receptor-mediated diseases and/or disorders and, hence, for the efficient binding of cytokines or growth factors by such active compounds.

WO2010/066740 describes Alphabody scaffolds as single-chain triple-stranded alpha-helical coiled coil scaffolds. However, it has not been disclosed how these Alphabody scaffolds can be manipulated to obtain Alphabodies specifically binding to targets with sufficient affinity in order to modulate the biological mechanisms in which these targets are involved.

### SUMMARY OF THE INVENTION

The present inventors have found methods which allow the generation of Alphabody polypeptides which specifically bind to a target of interest. It has been found that using the Alphabody scaffold, binders can be generated which bind to a target of interest with high affinity and specificity, such that binders can be generated which overcome one or more of the disadvantages of the prior art binders. Moreover it has been found that such binders have several advantages over the traditional (immunoglobulin and non-immunoglobulin) binding agents known in the art. Such advantages include, without limitation, the fact that they are compact and small in size (between 10 and 14 kDa, which is 10 times smaller than an antibody), they are extremely stable (having a melting temperature of more than 100 °C), they can be made resistant to different proteases, they are highly engineerable (in the sense that multiple substitutions will generally not obliterate their correct and stable folding), and have a structure which is based on natural motifs which have been redesigned via protein engineering techniques.

The present invention provides Alphabody polypeptides comprising an Alphabody (as defined herein, and also referred to as Alphabodies of the invention) that specifically bind to cytokines or growth factors and/or their receptors, as well as polypeptides that comprise or essentially consist of one or more such Alphabodies and to uses of such Alphabodies or polypeptides for prophylactic, therapeutic or diagnostic purposes.

In one aspect, the present invention provides Alphabody polypeptides which specifically bind to a cytokine or growth factor and/or to a cytokine or growth factor receptor.

In particular embodiments, the Alphabody polypeptides of the invention, specifically bind to a cytokine or growth factor, and more particularly to the receptor binding site on that cytokine or growth factor. In particular embodiments, the Alphabody polypeptides of the invention that bind to a cytokine or growth factor inhibit the interaction between that cytokine or growth factor and its cytokine or growth factor receptor(s).

In particular embodiments, the cytokine or growth factor is a heterodimeric cytokine or growth factor. In further particular embodiments, the cytokine or growth factor is a heterodimeric cytokine or growth factor, and the Alphabody is directed to a cytokine-specific subunit of the cytokine or a growth factor-specific subunit of the growth factor. In further particular embodiments, the cytokine is of the family of IL-12 cytokines. Most particularly the cytokine is IL-23.

In specific embodiments of the present invention, the cytokine to which the Alphabody polypeptides of the present invention specifically bind is a heterodimeric cytokine, such as for example but not limited to IL-12, IL-23, IL-27 or IL-35. In certain particular embodiments, the Alphabody polypeptides of the present invention specifically bind to IL-23, and more particularly to the p19-subunit of IL-23, to the p40 subunit of IL-23 or to both the p19 and the p40 subunit of IL-23. In certain particular embodiments, the Alphabody polypeptides of the present invention specifically bind to the p19 subunit of IL-23 and do not bind to the p40 subunit of IL-23. Alphabody polypeptides of the present invention that specifically bind to a cytokine are, in particular embodiments, amino acid sequences having at least 80% sequence identity with at least one of the amino acid sequences corresponding to any one of SEQ_ID1 to SEQ_ID127.

In further particular embodiments of the present invention, the cytokine or growth factor to which the Alphabody polypeptides of the present invention specifically bind is a class III receptor tyrosine kinase (RTK) ligand and/or its receptor, such as Flt3 ligand (Flt3L) or Flt3 receptor (Flt3R). In certain particular embodiments, the Alphabody polypeptides of the invention substantially interact with a single monomer of the Flt3L dimer, for example a human flt3L (hflt3L) dimer. Alphabody polypeptides of the present invention that specifically bind to a cytokine or growth factor are, in particular embodiments, amino acid sequences having the specific hFlt3L-binding residues presented in Table 5 herein, and/or having at least 80% sequence identity with at least one of the amino acid sequences corresponding to SEQ_ID134 or SEQ_ID135.

In other particular embodiments, the Alphabody polypeptides of the invention specifically bind to a cytokine or growth factor receptor, and more particularly to the cytokine or growth factor binding site on the cytokine or growth factor receptor. In particular embodiments, the Alphabody polypeptides of the invention that bind to a cytokine or growth factor receptor inhibit the interaction between that cytokine or growth factor receptor and its cytokine or growth factor ligand.

Examples of cytokine or growth factor receptors to which the Alphabody polypeptides of the present invention may specifically bind can be chosen from the group consisting of, but are not limited to, type 1 interleukin receptor, erythropoietin receptor, GM-CSF receptor, G-CSF receptor, oncostatin M receptor, leukemia inhibitory factor receptor, type II interleukin receptors, interferon-alpha/beta receptor, interferon-gamma receptor, interleukin-1 receptor, CSF1, C-kit receptor, interleukin-18 receptor, CD27, CD30, CD120, CD40, lymphotoxin beta receptor, interleukin-8 receptor, CCR1, CXCR4, MCAF receptor, NAP-2 receptor, CC chemokine receptors, CXC chemokine receptors, CX3C chemokine receptors, XC chemokine receptor (XCR1), TGF beta receptor 1 and TGF beta receptor 2.

In specific embodiments of the present invention, the cytokine or growth factor receptor to which the Alphabodies of the present invention specifically bind is a receptor for a heterodimeric cytokine or growth factor, such as for example but not limited to IL-12 receptor, the IL-23 receptor, the IL-27 receptor or the IL-35 receptor.

According to certain particular embodiments, the Alphabody polypeptides of the invention bind both to a cytokine or growth factor and to a cytokine or growth factor receptor; the Alphabody polypeptides in such embodiments are said to be 'bispecific'.

Thus according to a particular aspect, the present invention provides Alphabody polypeptides that comprise only one single Alphabody or that consist of only one single Alphabody, which specifically bind to a cytokine or growth factor or to a cytokine or growth factor receptor. Alternatively, the polypeptides of the present invention comprise more than one Alphabody which are interconnected in one polypeptide chain.

Thus the present invention provides Alphabody polypeptides that comprise or essentially consist of one or more Alphabodies of the present invention that specifically bind to a cytokine or growth factor and/or to a cytokine or growth factor receptor (also referred to herein as polypeptides of the invention). In further embodiments, Alphabody polypeptides comprise one or more Alphabodies of the present invention and optionally one or more further groups, optionally linked via one or more linking sequences.

In a further aspect, the present invention provides nucleic acid sequences encoding the Alphabody polypeptides (such as Alphabodies) or the polypeptides of the invention (also referred to herein as nucleic acid sequences of the invention).

In another further aspect, the present invention provides vectors comprising one or more nucleic acid sequences of the invention.

In still a further aspect, the present invention provides hosts or host cells that express or are capable of expressing one or more Alphabody polypeptides of the invention.

In yet a further aspect, the present invention provides pharmaceutical compositions comprising one or more Alphabody polypeptides and/or nucleic acid sequences according to the invention and optionally at least one pharmaceutically acceptable carrier (also referred to herein as pharmaceutical compositions of the invention). According to certain particular embodiments, the pharmaceutical compositions of the invention may further optionally comprise at least one other pharmaceutically active compound.

In another aspect, the present invention provides methods for the production of the Alphabody polypeptides or pharmaceutical compositions of the invention that specifically bind to a cytokine or growth factor and/or a cytokine or growth factor receptor, the methods at least comprising the steps of:
(i) expressing, in a suitable host cell or expression system, one or more Alphabody polypeptides of the invention, and
ii) isolating and/or purifying the Alphabody polypeptides of the invention.

According to particular embodiments, the methods for the production of the Alphabody polypeptides or pharmaceutical compositions of the invention that specifically bind to a cytokine or growth factor and/or a cytokine or growth factor receptor may at least comprise the steps of:
a) producing a nucleic acid or vector library encoding a single-chain Alphabody library comprising at least 100 different-sequence single-chain Alphabody polypeptides, wherein the Alphabody polypeptides differ from each other in at least one of a defined set of 5 to 20 variegated amino acid residue positions, and wherein at least 70% of the variegated amino acid residue positions are located either:
   (i) at heptad e-positions in a first alpha-helix of the Alphabody and at heptad g-positions in a second alpha-helix, parallel to said first alpha-helix, and optionally at heptad b-positions in said first alpha-helix of the Alphabody and/or at heptad c-positions in said second alpha-helix of the Alphabody, or
   (ii) at heptad b-, c- and f-positions in one alpha-helix of the Alphabody, or
   (iii) at positions in a linker fragment connecting two consecutive alpha-helices of the Alphabody,
b) introducing the nucleic acid or vector library into host cells and culturing the host cells in a medium under conditions suitable for the production of the single-chain Alphabody library,
c) optionally isolating the single-chain Alphabody library produced in step b) from the host cells and/or from the medium,
d) contacting the cytokine or growth factor, or cytokine or growth factor receptor, with the single-chain Alphabody library produced in step b or isolated in step c), and
e) isolating from the single-chain Alphabody library being contacted with the cytokine or growth factor, or cytokine or growth factor receptor, of interest in step d), the one or more single-chain Alphabodies having detectable binding affinity for, or a detectable in vitro effect on the activity of, the cytokine or growth factor and/or the cytokine or growth factor receptor.

In particular embodiments, the Alphabody library is further enriched for single-chain Alphabodies having detectable binding affinity for, or detectable in vitro effect on the activity of, the cytokine or growth factor and/or cytokine or growth factor receptor by iterative execution of steps d) and e), optionally supplemented with an amplification step following the isolation step d). In further particular embodiments, the method comprises the step of determining the amino acid sequence of one or more of the single-chain Alphabodies obtained in step e). In particular embodiments, the method comprises the step of synthesizing one or more of the single-chain Alphabodies obtained in step e).

According to a further aspect, the present invention provides the use of Alphabodies or polypeptides of the invention that specifically bind to a cytokine or growth factor and/or a cytokine or growth factor receptor for the preparation of a medicament for the prevention and/or treatment of at least one cytokine- or growth factor- mediated disease and/or disorder in which said cytokine or growth factor and/or said cytokine or growth factor receptor are involved. Accordingly, the invention provides Alphabodies, polypeptides and pharmaceutical compositions specifically binding to a cytokine or growth factor and/or a cytokine or growth factor receptor for use in the prevention and/or treatment of at least one cytokine/growth factor-mediated disease and/or disorder in which said cytokine or growth factor and/or said cytokine or growth factor receptor are involved. In particular embodiments, the present invention also provides methods for the prevention and/or treatment of at least one cytokine/growth factor-mediated disease and/or disorder, comprising administering to a subject in need thereof, a pharmaceutically active amount of one or more Alphabodies, polypeptides and/or pharmaceutical compositions of the invention.

Cytokine- or growth factor-mediated diseases and/or disorders in which a cytokine or growth factor and/or a cytokine or growth factor receptor are involved can be chosen from the group consisting of, but are not limited to inflammatory and/or autoimmune diseases and disorders. More particularly, the diseases and/or disorders are selected from inflammation and inflammatory disorders such as but not limited to bowel diseases (colitis, Crohn's disease, IBD), infectious diseases, psoriasis, and other autoimmune diseases (such as rheumatoid arthritis, Multiple Sclerosis, Spondyloarthritis, Sarcoidosis, Lupus, Behcet's disease), transplant rejection, cystic fibrosis, asthma, chronic obstructive pulmonary disease, cancer, viral infection, common variable immunodeficiency.

### DETAILED DESCRIPTION OF THE INVENTION

### [DEFINITIONS]

### [GENERAL DEFINITIONS]

As used herein, the singular forms 'a', 'an', and 'the' include both singular and plural referents unless the context clearly dictates otherwise.

The terms 'comprising', 'comprises' and 'comprised of as used herein are synonymous with 'including', 'includes' or 'containing', 'contains', and are inclusive or openended and do not exclude additional, non-recited members, elements or method steps.

The recitation of numerical ranges by endpoints includes all numbers and fractions subsumed within the respective ranges, as well as the recited endpoints.

The term 'about' as used herein when referring to a measurable value such as a parameter, an amount, a temporal duration, and the like, is meant to encompass variations of +/-10% or less, preferably +/-5% or less, more preferably +/-1 % or less, and still more preferably +/-0.1% or less of and from the specified value, insofar such variations are appropriate to perform in the disclosed invention. It is to be understood that the value to which the modifier 'about' refers is itself also specifically, and preferably, disclosed.

As used herein, an 'Alphabody (of the invention)' or 'Alphabodies (of the invention)' can generally be defined as self-folded, single-chain, triple-stranded, predominantly alpha-helical, coiled coil amino acid sequences, polypeptides or proteins. More particularly, Alphabodies as used in the context of the present invention can be defined as amino acid sequences, polypeptides or proteins having the general formula HRS1-L1-HRS2-L2-HRS3, wherein
- each of HRS1, HRS2 and HRS3 is independently a heptad repeat sequence (HRS) consisting of 2 to 7 consecutive heptad repeat units, at least 50% of all heptad a- and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
- each of L1 and L2 are independently a linker fragment, as further defined hereinafter, which covalently connect HRS1 to HRS2 and HRS2 to HRS3, respectively.

As used herein, a 'parallel Alphabody' shall have the meaning of an Alphabody (of the invention) wherein the alpha-helices of the triple-stranded, alpha-helical, coiled coil structure together form a parallel coiled coil structure, i.e., a coiled coil wherein all three alpha-helices are parallel.

As used herein, an 'antiparallel Alphabody' shall have the meaning of an Alphabody (of the invention) wherein the alpha-helices of the triple-stranded, alpha-helical, coiled coil structure together form an antiparallel coiled coil structure, i.e., a coiled coil wherein two alpha-helices are parallel and the third alpha-helix is antiparallel with respect to these two helices.

As will become clear from the further description herein, the invention also envisages polypeptides comprising a sequence with the general formula HRS1-L1-HRS2-L2-HRS3, but which in certain particular embodiments comprise further groups, moieties and/or residues, which are covalently linked, more particularly N- and/or C-terminal covalently linked, to a basic Alphabody structure having the formula HRS1-L1-HRS2-L2-HRS3. Thus reference is made herein generally to 'Alphabody polypeptides' which comprise or consist of an Alphabody according to the invention. The binding features described for an Alphabody herein can generally also be applied to Alphabody polypeptides comprising said Alphabody. The Alphabody polypeptides of the present invention are however characterized by the presence of at least one triple-helix structure (consisting of three helixes) which as such forms a coiled coil.

The terms 'heptad', 'heptad unit' or 'heptad repeat unit' are used interchangeably herein and shall herein have the meaning of a 7-residue (poly)peptide fragment that is repeated two or more times within each heptad repeat sequence of an Alphabody, polypeptide or composition of the invention and is represented as 'abcdefg' or 'defgabc', wherein the symbols 'a' to 'g' denote conventional heptad positions. Conventional heptad positions are assigned to specific amino acid residues within a heptad, a heptad unit, or a heptad repeat unit, present in an Alphabody, polypeptide or composition of the invention, for example, by using specialized software such as the COILS method of Lupas et al. (Science 1991, 252:1162-1164; http://www.russell.embl-heidelberg.de/cgi-bin/coils-svr.pl). However, it is noted that the heptads or heptad units as present in the Alphabodies of the invention (or polypeptides and compositions of the invention comprising these Alphabodies) are not strictly limited to the above-cited representations (i.e. 'abcdefg' or 'defgabc') as will become clear from the further description herein and in their broadest sense constitute a 7-residue (poly)peptide fragment per se, comprising at least assignable heptad positions a and d.

The terms 'heptad a-positions', 'heptad b-positions', 'heptad c-positions', 'heptad d-positions', 'heptad e-positions', 'heptad f-positions' and 'heptad g-positions' refer respectively to the conventional 'a', 'b', 'c', 'd', 'e', 'f and 'g' amino acid positions in a heptad, heptad repeat or heptad repeat unit of an Alphabody, polypeptide or composition of the invention.

A 'heptad motif as used herein shall have the meaning of a 7-residue (poly)peptide pattern. A 'heptad motif of the type 'abcdefg' can usually be represented as 'HPPHPPP', whereas a 'heptad motif of the type 'defgabc' can usually represented as 'HPPPHPP', wherein the symbol 'H' denotes an apolar or hydrophobic amino acid residue and the symbol 'P' denotes a polar or hydrophilic amino acid residue. However, it is noted that the heptad motifs as present in the Alphabodies of the invention (or polypeptides and compositions of the invention comprising these Alphabodies) are not strictly limited to the above-cited representations (i.e. 'abcdefg', 'HPPHPPP', 'defgabc' and 'HPPPHPP') as will become clear from the further description herein.

A 'heptad repeat sequence' ('HRS') as used herein shall have the meaning of an amino acid sequence or sequence fragment consisting of n consecutive heptads, where n is a number equal to or greater than 2.

In the context of the single-chain structure of the Alphabodies (as defined herein) the terms 'linker', 'linker fragment' or 'linker sequence' are used interchangeably herein and refer to an amino acid sequence fragment that is part of the contiguous amino acid sequence of a single-chain Alphabody, and which covalently interconnect the HRS sequences of that Alphabody.

In the context of the present invention, a 'coiled coil' or 'coiled coil structure' shall be used interchangeably herein and will be clear to the person skilled in the art based on the common general knowledge and the description and further references cited herein. Particular reference in this regard is made to review papers concerning coiled coil structures, such as for example, Cohen and Parry Proteins 1990, 7:1-15; Kohn and Hodges Trends Biotechnol 1998, 16:379-389; Schneider et al Fold Des 1998, 3:R29-R40; Harbury et al. Science 1998, 282:1462-1467; Mason and Arndt ChemBioChem 2004, 5:170-176; Lupas and Gruber Adv Protein Chem 2005, 70:37-78; Woolfson Adv Protein Chem 2005, 70:79-112; Parry et al. J Struct Biol 2008, 163:258-269; McFarlane et al. Eur J Pharmacol 2009:625:101-107.

An 'alpha-helical part of an Alphabody' shall herein have the meaning of that part of an Alphabody which has an alpha-helical secondary structure. Furthermore, any part of the full part of an Alphabody having an alpha-helical secondary structure is also considered an alpha-helical part of an Alphabody. More particularly, in the context of a binding site, where one or more amino acids located in an alpha-helical part of the Alphabody contribute to the binding site, the binding site is considered to be formed by an alpha-helical part of the Alphabody.

A 'solvent-oriented' or 'solvent-exposed' region of an alpha-helix of an Alphabody shall herein have the meaning of that part on an Alphabody which is directly exposed or which comes directly into contact with the solvent, environment, surroundings or milieu in which it is present. Furthermore, any part of the full part of an Alphabody which is directly exposed or which comes directly into contact with the solvent is also considered a solvent-oriented or solvent-exposed region of an Alphabody. More particularly, in the context of a binding site, where one or more amino acids located in a solvent-oriented part of the Alphabody contribute to the binding site, the binding site is considered to be formed by a solvent-oriented part of the Alphabody.

The term 'groove of an Alphabody' shall herein have the meaning of that part on an Alphabody which corresponds to the concave, groove-like local shape, which is formed by any pair of spatially adjacent alpha-helices within an Alphabody.

As used herein, amino acid residues will be indicated either by their full name or according to the standard three-letter or one-letter amino acid code.

As used herein, the term 'homology' denotes at least secondary structural similarity between two macromolecules, particularly between two polypeptides or polynucleotides, from same or different taxons, wherein said similarity is due to shared ancestry. Hence, the term 'homologues' denotes so-related macromolecules having said secondary and optionally tertiary structural similarity. For comparing two or more nucleotide sequences, the '(percentage of) sequence identity' between a first nucleotide sequence and a second nucleotide sequence may be calculated using methods known by the person skilled in the art, e.g. by dividing the number of nucleotides in the first nucleotide sequence that are identical to the nucleotides at the corresponding positions in the second nucleotide sequence by the total number of nucleotides in the first nucleotide sequence and multiplying by 100% or by using a known computer algorithm for sequence alignment such as NCBI Blast. In determining the degree of sequence identity between two Alphabodies, the skilled person may take into account so-called 'conservative' amino acid substitutions, which can generally be described as amino acid substitutions in which an amino acid residue is replaced with another amino acid residue of similar chemical structure and which has little or essentially no influence on the function, activity or other biological properties of the polypeptide. Possible conservative amino acid substitutions will be clear to the person skilled in the art. Alphabodies and nucleic acid sequences are said to be 'exactly the same' if they have 100% sequence identity over their entire length.

An Alphabody of the invention is said to 'specifically bind to' a particular target when that Alphabody of the invention has affinity for, specificity for, and/or is specifically directed against that target (or against at least one part or fragment thereof).

The 'specificity' of an Alphabody of the invention as used herein can be determined based on affinity and/or avidity. The 'affinity' of an Alphabody, polypeptide or composition of the invention is represented by the equilibrium constant for the dissociation of the Alphabody, polypeptide or composition and the target protein of interest to which it binds. The lower the KD value, the stronger the binding strength between the Alphabody, polypeptide or composition and the target protein of interest to which it binds. Alternatively, the affinity can also be expressed in terms of the affinity constant (KA), which corresponds to 1/KD. The binding affinity of an Alphabody of the invention can be determined in a manner known to the skilled person, depending on the specific target protein of interest. It is generally known in the art that the KD can be expressed as the ratio of the dissociation rate constant of a complex, denoted as kOff (expressed in seconds⁻¹ or s⁻¹), to the rate constant of its association, denoted kOn (expressed in molar⁻¹ seconds⁻¹ or M⁻¹ s⁻¹). A KD value greater than about 1 millimolar is generally considered to indicate non-binding or non-specific binding.

The 'avidity' of an Alphabody of the invention is the measure of the strength of binding between an Alphabody of the invention and the pertinent target protein of interest. Avidity is related to both the affinity between a binding site on the target protein of interest and a binding site on the Alphabody of the invention and the number of pertinent binding sites present on the Alphabody of the invention.

An Alphabody of the invention is said to be 'specific for a first target protein of interest as opposed to a second target protein of interest' when it binds to the first target protein of interest with an affinity that is at least 5 times, such as at least 10 times, such as at least 100 times, and preferably at least 1000 times higher than the affinity with which that Alphabody of the invention binds to the second target protein of interest. Accordingly, in certain embodiments, when an Alphabody is said to be 'specific for' a first target protein of interest as opposed to a second target protein of interest, it may specifically bind to (as defined herein) the first target protein of interest, but not to the second target protein of interest.

The 'half-life' of an Alphabody of the invention can generally be defined as the time that is needed for the in vivo serum or plasma concentration of the Alphabody to be reduced by 50%. The in vivo half-life of an Alphabody of the invention can be determined in any manner known to the person skilled in the art, such as by pharmacokinetic analysis. As will be clear to the skilled person, the half-life can be expressed using parameters such as the t1/2-alpha, t1/2-beta and the area under the curve (AUC). An increased half-life in vivo is generally characterized by an increase in one or more and preferably in all three of the parameters t1/2-alpha, t1/2-beta and the area under the curve (AUC).

As used herein, the terms 'inhibiting', 'reducing' and/or 'preventing' may refer to (the use of) an Alphabody according to the invention that specifically binds to a target protein of interest and inhibits, reduces and/or prevents the interaction between that target protein of interest, and its natural binding partner. The terms 'inhibiting', 'reducing' and/or 'preventing' may also refer to (the use of) an Alphabody according to the invention that specifically binds to a target protein of interest and inhibits, reduces and/or prevents a biological activity of that target protein of interest, as measured using a suitable in vitro, cellular or in vivo assay. Accordingly, 'inhibiting', 'reducing' and/or 'preventing' may also refer to (the use of) an Alphabody according to the invention that specifically binds to a target protein of interest and inhibits, reduces and/or prevents one or more biological or physiological mechanisms, effects, responses, functions pathways or activities in which the target protein of interest is involved. Such an action of the Alphabody according to the invention as an antagonist may be determined in any suitable manner and/or using any suitable (in vitro and usually cellular or in vivo) assay known in the art, depending on the target protein of interest.

As used herein, the terms 'enhancing', 'increasing' and/or 'activating' may refer to (the use of) an Alphabody according to the invention that specifically binds to a target protein of interest and enhances, increases and/or activates the interaction between that target protein of interest, and its natural binding partner. The terms 'enhancing', 'increasing' and/or 'activating' may also refer to (the use of) an Alphabody according to the invention that specifically binds to a target protein of interest and enhances, increases and/or activates a biological activity of that target protein of interest, as measured using a suitable in vitro, cellular or in vivo assay. Accordingly, 'enhancing', 'increasing' and/or 'activating' may also refer to (the use of) an Alphabody according to the invention that specifically binds to a target protein of interest and enhances, increases and/or activates one or more biological or physiological mechanisms, effects, responses, functions pathways or activities in which the target protein of interest is involved. Such an action of the Alphabody according to the invention as an agonist may be determined in any suitable manner and/or using any suitable (in vitro and usually cellular or in vivo) assay known in the art, depending on the target protein of interest.

The inhibiting or antagonizing activity or the enhancing or agonizing activity of an Alphabody of the invention may be reversible or irreversible, but for pharmaceutical and pharmacological applications will typically occur reversibly.

An Alphabody, polypeptide, composition or nucleic acid sequence of the invention is considered to be '(in) essentially isolated (form)' as used herein corresponds to an Alphabody, when it has been extracted or purified from the host cell and/or medium in which it is produced.

In respect of the Alphabodies the terms 'binding region', 'binding site' or 'interaction site' present on the Alphabodies shall herein have the meaning of a particular site, part, domain or stretch of amino acid residues present on the Alphabodies that is responsible for binding to a target molecule. Such binding region essentially consists of specific amino acid residues from the Alphabody which are in contact with the target molecule.

An Alphabody of the invention is said to show 'cross-reactivity' for two different target proteins of interest if it is specific for (as defined herein) both of these different target proteins of interest.

An Alphabody of the invention is said to be 'monovalent' if the Alphabody contains one binding site directed against or specifically binding to a site, determinant, part, domain or stretch of amino acid residues of the target of interest. In cases wherein two or more binding sites of an Alphabody are directed against or specifically bind to the same site, determinant, part, domain or stretch of amino acid residues of the target of interest, the Alphabody is said to be 'bivalent' (in the case of two binding sites on the Alphabody) or multivalent (in the case of more than two binding sites on the Alphabody), such as for example trivalent.

The term 'bispecific' when referring to an Alphabody implies that either a) two or more of the binding sites of an Alphabody are directed against or specifically bind to the same target of interest but not to the same (i.e. to a different) site, determinant, part, domain or stretch of amino acid residues of that target, the Alphabody is said to be 'bispecific' (in the case of two binding sites on the Alphabody) or multispecific (in the case of more than two binding sites on the Alphabody) or b) two or more binding sites of an Alphabody are directed against or specifically bind to different target molecules of interest. The term 'muitispecific' is used in the case that more than two binding sites are present on the Alphabody.

Accordingly, a 'bispecific Alphabody' or a 'multi-specific Alphabody' as used herein, shall have the meaning of a single-chain Alphabody of the formula (N-)HRS1-L1-HRS2-L2-HRS3(-C) comprising respectively two or at least two binding sites, wherein these two or more binding sites have a different binding specificity. Thus, an Alphabody is herein considered 'bispecific' or 'multispecific' if respectively two or more than two different binding regions exist in the same single-domain Alphabody.

As used herein, the term 'prevention and/or treatment' comprises preventing and/or treating a certain disease and/or disorder, preventing the onset of a certain disease and/or disorder, slowing down or reversing the progress of a certain disease and/or disorder, preventing or slowing down the onset of one or more symptoms associated with a certain disease and/or disorder, reducing and/or alleviating one or more symptoms associated with a certain disease and/or disorder, reducing the severity and/or the duration of a certain disease and/or disorder, and generally any prophylactic or therapeutic effect of the Alphabodies of the invention that is beneficial to the subject or patient being treated.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

### [BODY OF DESCRIPTION]

The present inventors have identified methods of making or producing target-binding Alphabody polypeptides, more particularly methods for providing cytokine- or growth factor-binding, or cytokine receptor- or growth factor receptor-binding, Alphabody polypeptides. In addition it has been found that target-binding Alphabody polypeptides can bind to the target with affinities at least comparable to traditional binding agents. Moreover, target-binding Alphabody polypeptides maintain the advantages identified for Alphabody scaffolds. Alphabodies not only have a unique structure but also have several advantages over the traditional (immunoglobulin and non-immunoglobulin) scaffolds known in the art. These advantages include, but are not limited to, the fact that they are compact and small in size (between 10 and 14 kDa, which is 10 times smaller than an antibody), they are extremely thermostable (i.e., they generally have a melting temperature of more than 100 °C), they can be made resistant to different proteases, they are highly engineerable (in the sense that multiple substitutions will generally not obliterate their correct and stable folding), and have a structure which is based on natural motifs which have been redesigned via protein engineering techniques.

### [INVENTION RELATED DESCRIPTION]

The present invention provides Alphabody polypeptides capable of binding to a cytokine or growth factor and/or a cytokine or growth factor receptor. Most particularly, the present invention provides Alphabody polypeptides which are capable of binding to a cytokine or growth factor and/or cytokine or growth factor receptor by a binding site which is located either primarily within the groove of an Alphabody, on the surface of an Alphabody helix, or is formed by a linker region of an Alphabody.

More specifically, Alphabody polypeptides directed against cytokines or growth factor and/or cytokine or growth factor receptors are provided wherein the binding site of the Alphabody polypeptide is formed either:
(i) primarily by one or more heptad e-positions in a first alpha-helix of the Alphabody polypeptide and heptad g-positions in a second alpha-helix, and optionally by one or more heptad b-positions in said first alpha-helix of the Alphabody polypeptide and/or heptad c-positions in said second alpha-helix of the Alphabody polypeptide, or
(ii) primarily by heptad b-, c- and f-positions in one alpha-helix of the Alphabody polypeptides, or
(iii) primarily by positions in a linker fragment connecting two consecutive alpha-helices of the Alphabody polypeptides. This will be detailed herein below.

More particularly, the binding site of the Alphabody which can be comprised in an Alphabody polypeptide is formed either:
(i) primarily by one or more heptad e-positions in a first alpha-helix of the Alphabody and heptad g-positions in a second alpha-helix, parallel to said first alpha-helix, and optionally by one or more heptad b-positions in said first alpha-helix of the Alphabody and/or heptad c-positions in said second alpha-helix of the Alphabody, or
(ii) primarily by heptad b-, c- and f-positions in one alpha-helix of the Alphabody, or
(iii) primarily by positions in a linker fragment connecting two consecutive alpha-helices of the Alphabody. This will be detailed herein below.

### [CYTOKINE CLASSES AND RECEPTOR CLASSES AND PARTICULAR EXAMPLES]

The term "growth factor" is typically used in the art to refer to a naturally occurring substance capable of stimulating cellular growth, proliferation and cellular differentiation. Growth factors are important for regulating a variety of cellular processes. The term 'cytokine' typically refers to a sub-class class of growth factors which function as cell-signaling protein molecules. The cytokines are sometimes classed as interleukins, lymphokines, chemokines, transforming growth factors and interferons, based on structure, function, secreting cell type or target cell, but this distinction is obsolete in view of the overlap between these classes. Similarly, for many growth factors the characterization as 'cytokine' or 'hormone' is debated by biochemists in the art.

The present invention relates to Alphabodies which can bind to a growth factor or growth factor receptor. More particularly the growth factor is an immunomodulatory growth factor, such as a cytokine or a growth factor involved in immunomodulation. In particular embodiments, the growth factors of interest in the context of the present invention are those that enhance the cellular immune response of type I or the antibody immune response of type II. Thus, the invention is particularly directed to Alphabodies against growth factors involved in immunomodulation such as the 'classical' cytokines but also including immunomodulatory growth factors such as fms-related tyrosine kinase 3 ligand, inhibins and activins. In particular embodiments, the Alphabodies or Alphabody polypeptides of the present invention can bind to a cytokine or growth factor that belongs to one or more of the following classes of cytokines/growth factors: immune/hematopoietin family, IL-1 family, IL-10 family, IL-12-family, IL-17-family, interferon family (IFNs), TNF family (TNFs), platelet-derived growth factor family (PDGFs), transforming growth factor-beta family (TGF-β) and/or chemokine family.

In particular embodiments the invention relates to Alphabodies directed to cytokines and to growth factors characterized by a 4-helix bundle fold and/or to their receptors. Indeed, growth factors such as Fms-like tyrosine kinase 3 (Flt3) are characterized by the presence of a 4 helix bundle fold which is also found in the four alpha-helix bundle family of cytokines.

In particular embodiments, where the Alphabody polypeptides of the present invention bind to a cytokine or growth factor of the immune/hematopoietin family, they may bind to one or more cytokines or growth factor that belongs to one or more of the following subclasses of hematopoietins: gp130 (IL6ST) shared hematopoietins, IL13RA1 shared hematopoietins, IL12RB1 shared hematopoietins, IL3RB (CSF2RB) shared hematopoietins, ILRG shared hematopoietins.

More particularly, in these embodiments where the Alphabody polypeptides of the present invention bind to a cytokine or growth factor of the immune/hematopoietin family, they may bind to one or more cytokines chosen from the group consisting of: cardiotrophin 1, cardiotrophin-like cytokine factor 1, cardiotrophin-like cytokine factor 1, ciliary neurotrophic factor, interleukin 11, interleukin 6 (interferon, beta 2), leukemia inhibitory factor (cholinergic differentiation factor), oncostatin M, interleukin 4, isoform 1, interleukin 13, interleukin 12A, interleukin 23, alpha subunit p19, colony stimulating factor 2, interleukin 3, interleukin 5, interleukin 2, interleukin 4 isoform 1, interleukin 7, interleukin 9, interleukin 15, preproprotein, interleukin 21, colony stimulating factor 3 isoform a, erythropoietin, and thymic stromal lymphopoietin isoform 1,.

In particular embodiments, where the Alphabodies or Alphabody polypeptides of the present invention bind to a cytokine of the IL-1 family, they may bind to one or more cytokines chosen from the group consisting of: interleukin 1 alpha proprotein and interleukin 1 beta proprotein.

In particular embodiments, where the Alphabodies or Alphabody polypeptides of the present invention bind to a cytokine of the IL-10 family, they may bind to one or more cytokines chosen from the group consisting of: interleukin 10, interleukin 19 isoform 2, interleukin 20, interleukin 22, interleukin 24 isoform 1, interleukin 28A, interleukin 28B and interleukin 29.

In particular embodiments, where the Alphabodies of the present invention bind to a cytokine of the IL-12 family, they may bind to one or more cytokines chosen from the group consisting of: interleukin 12 and interleukin 23.

In particular embodiments, where the Alphabodies or Alphabody polypeptides of the present invention bind to a cytokine of the IL-17 family, they may bind to one or more cytokines chosen from the group consisting of: interleukin 17, interleukin 17B and interleukin 17E isoform 1.

In particular embodiments, where the Alphabody polypeptides of the present invention bind to a cytokine of the interferon family, they may bind to one or more cytokines chosen from the group consisting of: interferon alpha 1, interferon beta 1, interferon kappa, interferon epsilon 1, interferon omega 1 and interferon gamma.

In particular embodiments, where the Alphabodies or Alphabody polypeptides of the present invention bind to a cytokine of the tumor necrosis factor (TNF) family, they may bind to one or more cytokines chosen from the group consisting of: tumor necrosis factor ligand superfamily member 7, tumor necrosis factor ligand superfamily member 14 isoform 1 precursor, tumor necrosis factor ligand superfamily member 13 isoform alphaproprotein, tumor necrosis factor ligand superfamily, member 11 isoform 1, tumor necrosis factor alpha, tumor necrosis factor (ligand) superfamily member 9, tumor necrosis factor (ligand) superfamily member 8, tumor necrosis factor (ligand) superfamily member 4, tumor necrosis factor (ligand) superfamily member 18, tumor necrosis factor (ligand) superfamily member 13b, tumor necrosis factor (ligand) superfamily member 12 isoform 1 precursor, tumor necrosis factor (ligand) superfamily member 10, lymphotoxin-beta isoform a, lymphotoxin alpha, fas ligand, ectodysplasin A isoform EDA-A2, nerve growth factor, CD27 ligand, CD30 ligand and CD40 ligand.

In particular embodiments, where the Alphabody polypeptides of the present invention bind to a growth factor of the platelet-derived growth factor (PDGF) family, they may bind to one or more growth factors chosen from the group consisting of: colony stimulating factor 1 isoform a, epidermal growth factor (beta-urogastrone), fms-related tyrosine kinase 3 ligand, hepatocyte growth factor isoform 1 preproprotein, KIT ligand isoform b, PH domain-containing protein, platelet-derived growth factor beta isoform 1 preproprotein, platelet-derived growth factor C, vascular endothelial growth factor B, vascular endothelial growth factor C preproprotein and vascular endothelial growth factor isoform a.

In particular embodiments, the Alphabody polypeptides of the present invention bind to Flt3L or its receptor Flt3R. 'Flt3' is known in the art as 'Fms-like tyrosine kinase 3'. Other names for Flt3 in the literature are 'Flk-2' ('fetal liver kinase 2') and 'STK-1' ('stem cell tyrosine kinase 1'). 'Flt3R' is known in the art as 'Flt3 receptor'. The murine Flt3/Flk-2 receptor was identified in the early 1990s (Matthews et al., Cell, 1991, 65:1143-1152; Rosnet et al., Oncogene, 1991, 6:1641-1650). Subsequently, using DNA probes based on the murine Flt3/Flk-2 sequence, the human Flt3 cDNA was isolated and cloned (Rosnet et al., Blood, 1993, 82:1110-1119; Small et al., PNAS, 1994, 91:459-463). Flt3R is a member of the tyrosine kinase III family which also includes CSF1-R (colony stimulating factor 1 receptor), c-kit and PDGF-Rα/β (platelet-derived growth factor receptors α and β). These receptors are bitopic type I membrane proteins which are active as a dimer. They consist of an ectodomain that is built up of 5 immunoglobulin-like subdomains, a transmembrane domain, and a cytoplasmic part that contains an ATP-binding site and a kinase domain. Dimeric ligands are needed to dimerize and activate these receptors. Flt3R is involved in the proliferation, differentiation and apoptosis of haematopoietic cells. It is mainly expressed by early myeloid and lymphoid progenitor cells. 'Flt3L' is known in the art as 'Flt3 ligand'. Murine Flt3L was identified as the cognate protein ligand for the murine Flt3 receptor by its ability to bind an Flt3-Fc fusion construct (Lyman et al., Cell, 1993, 75:1157-1167; Hannum et al., Nature, 1994, 368:643-648). Consequently, the human Flt3L cDNA was cloned (Lyman et al., Blood, 1994, 83:2795-2801; Hannum et al., Nature, 1994, 368:643-648). To date, Flt3L is the only known ligand to Flt3R. Flt3L is expressed by all cell types, except in brain tissue. In synergy with other factors (e.g., IL3, c-kit ligand, CSF, IL7, IL11), Flt3L can stimulate the proliferation and differentiation of certain early hematopoietic progenitor cells. The Flt3 system is also an important player in the development of dendritic cells.

In particular embodiments, where the Alphabody polypeptides of the present invention bind to a growth factor of the transforming growth factor-beta family (TGF-β), they may bind to one or more growth factors chosen from the group consisting of: transforming growth factor beta 3, transforming growth factor beta 2, transforming growth factor beta 1, inhibin beta C chain preproprotein, inhibin beta B subunit, inhibin beta A, growth differentiation factor 5 preproprotein, bone morphogenetic protein 7, bone morphogenetic protein 2, and activin beta E.

In particular embodiments, where the Alphabody polypeptides of the present invention bind to a cytokine of the chemokine family, they may bind to one or more cytokines that belongs to one or more of the following subclasses of chemokines: C subfamily, CC subfamily, CXC subfamily and CX3C subfamily.

More particularly, where the Alphabody polypeptides of the present invention bind to a cytokine of the chemokine family, they may bind to one or more cytokines chosen from the group consisting of: chemokine (C motif) ligand 1, chemokine (C motif) ligand 2, chemokine (C-C motif) ligand 14 isoform 1, chemokine (C-C motif) ligand 15, chemokine (C-C motif) ligand 20, chemokine (C-C motif) ligand 26, chemokine (C-C motif) ligand 3, chemokine (C-C motif) ligand 4, chemokine (C-C motif) ligand 7, chemokine (C-C motif) ligand 1, chemokine (C-C motif) ligand 11, chemokine (C-C motif) ligand 13, chemokine (C-C motif) ligand 16, chemokine (C-C motif) ligand 17, chemokine (C-C motif) ligand 19, chemokine (C-C motif) ligand 2, chemokine (C-C motif) ligand 21, chemokine (C-C motif) ligand 22, chemokine (C-C motif) ligand 23 isoform CKbeta8-1, chemokine (C-C motif) ligand 24, chemokine (C-C motif) ligand 25 isoform 1, chemokine (C-C motif) ligand 27, chemokine (C-C motif) ligand 28, chemokine (C-C motif) ligand 5, chemokine (C-C motif) ligand 8, chemokine (C-X-C motif) ligand 1, chemokine (C-X-C motif) ligand 12 (stromal cell-derived factor 1)isoform beta, chemokine (C-X-C motif) ligand 13 (B-cell chemoattractant), chemokine (C-X-C motif) ligand 16, chemokine (C-X-C motif) ligand 2, chemokine (C-X-C motif) ligand 3, chemokine (C-X-C motif) ligand 5, chemokine (C-X-C motif) ligand 6 (granulocyte chemotactic protein2), interleukin 8, pro-platelet basic protein, platelet factor (PF)4, groa, MIG, ENA-78, macrophage inflammatory protein (MIP) I a, MIP I monocyte chemoattractant protein (MCP)- 1, 1-3 09, HC 14, C 10, Regulated on Activation, Normal T-cell Expressed, Secreted (RANTES), chemokine (C-X-C motif) ligand 10, chemokine (C-X-C motif) ligand 11, chemokine (C-X-C motif) ligand 9 and chemokine (C-X3-C motif) ligand 1.

In particular embodiments, the Alphabody polypeptides of the present invention can bind to one or more cytokine or growth factor receptors that belong to one or more of the following classes of cytokine or growth factor receptors: type I cytokine receptors, type II cytokine receptors, immunoglobulin superfamily, Tumor necrosis factor receptor family, chemokine receptors and TGF beta receptors.

In further particular embodiments, where the Alphabody polypeptides of the present invention bind to a cytokine or growth factor receptor of the type I cytokine receptor family, they may bind to one or more of the following cytokine receptors: type 1 interleukin receptor, erythropoietin receptor, GM-CSF receptor, G-CSF receptor, oncostatin M receptor and leukemia inhibitory factor receptor.

In certain particular embodiments, where the Alphabody polypeptides of the present invention bind to a cytokine receptor of the type II cytokine receptor family, they may bind to one or more of the following cytokine receptors: type II interleukin receptors, interferon-alpha/beta receptor and interferon-gamma receptor.

In certain particular embodiments, where the Alphabody polypeptides of the present invention bind to a cytokine receptor of the immunoglobulin superfamily, they may bind to one or more of the following cytokine receptors: interleukin-1 receptor, CSF1, C-kit receptor and interleukin-18 receptor.

In certain particular embodiments, where the Alphabody polypeptides of the present invention bind to a cytokine receptor of the tumor necrosis factor receptor family, they may bind to one or more of the following cytokine receptors: CD27, CD30, CD120, CD40 and lymphotoxin beta receptor.

In certain particular embodiments, where the Alphabody polypeptides of the present invention bind to a cytokine receptor of the chemokine receptor family, they may bind to one or more of the following cytokine receptors: interleukin-8 receptor, CCR1, CCR5, CXCR4, CXCR7, MCAF receptor, NAP-2 receptor, CC chemokine receptors, CXC chemokine receptors, CX3C chemokine receptors, and XC chemokine receptor (XCR1).

In certain particular embodiments, where the Alphabody polypeptides of the present invention bind to a growth factor receptor of the TGF beta receptor family, they may bind to one or more of the following cytokine receptors: TGF beta receptor 1 and TGF beta receptor 2.

### [ALPHABODIES, POLYPEPTIDES AND COMPOSITIONS AGAINST HETERODIMERIC CYTOKINES AND/OR HETERODIMERIC CYTOKINE RECEPTORS]

In specific embodiments of the present invention, the Alphabody polypeptides of the present invention specifically bind to a heterodimeric cytokine or growth factor (as defined herein), and/or to a heterodimeric cytokine or growth factor receptor.

In its broadest interpretation, the term 'heterodimeric cytokine(s) or growth factor(s)' as used herein includes any cytokine or growth factor that comprises at least two, and in particular cases only two, subunits. More particularly, the terms 'heterodimeric cytokine' or 'heterodimeric growth factor' as used herein encompasses both heterodimeric cytokines or growth factors involved in cell-mediated (THI) immunity and heterodimeric cytokines or growth factors involved in humoral (TH2) immunity.

In further particular embodiments, Alphabody polypeptides are provided against heterodimeric cytokines or growth factors whereby the Alphabody is specific for the subunit of the cytokine or growth factor which is specific for the cytokine or growth factor. Alternatively the subunit of interest is the subunit which is common to two or more cytokines or growth factors. Thus, in particular embodiments of the present invention, Alphabodies are provided which are directed against a specific subunit.

According to one particular, but non-limiting embodiment, the Alphabody polypeptides of the invention specifically bind to a subunit selected from a p19 subunit or a p19-like subunit, such as a p19 subunit present in IL-23, a p35 subunit present in IL-12 and IL-35, or a p28 subunit present in IL-27 or a homolog thereof. More particularly, the Alphabody polypeptides are capable of binding a cytokine comprising one or more of these subunits. According to an even more particular, but non-limiting embodiment, the Alphabody polypeptides of the invention specifically bind to a p19 subunit or a p19-like subunit of a heterodimeric cytokine. These Alphabody polypeptides of the invention that specifically bind to a p19 or a p19-like subunit, such as to the p19 subunit of IL-23, compared to a p35 subunit and a p40 subunit, may have advantages for prophylactic, therapeutic and/or diagnostic purposes compared to the Alphabodies of the invention that specifically bind to p35(-like) subunit or p40(-like) subunit.

In particular embodiments, the Alphabody polypeptides of the invention specifically bind to the p19 subunit of IL-23 or a homolog thereof.

According to another particular embodiment, the Alphabody polypeptides of the invention specifically bind to a heterodimeric cytokine that comprises a p40 subunit or a p40-like subunit, such as a p40 subunit present in IL-12 and IL-23 or Epstein-Barr virus (EBV)-induced molecule 3 (EBI3) present in IL-27 and IL-35.

According to a specific embodiment, the Alphabody polypeptides of the invention specifically bind to a p19 subunit or a p19-like subunit of a heterodimeric cytokine or to a p40 subunit or a p40-like subunit of a heterodimeric cytokine or to both a p19 subunit or a p19-like subunit and a p40 subunit or a p40-like subunit of the same heterodimeric cytokine. In an even more particular embodiment, the Alphabodies of the present invention exclusively bind to a p19 subunit or a p19-like subunit of a heterodimeric cytokine and not to a p40-like subunit of a heterodimeric cytokine.

In certain embodiments, the Alphabody polypeptides of the invention can specifically bind to a heterodimeric cytokine that comprises at least one p19 subunit or p19-like subunit and at least one p40 subunit or p40-like subunit.

Accordingly, in specific embodiments of the present invention, the Alphabody polypeptides of the present invention specifically bind to a heterodimeric cytokine (as defined herein) chosen from the group consisting of IL-12, IL-23, IL-27 and IL-35.

In a particular embodiment, the Alphabody polypeptides of the present invention bind to IL-23. Thus, according to one specific embodiment, the Alphabody polypeptides of the present invention specifically bind either to the p19 subunit of IL-23 or to the p40 subunit of IL-23 or to both the p40 and the p19 subunit of IL-23.

In a further particular embodiment, the Alphabody polypeptides of the present invention specifically and exclusively bind to the p19 subunit of IL-23 but not to the p40 subunit of IL-23. More particularly, the Alphabody polypeptides specifically bind IL-23. Such IL-23-specific Alphabody polypeptides are of interest for preventing and/or treating diseases and/or disorders in which IL-23 is involved.

In further particular embodiments, the Alphabody polypeptides of the present invention comprise an amino acid sequence having at least 80% sequence identity with at least one of the amino acid sequences corresponding to any one of SEQ_ID 1 to SEQ_ID127.

In another specific embodiment, the Alphabody polypeptides of the present invention specifically bind to IL-12 or a subunit thereof. More particularly, the Alphabody polypeptides of the invention bind to the p35 subunit of IL-12. Such Alphabody polypeptides of the invention that specifically bind to IL-12 can be of interest for preventing and/or treating diseases and/or disorders in which IL-12 is involved.

In yet another specific embodiment, the Alphabody polypeptides of the present invention specifically bind to IL-27, more particularly to the p28 subunit of IL-27. Such Alphabody polypeptides of the invention that specifically bind to IL-27 can be used for preventing and/or treating diseases and/or disorders in which IL-27 is involved.

In another specific embodiment, the Alphabody polypeptides of the present invention specifically bind to IL-35. In particular embodiments, they bind to the p35 subunit of IL-35. Such Alphabody polypeptides of the invention that specifically bind to IL-35 are of use for preventing and/or treating diseases and/or disorders in which IL-35 is involved.

In particular embodiments, the Alphabody polypeptides of the present invention bind to one or more heterodimeric cytokine receptors, such as for example but not limited to the IL-12 receptor, the IL-23 receptor, the IL-27 receptor or the IL-35 receptor.

For example, in particular embodiments the Alphabody polypeptides of the present invention bind to the IL-23R subunit of the IL-23 receptor. In further embodiments, the Alphabody polypeptides of the present invention bind to the IL12R-β1 and/or to the IL12R-β2 subunit of the IL-12 receptor. In further particular embodiments, the Alphabody polypeptides of the present invention bind to the IL-27R and/or to the gp130 subunit of the IL-27 receptor.

In particular embodiments, the Alphabody polypeptides of the invention bind specifically to at least two different subunits of a heterodimeric cytokine. More particularly, such an Alphabody polypeptide of the invention specifically binds to the interface of two different subunits that occur in the same heterodimeric cytokine, such as to the p19/p40 interface in IL-23 or to the p35/p40 interface in IL-12 or to the p28/EBI3 interface in IL-27, or to the the p35/EBI3 interface in IL-35.

Also, where the Alphabody polypeptides of the invention bind specifically to at least two different subunits of a heterodimeric cytokine receptor, it should be clear that such Alphabody polypeptides of the invention may specifically bind to the interface of two different subunits that occur in the same heterodimeric cytokine receptor, such as to the IL-23R/ IL12R-β2 interface in IL-23 receptor or to the IL12R-β1/ IL12R-β2 interface in IL-12 receptor or to the IL-27R/gp130 interface in IL-27 receptor.

It should be noted that the Alphabody polypeptides of the present invention that bind to one or more heterodimeric cytokines and/or to one or more heterodimeric cytokine receptors can, optionally, further bind to one or more other (non-heterodimeric) cytokines and/or one or more other (non-heterodimeric) cytokine receptors.

The present inventors have identified methods for generating Alphabody polypeptides binding specifically to a cytokine or growth factor. Thus, cytokine- or growth factor-binding Alphabody polypeptides are provided. Examples of cytokines or growth factors to which the Alphabody polypeptides can be specifically directed include cytokines or growth factor chosen from the group consisting of but not limited to cardiotrophin 1, cardiotrophin-like cytokine factor 1, cardiotrophin-like cytokine factor 1, ciliary neurotrophic factor, interleukin 11, interleukin 6 (interferon, beta 2), leukemia inhibitory factor (cholinergic differentiation factor), oncostatin M, interleukin 4, isoform 1, interleukin 13; interleukin 12A, interleukin 23, alpha subunit p19, colony stimulating factor 2, interleukin 3, interleukin 5, interleukin 2, interleukin 4 isoform 1, interleukin 7, interleukin 9, interleukin 15, preproprotein, interleukin 21, colony stimulating factor 3 isoform a, erythropoietin, thymic stromal lymphopoietin isoform 1, interleukin 1 alpha proprotein and interleukin 1 beta proprotein, interleukin 10, interleukin 19 isoform 2, interleukin 20, interleukin 22, interleukin 24 isoform 1, interleukin 28A, interleukin 28B and interleukin 29, interleukin 17, interleukin 17B and interleukin 17E isoform 1, interferon alpha 1, interferon beta 1, interferon kappa, interferon epsilon 1, interferon omega 1, interferon gamma, tumor necrosis factor ligand superfamily member 7, tumor necrosis factor ligand superfamily member 14 isoform 1 precursor, tumor necrosis factor ligand superfamily member 13 isoform alphaproprotein, tumor necrosis factor ligand superfamily, member 11 isoform 1, tumor necrosis factor alpha, tumor necrosis factor (ligand) superfamily member 9, tumor necrosis factor (ligand) superfamily member 8, tumor necrosis factor (ligand) superfamily member 4, tumor necrosis factor (ligand) superfamily member 18, tumor necrosis factor (ligand) superfamily member 13b, tumor necrosis factor (ligand) superfamily member 12 isoform 1 precursor, tumor necrosis factor (ligand) superfamily member 10, lymphotoxin-beta isoform a, lymphotoxin alpha, fas ligand, ectodysplasin A isoform EDA-A2, nerve growth factor, CD27 ligand, CD30 ligand, CD40 ligand, colony stimulating factor 1 isoform a, epidermal growth factor (beta-urogastrone), fms-related tyrosine kinase 3 ligand, hepatocyte growth factor isoform 1 preproprotein, KIT ligand isoform b, PH domain-containing protein, platelet-derived growth factor beta isoform 1 preproprotein, platelet-derived growth factor C, vascular endothelial growth factor B, vascular endothelial growth factor C preproprotein, vascular endothelial growth factor isoform a, transforming growth factor beta 3, transforming growth factor beta 2, transforming growth factor beta 1, inhibin beta C chain preproprotein, inhibin beta B subunit, inhibin beta A, growth differentiation factor 5 preproprotein, bone morphogenetic protein 7, bone morphogenetic protein 2, activin beta E, chemokine (C motif) ligand 1, chemokine (C motif) ligand 2, chemokine (C-C motif) ligand 14 isoform 1, chemokine (C-C motif) ligand 15, chemokine (C-C motif) ligand 20, chemokine (C-C motif) ligand 26, chemokine (C-C motif) ligand 3, chemokine (C-C motif) ligand 4, chemokine (C-C motif) ligand 7, chemokine (C-C motif) ligand 1, chemokine (C-C motif) ligand 11, chemokine (C-C motif) ligand 13, chemokine (C-C motif) ligand 16, chemokine (C-C motif) ligand 17, chemokine (C-C motif) ligand 19, chemokine (C-C motif) ligand 2, chemokine (C-C motif) ligand 21, chemokine (C-C motif) ligand 22, chemokine (C-C motif) ligand 23 isoform CKbeta8-1, chemokine (C-C motif) ligand 24, chemokine (C-C motif) ligand 25 isoform 1, chemokine (C-C motif) ligand 27, chemokine (C-C motif) ligand 28, chemokine (C-C motif) ligand 5, chemokine (C-C motif) ligand 8, chemokine (C-X-C motif) ligand 1, chemokine (C-X-C motif) ligand 12 (stromal cell-derived factor 1)isoform beta, chemokine (C-X-C motif) ligand 13 (B-cell chemoattractant), chemokine (C-X-C motif) ligand 16, chemokine (C-X-C motif) ligand 2, chemokine (C-X-C motif) ligand 3, chemokine (C-X-C motif) ligand 5, chemokine (C-X-C motif) ligand 6 (granulocyte chemotactic protein2), interleukin 8, pro-platelet basic protein, platelet factor (PF)4, groa, MIG, ENA-78, macrophage inflammatory protein (MIP) I a, MIP I monocyte chemoattractant protein (MCP)- 1, 1-3 09, HC 14, C 10, Regulated on Activation, Normal T-cell Expressed, Secreted (RANTES), chemokine (C-X-C motif) ligand 10, chemokine (C-X-C motif) ligand 11, chemokine (C-X-C motif) ligand 9 and chemokine (C-X3-C motif) ligand 1.

Typically, the Alphabody polypeptides of the invention will bind to a target protein of interest with a dissociation constant (KD) of less than about 1 micromolar (1 µM), and preferably less than about 1 nanomolar (1 nM) [i.e., with an association constant (KA) of about 1,000,000 per molar (10⁶ M⁻¹, 1 E6 /M) or more and preferably about 1,000,000,000 per molar (10⁹ M⁻¹, 1 E9 /M) or more]. A KD value greater than about 1 millimolar is generally considered to indicate non-binding or non-specific binding. It is generally known in the art that the KD can also be expressed as the ratio of the dissociation rate constant of a complex, denoted as kOff (expressed in seconds⁻¹ or s⁻¹), to the rate constant of its association, denoted kOn (expressed in molar⁻¹ seconds⁻¹ or M⁻¹ s⁻¹). In particular, an Alphabody polypeptide of the invention will bind to the target protein of interest with a kOff ranging between 0.1 and 0.0001 s⁻¹ and/or a kOn ranging between 1,000 and 1,000,000 M⁻¹ s⁻¹. Binding affinities, kOff and kOn rates may be determined by means of methods known to the person skilled in the art, for example ELISA methods, isothermal titration calorimetry, surface plasmon resonance, fluorescence-activated cell sorting analysis, and the more.

The target-binding Alphabody polypeptides according to the present invention are amino acid sequences comprising the general formula HRS1-L1-HRS2-L2-HRS3, optionally comprising additional N- and C-terminal linked groups, residues or moieties resulting in the formula N-HRS1-L1-HRS2-L2-HRS3-C. The optional N and C extensions can be, for example, a tag for detection or purification (e.g., a His-tag) or another protein or protein domain, in which case the full construct is denoted a fusion protein. For the sake of clarity, the optional extensions N and C are herein considered not to form part of a single-chain Alphabody structure, which is defined by the general formula 'HRS1-L1-HRS2-L2-HRS3'.

As indicated above, a heptad repeat of an Alphabody is generally represented as 'abcdefg' or 'defgabc', wherein the symbols 'a' to 'g' denote conventional heptad positions. The 'a-positions' and 'd-positions' in each heptad unit of an Alphabody of the invention are amino acid residue positions of the coiled coil structure where the solvent-shielded (i.e., buried) core residues are located. The 'e-positions' and 'g-positions' in each heptad unit of an Alphabody of the invention are amino acid residue positions of the coiled coil structure where the amino acid residues which are partially solvent-exposed are located. In a triple-stranded coiled coil, these 'e-positions' and 'g-positions' are located in the groove formed between two spatially adjacent alpha-helices, and the corresponding amino acid residues are commonly denoted the 'groove residues'. The 'b-positions', 'c-positions' and 'f-positions' in each heptad unit of an Alphabody of the invention are the most solvent-exposed positions in a coiled coil structure.

It is noted that in the prior art, a heptad may be referred to as 'heptad repeat' because the 7-residue fragment is usually repeated a number of times in a true coiled coil amino acid sequence.

A heptad motif (as defined herein) of the type 'abcdefg' is typically represented as 'HPPHPPP', whereas a 'heptad motif of the type 'defgabc' is typically represented as 'HPPPHPP', wherein the symbol 'H' denotes an apolar or hydrophobic amino acid residue and the symbol 'P' denotes a polar or hydrophilic amino acid residue. Typical hydrophobic residues located at a- or d-positions include aliphatic (e.g., leucine, isoleucine, valine, methionine) or aromatic (e.g., phenylalanine) amino acid residues. Heptads within coiled coil sequences do not always comply with the ideal pattern of hydrophobic and polar residues, as polar residues are occasionally located at 'H' positions and hydrophobic residues at 'P' positions. Thus, the patterns 'HPPHPPP' and 'HPPPHPP' are to be considered as ideal patterns or characteristic reference motifs. Occasionally, the characteristic heptad motif is represented as 'HPPHCPC' or 'HxxHCxC' wherein 'H' and 'P' have the same meaning as above, 'C' denotes a charged residue (lysine, arginine, glutamic acid or aspartic acid) and 'x' denotes any (unspecified) natural amino acid residue. Since a heptad can equally well start at a d-position, the latter motifs can also be written as 'HCPCHPP' or 'HCxCHxx'. It is noted that single-chain Alphabodies are intrinsically so stable that they do not require the aid of ionic interactions between charged ('C') residues at heptad e- and g-positions.

A heptad repeat sequence (HRS) (as defined herein) is typically represented by (abcdefg)ₙ or (defgabc)ₙ in notations referring to conventional heptad positions, or by (HPPHPPP)ₙ or (HPPPHPP)ₙ in notations referring to the heptad motifs, with the proviso that not all amino acid residues in a HRS should strictly follow the ideal pattern of hydrophobic and polar residues. In order to identify heptad repeat sequences, and/or their boundaries, these heptad repeat sequences comprising amino acids or amino acid sequences that deviate from the consensus motif, and if only amino acid sequence information is at hand, then the COILS method of Lupas et al. (Science 1991, 252:1162-1164) is a suitable method for the determination or prediction of heptad repeat sequences and their boundaries, as well as for the assignment of heptad positions. Furthermore, the heptad repeat sequences can be resolved based on knowledge at a higher level than the primary structure (i.e., the amino acid sequence). Indeed, heptad repeat sequences can be identified and delineated on the basis of secondary structural information (i.e. alpha-helicity) or on the basis of tertiary structural (i.e., protein folding) information. A typical characteristic of a putative HRS is an alpha-helical structure. Another (strong) criterion is the implication of a sequence or fragment in a coiled coil structure. Any sequence or fragment that is known to form a regular coiled coil structure, i.e., without stutters or stammers as described in Brown et al. Proteins 1996, 26:134-145, is herein considered a HRS. Also and more particularly, the identification of HRS fragments can be based on high-resolution 3-D structural information (X-ray or NMR structures). Finally, but not limited hereto, and unless clear evidence of the contrary exists, or unless otherwise mentioned, the boundaries to any HRS fragment may be defined as the first (respectively last) a- or d-position at which a standard hydrophobic amino acid residue (selected from the group valine, isoleucine, leucine, methionine, phenylalanine, tyrosine or tryptophan) is located.

The linkers within a single-chain structure of the Alphabodies (as defined herein) interconnect the HRS sequences, and more particularly the first to the second HRS, and the second to the third HRS in an Alphabody. Each linker sequence in an Alphabody commences with the residue following the last heptad residue of the preceding HRS and ends with the residue preceding the first heptad residue of the next HRS. Connections between HRS fragments via disulfide bridges or chemical cross-linking or, in general, through any means of inter-chain linkage (as opposed to intra-chain linkage), are explicitly excluded from the definition of a linker fragment (at least, in the context of an Alphabody) because such would be in contradiction with the definition of a single-chain Alphabody. A linker fragment in an Alphabody is preferably flexible in conformation to ensure relaxed (unhindered) association of the three heptad repeat sequences as an alpha-helical coiled coil structure. Further in the context of an Alphabody, 'L1' shall denote the linker fragment one, i.e., the linker between HRS1 and HRS2, whereas 'L2' shall denote the linker fragment two, i.e., the linker between HRS2 and HRS3. Suitable linkers for use in the Alphabodies of the invention will be clear to the skilled person, and may generally be any linker used in the art to link amino acid sequences, as long as the linkers are structurally flexible, in the sense that they do not affect the characteristic three dimensional coiled coil structure of the Alphabody. The two linkers L1 and L2 in a particular Alphabody of the invention, may be the same or may be different. Based on the further disclosure herein, the skilled person will be able to determine the optimal linkers for a specific Alphabody of the invention, optionally after performing a limited number of routine experiments. In particular embodiments, the linkers L1 and L2 are amino acid sequences consisting of at least 4, in particular at least 8, more particularly at least 12 amino acid residues, with a non-critical upper limit chosen for reasons of convenience being about 30 amino acid residues. In a particular, non-limiting embodiment, preferably at least 50% of the amino acid residues of a linker sequence are selected from the group proline, glycine, and serine. In further non-limiting embodiments, preferably at least 60%, such as at least 70%, such as for example 80% and more particularly 90% of the amino acid residues of a linker sequence are selected from the group proline, glycine, and serine. In other particular embodiments, the linker sequences essentially consist of polar amino acid residues; in such particular embodiments, preferably at least 50%, such as at least 60%, such as for example 70% or 80% and more particularly 90% or up to 100% of the amino acid residues of a linker sequence are selected from the group consisting of glycine, serine, threonine, alanine, proline, histidine, asparagine, aspartic acid, glutamine, glutamic acid, lysine and arginine.

In particular embodiments of the invention, each of L1 and L2 are independently a linker fragment, covalently connecting HRS1 to HRS2 and HRS2 to HRS3, respectively, and consisting of at least 4 amino acid residues, preferably at least 50% of which are selected from the group proline, glycine, serine.

The 'coiled coil' structure of an Alphabody can be considered as being an assembly of alpha-helical heptad repeat sequences wherein the helical heptad repeat sequences are as defined supra;
- the said alpha-helical heptad repeat sequences are wound (wrapped around each other) with a left-handed supertwist (supercoiling);
- the core residues at a- and d-positions form the core of the assembly, wherein they pack against each other in a knobs-into-holes manner as defined in the Socket algorithm (Walshaw and Woolfson J Mol Biol 2001, 307:1427-1450) and reiterated in Lupas and Gruber Adv Protein Chem 2005, 70:37-78;
- the core residues are packed in regular core packing layers, where the layers are defined as in Schneider et al Fold Des 1998, 3:R29-R40.

The coiled coil structure of the Alphabodies of the present invention is not to be confused with ordinary three-helix bundles. Criteria to distinguish between a true coiled coil and non-coiled coil helical bundles are provided in Desmet et al. WO 2010/066740 A1 and Schneider et al Fold Des 1998, 3:R29-R40; such criteria essentially relate to the presence or absence of structural symmetry in the packing of core residues for coiled coils and helix bundles, respectively. Also the presence or absence of left-handed supercoiling for coiled coils and helix bundles, respectively, provides a useful criterion to distinguish between both types of folding.

While aforegoing criteria in principle apply to 2-stranded, 3-stranded, 4-stranded and even more-stranded coiled coils, the Alphabodies of the present invention are restricted to 3-stranded coiled coils. The coiled coil region in an Alphabody can be organized with all alpha-helices in parallel orientation (corresponding to a 'parallel Alphabody' as described in EP2161278 by Applicant Complix NV) or with one of the three alpha-helices being antiparallel to the two other (corresponding to an 'antiparallel Alphabody' as described in WO 2010/066740 by Applicant Complix NV).

The alpha-helical part of an Alphabody (as defined herein) will usually grossly coincide with the heptad repeat sequences although differences can exist near the boundaries. For example, a sequence fragment with a clear heptad motif can be non-helical due to the presence of one or more helix-distorting residues (e.g., glycine or proline). Reversely, part of a linker fragment can be alpha-helical despite the fact that it is located outside a heptad repeat region. Further, any part of one or more alpha-helical heptad repeat sequences is also considered an alpha-helical part of a single-chain Alphabody.

The solvent-oriented region of (the alpha-helices of) an Alphabody (as defined herein) is an important Alphabody region. In view of the configuration of the alpha-helices in an Alphabody, wherein the residues at heptad a- and d-positions form the core, the solvent-oriented region is largely formed by b-, c- and f-residues. There are three such regions per single-chain Alphabody, i.e., one in each alpha-helix. Any part of such solvent-oriented region is also considered a solvent-oriented region. For example, a subregion composed of the b-, c- and f-residues from three consecutive heptads in an Alphabody alpha-helix will also form a solvent-oriented surface region.

Residues implicated in the formation of (the surface of) a groove between two adjacent alpha-helices in an Alphabody are generally located at heptad e- and g-positions, but some of the more exposed b- and c-positions as well as some of the largely buried core a- and d-positions may also contribute to a groove surface; such will essentially depend on the size of the amino acid side-chains placed at these positions. If the said spatially adjacent alpha-helices run parallel, then one half of the groove is formed by b- and e-residues from a first helix and the second half by c- and g-residues of the second helix. If the said spatially adjacent alpha-helices are antiparallel, then there exist two possibilities. In a first possibility, both halves of the groove are formed by b- and e-residues. In the second possibility, both halves of the groove are formed by c- and g-residues. The three types of possible grooves are herein denoted by their primary groove-forming (e- and g-) residues: if the helices are parallel, then the groove is referred to as an e/g-groove; if the helices are antiparallel, then the groove is referred to as either an e/e-groove or a g/g-groove. Parallel Alphabodies have three e/g-grooves, whereas antiparallel Alphabodies have one e/g-groove, one e/e-groove and one g/g-groove. Any part of an Alphabody groove is also considered a groove region.

The inventors have identified methods for obtaining the target-specific, and more particularly cytokine or growth factor or cytokine- or growth factor-receptor-specific Alphabodies and Alphabody polypeptides comprising such Alphabodies. These methods are based on the concept of random library screening.

### [PRODUCTION OF LIBRARIES]

The target-specific Alphabodies or Alphabody polypeptides according to the present invention can be obtained by methods which involve generating a random library of Alphabody polypeptides and screening this library for an Alphabody polypeptide capable of specifically binding to a target of interest, and in particular to a cytokine or growth factor and/or cytokine or growth factor receptor of interest.

A first step in the methods according to the invention comprises the production of a library (i.e., collection or set) of Alphabody polypeptide sequences, which differ from each other in at least one of a defined set of 5 to 20 variegated amino acid residue positions. Therefore, the sequences within a library of Alphabody polypeptides differ from each other at particular amino acid positions that are comprised in a selected or defined set. Accordingly, the term 'different-sequence' refers to the occurrence of sequence variation or sequence differences in a defined set of amino acid residue positions between two or more Alphabody polypeptides of the libraries of the invention.

A library or collection of Alphabody polypeptide sequences may contain any suitable number of different Alphabody (polypeptide) sequences, such as at least 2, at least 5, at least 10, at least 50, at least 100, at least 1000, at least 10,000, at least 10⁵, at least 10⁶, at least 10⁷, at least 10⁸, at least 109 or more different-sequence (single-chain) Alphabodies or Alphabody polypeptides.

More particularly, a library or collection of different Alphabody (polypeptide) sequences according to the present invention contains at least 100 different-sequence Alphabody polypeptides, such as at least 200, at least 300, at least 400, at least 500, such as at least 1000, at least 10000, at least 10⁵, at least 10⁶, at least 10⁷, at least 10⁸, at least 10⁹ or more sequences.

In particular embodiments, a set, collection or library (as used interchangeably herein) of Alphabody polypeptide sequences of the present invention contains at least 100 different-sequence Alphabody polypeptides.

In addition, the single-chain Alphabody libraries of the invention are characterized in that the different-sequence single-chain Alphabody polypeptides comprised in those libraries differ from each other in at least one of a defined set of variegated amino acid residue positions.

Accordingly, the different Alphabody sequences, also referred to herein as different-sequence (single-chain) Alphabodies, comprised in the libraries of the invention only differ from each other in a defined, i.e. fixed or predetermined, set of amino acid residue positions.

Such a defined set of variegated amino acid residue positions consists of a number of particular amino acid residue positions, which are characterized by variety or diversity of amino acid residue types when the different-sequence (single-chain) Alphabodies within the produced library are compared to each other.

The notion 'variegated amino acid residue position', when referring to a library of different-sequence Alphabodies, refers to an amino acid residue position at which at least two different amino acid residue types (amino acid residues of a defined type, for example natural amino acid residue types) are located when at least two of the amino acid sequences of the different-sequence Alphabodies from the said library of Alphabodies are compared to each other (note that these positions will not differ for any two different-sequence Alphabodies of the library, but that the library comprises at least two different-sequence Alphabodies which differ in this amino acid residue position). A 'set of variegated amino acid residue positions', when referring to a library of different-sequence Alphabodies, refers to the set of amino acid residue positions at which at least two different amino acid residue types are located when at least two of the amino acid sequences of the different-sequence Alphabodies from the said library of Alphabodies are compared to each other (note that these positions will not differ for any two different-sequence Alphabodies of the library). A 'defined set of variegated amino acid residue positions', when referring to a library of different-sequence Alphabodies, refers to the specific set of amino acid residue positions at which at least two different amino acid residue types are located when all amino acid sequences from the said library of different-sequence Alphabodies are simultaneously compared to each other. Thus, the simultaneous comparison of all amino acid sequences from the said library of different-sequence Alphabodies, and the identification of amino acid residue positions at which at least two different amino acid residue types are located in such simultaneous comparison, allows to identify the said defined set of variegated amino acid residue positions in an Alphabody library. It is herein submitted that a skilled person will known how to determine the sequences in a library of sequences such as a single-chain Alphabody library. It is herein further understood that Alphabody sequences can be compared both at the level of amino acid sequences or nucleotide sequences representing (encoding) these amino acid sequences.

A preferred method to compare two or more different-sequence Alphabodies is based on a pair-wise or multiple sequence alignment, generated by a known computer algorithm for automated sequence alignment such as NCBI Blast. Alternatively, two or more different-sequence Alphabodies can be compared on the basis of a pair-wise or multiple sequence alignment which is generated by a skilled user, such method of alignment also being known as manual sequence alignment. Both of the techniques of automated and manual sequence alignment applied to different-sequence Alphabodies can be based on the maximization of global sequence identity or global sequence similarity (or homology or correspondence), or on the maximization of sequence identity or similarity of the core amino acid positions (i.e., the heptad a- and d- positions as defined herein) of the different-sequence Alphabodies.

Accordingly, a defined set of variegated amino acid residue positions can be deduced from an amino acid or nucleotide sequence comparison of all, or at least a representative subset of all different-sequence Alphabodies in an Alphabody library. It is also acknowledged that, upon generating an Alphabody library, so-called unintended mutations, insertions or deletions may occur. Such unintended mutations, insertions or deletions are nucleotide or amino acid mutations, insertions or deletions that occur at positions which were not intended to be variegated at the time of designing or generating the said Alphabody library. As will be acknowledged by a skilled person, and on condition that the said Alphabody library was generated with state-of-the-art technology, such unintended mutations, insertions or deletions will occur only sporadically and in a scattered fashion (i.e., at different positions) within the Alphabody library sequences. Preferably, such unintended mutations, insertions or deletions will occur at any given position with a frequency of less than 10%, more preferably less than 5%, more preferably less than 2%, 1%, or even less than 1%. Accordingly, unintended mutations, insertions or deletions within an Alphabody library can be identified on the basis of their low frequency as compared to the variability observed at positions showing intended sequence variation. Thus, a preferred method to determine a defined set of variegated amino acid residue positions within an Alphabody library of the invention, without possessing knowledge about the design or production of said library, is by determining the nucleotide or amino acid sequences of at least a representative subset of sequences contained within this library, followed by comparing all determined sequences in a (preferably multiple) alignment, followed by identifying the positions at which sequence variation is observed, followed by identifying the frequencies of the nucleotides or amino acid residue types observed at each variable position, followed by identifying the positions having a mutation, insertion or deletion frequency higher than a given cutoff percentage, wherein this cutoff percentage is set at 10%, 5%, 2%, 1% or even less than 1% such as 0.5%, 0.1% or 0% (in which case all observed variations are included in the defined set of variegated amino acid residue positions. Alternatively, if knowledge about the original design or production of a single-chain Alphabody library is available, then the set of variegated amino acid residue positions in this library is preferably based on said knowledge instead of on the analysis of the library itself. Methods to design single-chain Alphabody libraries, including the selection of variegated positions, are described below.

According to the methods of the present invention, the number of variegated amino acid residue positions in such a defined set can range from 5 to 20 amino acid residue positions. Thus, a defined set of variegated amino acid residue positions in a library of the invention may comprise 5 to 20, such as 5, 6, 7, 8, 9, 10, 11, 12, 13, 14, 15, 16, 17, 18, 19, or 20, defined variegated amino acid residue positions.

In the methods of the present invention, the different-sequence Alphabody polypeptides comprised in a library of the invention can differ from each other in at least one amino acid residue position of such a defined set of 5 to 20 positions. Thus, for example when the defined set of variegated amino acid residue positions in a library comprises a set of 13 variegated amino acid residue positions, the different-sequence Alphabody polypeptides in the libraries of the invention can differ from each other in 1, 2, 3, 4, 5, 6, 7, 8, 9, 10, 11, 12, or all 13 of these amino acid residue positions. Accordingly, it is clear that the different-sequence Alphabody polypeptide sequences comprised in a library of the invention can be distinguished from each other by the sequence difference(s) present in the defined set of 5 to 20 variegated amino acid residue positions, with the proviso that additional, preferably sporadic, unintended mutations, insertions or deletions may occur at positions other than those of the defined set of variegated positions.

Within the general Alphabody structure HRS1-L1-HRS2-L2-HRS3 which forms a coiled coil, the heptad repeat (HR) sequences 1, 2 and 3 each form an alpha-helix. These three helices (present in an Alphabody) that are formed respectively by the heptad repeat (HR) sequences 1, 2 and 3 are typically referred to as helices A, B and C, respectively The heptad repeat sequences comprise at least two heptad sequences, each of which is generally represented by 'abcdefg' or 'defgabc' (as further described herein).

Making use of the Alphabody scaffold, different types of Alphabody libraries can be generated, depending on the location of the set of variegated amino acids within the Alphabody structure. The location of the set of variegated amino acids determines the type and the location of the binding site that is generated on the Alphabodies within the library. Reference can be made in this context to 'groove libraries' (where binding sites are predominantly formed by amino acid residues located in the groove between two of the three helices of the Alphabody), 'surface libraries' (where binding sites are predominantly formed by amino acid residues located on the surface of one of the three helices of the Alphabody), or 'loop libraries' (where binding sites are predominantly formed by amino acid residues located in one or more of the loop or linker sequences of the Alphabody).

Libraries wherein the variegated amino acid residue positions are located exclusively (i.e. for 100%) in either a groove, surface or loop of the Alphabody are referred to herein as 'pure groove', 'pure surface', or 'pure linker' libraries. However, as will be detailed herein below, the methods envisage the use of Alphabody libraries, which need not be 'pure' groove, surface or linker libraries.

In one embodiment of the methods of the invention, Alphabody libraries are used wherein the variegated amino acid residue positions are located predominantly in the groove between two of the three helices of the Alphabody. As a result, in the Alphabodies obtained by the methods of the invention in these embodiments, the binding site for binding to a protein is formed predominantly by amino acid residue positions located in the groove between two of the three helices of the Alphabody.

Residues implicated in the formation of (the surface of) a groove between two adjacent alpha-helices in an Alphabody are generally located at the heptad e- and g-positions, but some of the more exposed b- and c-positions as well as some of the largely buried core a- and d-positions may also contribute to a groove surface; such will essentially depend on the size of the amino acid side-chains placed at these positions.

Depending on the nature of the Alphabodies generated (i.e. parallel or anti-parallel), the positions within the Alphabody sequence which need to be variegated in order to obtain a library which generates target binding Alphabodies wherein the binding site is located in the groove will vary.

When the two spatially adjacent alpha-helices of the Alphabody between which the groove for binding is formed run parallel, as is the case for helices A and B, A and C, or B and C in parallel Alphabodies and for helices A and C in anti-parallel Alphabodies, then the binding site located in the groove can be formed by at least e-residues from a first helix and g-residues from a parallel second helix. In addition to the e-residues from a first helix and the g-residues from a parallel second helix, the binding site may optionally further be formed by residues at b-positions in the first helix and/or residues at c-positions in the parallel second helix.

Thus, in these embodiments, in order to obtain Alphabodies directed against the protein of interest for which the binding site is located in the groove, the variegated amino acid residue positions of the Alphabody libraries used in the generation of target-specific Alphabodies are located at heptad e-positions in a first alpha-helix of the Alphabody polypeptides and at heptad g-positions in a second alpha-helix, parallel to the first alpha-helix, and optionally also at heptad b-positions in the first alpha-helix and/or at heptad c-positions in the parallel second alpha-helix of the Alphabody.

In particular embodiments of the invention, it is envisaged that Alphabody libraries are used wherein the variegated amino acid residue positions are predominantly (i.e. for at least 70%) located at heptad e-positions in a first alpha-helix of the Alphabody polypeptides and at heptad g-positions in a second alpha-helix, parallel to the first alpha-helix, and optionally at heptad b-positions in the first alpha-helix of the Alphabody polypeptides and/or at heptad c-positions in the parallel second alpha-helix of the Alphabody polypeptides.

Alternatively or additionally, use can be made of Alphabody scaffolds comprising two spatially adjacent alpha-helices that are positioned anti-parallel (if a binding site located in the groove between these helices is envisaged). This is typically the case for helices A and B or B and C in anti-parallel Alphabodies. In these embodiments, there are typically two possibilities for the type of variegated amino acid residue positions that need to be variegated to ensure that the binding site is formed by the groove.

In a first possibility, the groove between two anti-parallel helices is formed by at least e-residues from a first helix and e-residues from an anti-parallel second helix. Thus, in order to obtain a binding site formed within the groove, at least some of these amino acid residue positions are variegated. In addition to the e-residues from a first helix and the e-residues from an anti-parallel second helix, such a binding site may optionally further be formed by residues at b-positions in the first helix and/or residues at b-positions in the anti-parallel second helix.

Thus, in these embodiments, in order to obtain Alphabodies of the present invention for which the binding site is located in the groove, Alphabody libraries are used wherein the variegated amino acid residue positions are located at heptad e-positions in a first alpha-helix of the Alphabody and at heptad e-positions in a second alpha-helix, anti-parallel to the first alpha-helix, and optionally at heptad b-positions in the first alpha-helix of the Alphabody and/or at heptad b-positions in the second alpha-helix of the Alphabody polypeptides.

In particular embodiments of the invention, it is envisaged that Alphabody libraries are used wherein the variegated amino acid residue positions are predominantly (i.e. for at least 70%) located at heptad e-positions in a first alpha-helix of the Alphabody and at heptad e-positions in a second alpha-helix, anti-parallel to the first alpha-helix, and optionally at heptad b-positions in the first alpha-helix of the Alphabody and/or at heptad b-positions in the second alpha-helix of the Alphabody.

In antiparallel Alphabody scaffolds, another groove is formed by at least g-residues from a first helix and g-residues from an anti-parallel second helix. In addition to the g-residues from a first helix and the g-residues from an anti-parallel second helix, such a binding site may optionally further be formed by residues at c-positions in the first helix and/or residues at c-positions in the anti-parallel second helix.

Thus, in these embodiments, in order to obtain Alphabodies of the present invention for which the binding site is located in the groove, Alphabody libraries are used wherein the variegated amino acid residue positions are located at heptad g-positions in a first alpha-helix of the Alphabody and at heptad g-positions in a second alpha-helix, anti-parallel to the first alpha-helix, and optionally at heptad c-positions in the first alpha-helix of the Alphabody and/or at heptad c-positions in the second alpha-helix of the Alphabody.

In particular embodiments of the invention, it is envisaged that Alphabody libraries are used wherein the variegated amino acid residue positions are predominantly (i.e. for at least 70%) located at heptad g-positions in a first alpha-helix of the Alphabody and at heptad g-positions in a second alpha-helix, anti-parallel to the first alpha-helix, and optionally at heptad c-positions in the first alpha-helix of the Alphabody and/or at heptad c-positions in the second alpha-helix of the Alphabody.

The three types of possible groove binding sites formed by variegated amino acid residue positions in the libraries used in the methods of the invention are herein denoted by their primary groove-forming (i.e. e- and g-) residue positions.

Accordingly, if the two adjacent helices forming the binding groove are oriented parallel with respect to each other, then the groove is referred to as an e/g-groove; if those two helices are oriented anti-parallel with respect to each other, then the groove is referred to as either an e/e-groove or a g/g-groove.

Thus, it will be clear that parallel Alphabodies, which have three alpha-helices each oriented parallel to one another, contain three e/g-grooves. Anti-parallel Alphabodies, on the other hand, which comprise two alpha-helices that are parallel to each other and one alpha-helix that runs anti-parallel, contain one e/g-groove, one e/e-groove and one g/g-groove. Any part of an Alphabody groove is also considered a groove region.

In principle, 'pure' groove libraries are Alphabody libraries characterized in that the variegated amino acid residue positions in such libraries are all located at heptad e- or g-positions in a first alpha-helix of the Alphabody and at heptad e- or g-positions in a second alpha-helix, and optionally at heptad b- or c-positions in the first alpha-helix of the Alphabody and/or at heptad b- or c-positions in the second alpha-helix of the Alphabody.

It has been found by the present inventors that Alphabody libraries which are characterized in that the variegated amino acid residue positions in such libraries are not exclusively but only predominantly located at heptad e- or g-positions in a first alpha-helix of the Alphabody and at heptad e- or g-positions in a second alpha-helix, and optionally at heptad b- or c-positions in the first alpha-helix of the Alphabody and/or at heptad b- or c-positions in the second alpha-helix of the Alphabody, generate more and better target-specific Alphabody polypeptides. Accordingly, the methods of the present invention specifically envisage the use of Alphabody libraries wherein the variegated amino acid residue positions in such libraries, are predominantly (i.e. for at least 70%), but not exclusively, located at heptad e- or g-positions in a first alpha-helix of the Alphabody and at heptad e- or g-positions in a second alpha-helix, and optionally at heptad b- or c-positions in the first alpha-helix of the Alphabody and/or at heptad b- or c-positions in the second alpha-helix of the Alphabody. In particular embodiments, the variegated amino acid residue positions in the Alphabody libraries used, are located for at least 70% at the indicated groove-forming positions. In further particular embodiments, at least one of the variegated amino acid residue positions in the libraries is located outside the positions corresponding to the heptad e- or g-positions in a first alpha-helix of the Alphabody and heptad e- or g-positions in a second alpha-helix, and heptad b- or c-positions in the first alpha-helix of the Alphabody and heptad b- or c-positions in the second alpha-helix of the Alphabody. It will be clear to the skilled person that throughout the application, when reference is made to helix positions, it is intended to refer to the corresponding helix of the Alphabody, optionally present in an Alphabody polypeptide.

Additionally or alternatively, it is envisaged that the methods of the present invention comprise the use of Alphabody libraries in which the binding site is formed predominantly by the surface of one of the three helices of the Alphabody. Accordingly, in these embodiments, Alphabody libraries are used in which the variegated amino acid residue positions are located predominantly on the surface of one or more of the three helices of the Alphabody. Such Alphabody libraries are referred to herein as 'surface libraries'.

The amino acid residue positions considered to be at the surface of the Alphabody can be located in either helix A, B or C. The most protruding and thus solvent-oriented residues are located at b-, c- and f-positions in each of the alpha-helices of an Alphabody. Thus, in particular embodiments, the Alphabody libraries of the invention comprise variegated amino acid residue positions that are located at heptad b-, c- and f-positions in one alpha-helix of the Alphabody.

In principle, 'pure' surface libraries are Alphabody libraries characterized in that the variegated amino acid residue positions in such libraries are all located at heptad b-, c- and f-positions in one alpha-helix of the Alphabody.

It has been found by the present inventors that Alphabody libraries which are characterized in that the variegated amino acid residue positions are not exclusively but only predominantly located at heptad b-, c- and f-positions of an alpha-helix of the Alphabody, generate more and better target-specific Alphabodies. Accordingly, the methods of the present invention specifically envisage the use of Alphabody libraries wherein the variegated amino acid residue positions in such libraries are predominantly (i.e. for at least 70%), but not exclusively located at heptad b-, c- and f-positions of an alpha-helix of the Alphabody. In particular embodiments, the variegated amino acid residue positions in the Alphabody libraries used, are located for at least 70% at the indicated solvent-exposed positions. In further particular embodiments, at least one of the variegated amino acid residue positions in the libraries, is located outside the positions corresponding to heptad b-, c- and f-positions of an alpha-helix of the Alphabody.

Additionally or alternatively, it is envisaged that the methods of the present invention comprise the use of Alphabody libraries in which the binding site is formed predominantly by amino acid residue positions located in one or more of the loop or linker sequences interconnecting the alpha-helices of the Alphabody.

The amino acid residue positions located in a loop correspond to the amino acid positions present in one or more of the flexible linkers between the heptad repeat sequences. The two loops of Alphabodies in the anti-parallel orientation (i.e. loop AB and loop BC) are positioned or located at opposite sides of the Alphabody coiled coil. Either of these loops can be used for introducing sequence variation at certain variegated positions. In particular embodiments, libraries are used wherein all of the amino acid residue positions of a loop are variegated. In further particular embodiments, a selection of the residue positions located in the centre or middle of this loop are variegated. In further particular embodiments, alternating residue positions over the complete length of this loop sequence are variegated.

'Pure' linker or loop libraries are Alphabody libraries characterized in that the variegated amino acid residue positions in such libraries are all located within a linker of the Alphabody.

As indicated above, it has been found by the present inventors that Alphabody libraries which are characterized in that the variegated amino acid residue positions are not exclusively but only predominantly located at linker positions in the Alphabody, generate more and better target-specific Alphabodies. Accordingly, the methods of the present invention specifically envisage the use of Alphabody libraries wherein the variegated amino acid residue positions in such libraries are predominantly (i.e. for at least 70%), but not exclusively located in one of the linkers of the Alphabody. In particular embodiments, the variegated amino acid residue positions in the Alphabody libraries used, are located for at least 70% at the indicated linker positions. In further particular embodiments, at least one of the variegated amino acid residue positions in the libraries is located outside the amino acid residue positions in a linker or loop of an alpha-helix of the Alphabody.

Thus, in particular embodiments of the methods of the invention, Alphabody libraries are used which are characterized in that the set of variegated amino acids contains at least one, more particularly two or more amino acid residue positions which are located outside respectively, the groove, surface or loop of an Alphabody such that the binding site is predominantly but not exclusively formed respectively by that groove, surface or loop. In these embodiments, the percentage of variegated amino acid positions within the groove, surface or loop (i.e. linker) of an Alphabody having a binding site that is predominantly formed by that groove, surface or loop, respectively, is less than 100%. However, the percentage of variegated amino acid positions that is located within the groove, surface or loop (i.e. linker) is typically at least 70%.

More particularly, in some embodiments of the present invention, the libraries used are not pure groove, surface or loop libraries. More particularly at least 70% but not all of the variegated amino acid positions, such as for example less than 95%, such as less than 90%, or less than 85% of the variegated amino acid positions are located in either a groove, surface or loop of the Alphabody.

Depending on the number of amino acid residue positions variegated, the percentages described above will correspond to a different number of actual amino acid residue positions. Accordingly, as will be clear from the above, in particular embodiments of the methods of the invention Alphabody libraries are used in which for example at least 5% (i.e. at least 1 position of the 5 to 20), or particularly at least 10% (i.e. at least 1 or at least 2 positions), or particularly at least 15% (i.e. at least 1 to at least 3 positions), or particularly at least 20% (i.e. at least 1 to at least 4 positions), or particularly at least 25% (i.e. at least 1 to at least 5 positions), or particularly 30% (i.e. between 2 and 6 positions) of these 5 to 20 positions are located at positions other than:
(i) at heptad e- or g-positions in a first alpha-helix of the Alphabody and at heptad e- or g-positions in a second alpha-helix, and optionally at heptad b-or c-positions in the first alpha-helix of the Alphabody and/or at heptad b- or c-positions in the second alpha-helix of the Alphabody, such as
   (i1) at heptad e-positions in a first alpha-helix of the Alphabody and at heptad g-positions in a second alpha-helix, parallel to the first alpha-helix, and optionally at heptad b-positions in the first alpha-helix of the Alphabody and/or at heptad c-positions in the second alpha-helix of the Alphabody,
      or
   (i2) at heptad e-positions in a first alpha-helix of the Alphabody and at heptad e-positions in a second alpha-helix, anti-parallel to the first alpha-helix, and optionally at heptad b-positions in the first alpha-helix of the Alphabody and/or at heptad b-positions in the second alpha-helix of the Alphabody,
      or
   (i3) at heptad g-positions in a first alpha-helix of the Alphabody and at heptad g-positions in a second alpha-helix, anti-parallel to the first alpha-helix, and optionally at heptad c-positions in the first alpha-helix of the Alphabody and/or at heptad c-positions in the second alpha-helix of the Alphabody
   or
(ii) at heptad b-, c- and f-positions in one alpha-helix of the Alphabody,
   or
(iii) at positions in a linker fragment connecting two consecutive alpha-helices of the Alphabody.

Accordingly, the different-sequence (single-chain) Alphabodies in a library differ in a defined set of 5 to 20 amino acid residue positions, wherein, for each library at least 70% (i.e. at least 4 to at least 14 positions) of these 5 to 20 positions, such as at least 75% (i.e. at least 4 to at least 15 positions), at least 80% (i.e. at least 4 to at least 16 positions), at least 85% (i.e. at least 4 to at least 17 positions), such as at least 90% (i.e. at least 5 to at least 18 positions), for example at least 95% (i.e. at least 5 to at least 19 positions), or more, such as 100% (i.e. all 5 to 20 positions) are located
(i) in the groove formed by or between two adjacent alpha-helices, or
(ii) on the surface in one alpha-helix or
(iii) in one or both of the loops/linker fragments interconnecting two alpha-helices of each Alphabody in the library;
more particularly these positions are located at the positions recited for each of the options above.

It is noted that for the Alphabody libraries wherein the variegated amino acid residue positions are located primarily (i.e. at least 70%) in the groove, the remaining variegated positions that are located elsewhere than at the indicated heptad e-, g-, b- or c-positions may for example be located at heptad a-positions, at heptad d-positions, at heptad f-positions or at amino acid residue positions in the linkers or loops of the Alphabody that interconnect the alpha-helices. The a-positions and d-positions (also referred to as core residues) in each heptad unit of an Alphabody of the invention are amino acid residue positions of the coiled coil structure which form essentially the solvent-shielded (i.e. buried) part of the Alphabody. It is envisaged that in most 'groove' Alphabody libraries used in the context of the present invention, all or some of these core residues are kept conserved in order to maintain the stability of the Alphabody. In these embodiments, the remaining variegated residues can be located for instance at linker residue positions or heptad f-positions.

Similarly for the Alphabody libraries wherein the variegated amino acid residue positions are located primarily (i.e. at least 70%) at the surface of an Alphabody helix, the remaining variegated positions that are not located at heptad b-, c-, or f-positions, may for example be located at heptad e-, g-, a- or d-positions or at amino acid residue positions in the linkers or loops of the Alphabody that interconnect the alpha-helices. As detailed above, it is envisaged that in most 'surface' Alphabody libraries used in the context of the present invention, all or some of the core residues (at a- and d-positions) are kept conserved in order to maintain the stability of the Alphabody. In these embodiments of the invention, the remaining variegated positions may be located for instance at heptad e- or g-positions or at linker residue positions.

Finally, for the Alphabody libraries wherein the variegated amino acid residue positions are located primarily (i.e. at least 70%) in a loop or linker sequence of the Alphabody, the remaining variegated positions that are not located in the linker or loop may for example be located at heptad e-, g-, f-, b-, c-, a- or d-positions or at amino acid residue positions in the other of the two linkers or loops of the Alphabody. Again, the a-positions and d-positions in each heptad unit of an Alphabody will in some embodiments not be varied to ensure stability. In these embodiments, the remainder of the variegated amino acid residue positions may be located at heptad e-, g-, f-, b-, or c-positions or at amino acid residue positions in the other of the two linkers or loops of the Alphabody.

When referring to variegated amino acid residue positions located in the groove formed by or between two adjacent alpha-helices of an Alphabody, it is meant that these variegated amino acid residue positions are located in one and the same groove, i.e. formed by the two same adjacent alpha-helices of the Alphabodies comprised in a library.

Similarly, when referring to variegated amino acid residue positions located in one alpha-helix of the Alphabody, it is meant that these variegated amino acid residue positions are located in one and the same alpha-helix of the Alphabodies comprised in a library.

Also, when referring to variegated amino acid residue positions located at positions in one or more linker or loop fragments interconnecting two consecutive alpha-helices, it is meant that these variegated amino acid residue positions are located in the same one or more linker or loop fragments interconnecting the same two consecutive alpha-helices in the Alphabodies comprised in a library.

However, it can be envisaged that different groove, surface or loop libraries are combined for use in the methods of the present invention. More particularly, it can be envisaged that a surface library comprising variegated amino acids primarily located on the surface of helix C is combined with a surface library comprising variegated amino acids primarily located on the surface of helix A.

Thus in certain particular embodiments of the methods of the present invention, a mixture of Alphabody libraries can be used comprising 2 to 6 different constituting libraries.

The libraries used in the context of the present invention contain sequence variations between Alphabody polypeptides, wherein this sequence variation exclusively resides in 5 to 20 defined amino acid residue positions, of which at least 70% of these positions are located either in a groove formed between two adjacent alpha-helices, or in one alpha-helix or at positions in a loop (fragment) or linker (fragment) connecting two consecutive alpha-helices of the Alphabody. Indeed, given the unique structural features of the Alphabody scaffold, two conceptually different types of randomization, namely in the coiled-coil region and in the unstructured linkers between helices, can be designed.

In particular embodiments of the present invention, the constant, non-variegated part of the single-chain Alphabodies that are present in the single-chain Alphabody libraries do not correspond to a naturally occurring protein sequence, and thus the sequence representing the non-variegated part or scaffold is of non-natural origin. Indeed, typically, the constant, non-variegated part of the Alphabodies in a library of the invention is an artificial sequence.

It will be understood by the skilled person that the Alphabody libraries comprising the variegations as described above, for use in the methods of the present invention, are typically generated by recombinant DNA techniques. More particularly, libraries of nucleic acid sequences encoding Alphabodies each differing in particular amino acid positions can be obtained by site-directed or random mutagenesis of a template sequence. As will be acknowledged by a skilled person, random amino acid residues can be introduced at specific positions in an amino acid sequence by, for example, selecting (introducing) 'NNK' or 'NNS' codons at corresponding positions in the nucleotide sequence encoding said amino acid sequence.

Thus, the generation of a (partially) randomized single-chain Alphabody library requires the (partial) randomization of specific positions within a template or standard or reference Alphabody scaffold sequence. Such methods for producing libraries are known to the skilled person and commercial services are available for generating such libraries. The nucleotide(s) determining the relevant amino acid residues in the positions of interest are mutated in different ways such as to obtain a library of sequences encoding different Alphabodies.

A template Alphabody scaffold sequence is the sequence of a reference Alphabody which has been selected on the basis of its (near-) optimal physico-chemical properties. As demonstrated in the examples in WO 2010/066740, single-chain Alphabodies generally have a high thermal (i.e., thermodynamic) stability, a high solubility, a high resistance to variations in pH, and, importantly, a high tolerability to amino acid sequence variation.

The variegation envisaged in the libraries used in the methods of the invention, is envisaged to encompass both naturally occurring and synthetic amino acid residues. However, in particular embodiments of the invention, the variegated amino acid residue positions, i.e. wherein the different-sequence Alphabody polypeptides comprised in the libraries of the invention differ from each other, are exclusively occupied by naturally occurring amino acid typessuch as glycine, alanine, proline, asparagine, aspartic acid, glutamine, glutamic acid, histidine, arginine, lysine, threonine, serine, cysteine, leucine, isoleucine, methionine, phenylalanine, tyrosine, tryptophan and valine.

In order to obtain the cytokine or growth factor or cytokine- or growth factor-receptor binding Alphabodies of the present invention, the methods of the invention further comprise the step of selecting from the single-chain Alphabody library at least one single-chain Alphabody having detectable binding affinity for, or detectable in vitro activity on, the cytokine or growth factor and/or the cytokine or growth factor receptor of interest.

Screening methods for the binding of an Alphabody to a target protein are known in the art and will be detailed below. It is noted that the screening of an Alphabody library may be performed in different ways, and that the screening method will be adjusted to the form in which the Alphabody library is provided.

Indeed, the Alphabody libraries used in the methods of the present invention can be provided in different forms, and can be but are not limited to protein libraries, nucleic acid libraries, vector libraries or host cell libraries.

In particular embodiments, the libraries used in the context of the present invention are libraries of host cells, wherein each host cell comprises at least one member of a nucleic acid or vector library encoding a single-chain Alphabody library of the invention, or a mixture of single-chain Alphabody libraries of the invention. More particularly, the libraries are libraries of host cells wherein Alphabodies are expressed.

In particular embodiments, the Alphabodies of the library are displayed on the surface of a phage particle, a ribosome, a bacterium, a yeast cell, a mammalian cell or any other suitable (micro)organism, so as to facilitate screening or selection to isolate the desired Alphabody sequences having detectable binding affinity for, or detectable in vitro activity on, the protein of interest. Suitable methods, techniques and host organisms for displaying and selecting or screening (a set, collection or library of) variegated polypeptide sequences or nucleotide sequences encoding such variegated polypeptide sequences, and which are applicable to Alphabodies, are known to the person skilled in the art. Such methods are described, for example, in Georgiou et al., Nat Biotechnol, 15:29-34, 1997; Wittrup, Curr Opin Biotechnol, 12:395-399, 2001; Lipovsek and Pluckthun, J Immunol Methods 290:51-67, 2004; Reiersen et al., Nucl Acids Res, 33:e10, 2005; Levin and Weiss, Mol BioSyst, 2:49-57, 2006; Bratkovic, Cell Mol Life Sci 67:749-767, 2010.

For example, the technology of phage library display, and the selection by means of a phage display technique may be chosen as a method for high-throughput identification of protein-specific binders, because it is one of the most robust and versatile selection techniques available (Scott and Smith, Science, 249:386-390, 1990; Bratkovic, Cell Mol Life Sci 67:749-767, 2010). A major advantage of this technology is the coupling of genotype (i.e., the encapsulated DNA encoding the displayed protein) and phenotype (i.e., the displayed protein such as an Alphabody of the present invention) which allows affinity-based selection from large libraries with millions to trillions of polypeptide variants in a relatively simple in vitro assay.

In certain particular embodiments, the methods of the present invention may further comprise the step of isolating the single-chain Alphabody library. This can be ensured by expressing the nucleic acid or vector library under suitable conditions and isolating the produced Alphabodies from the host cells and/or from the medium.

In certain particular embodiments, the present invention provides methods for the production of at least one single-chain Alphabody polypeptide having detectable binding affinity for, or detectable in vitro activity on, a cytokine or growth factor and/or cytokine or growth factor receptor of interest, at least comprising the step of producing a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
(i) at heptad e- or g-positions in a first alpha-helix of the Alphabody polypeptides and at heptad e- or g-positions in a second alpha-helix, and optionally at heptad b- or c-positions in the first alpha-helix of the Alphabody polypeptides and/or at heptad b- or c-positions in the second alpha-helix of the Alphabody polypeptides, such as, more particularly:
   (i1) at heptad e-positions in a first alpha-helix of the Alphabody polypeptides and at heptad g-positions in a second alpha-helix, parallel to the first alpha-helix, and optionally at heptad b-positions in the first alpha-helix of the Alphabody polypeptides and/or at heptad c-positions in the second alpha-helix of the Alphabody polypeptides,
      or
   (i2) at heptad e-positions in a first alpha-helix of the Alphabody polypeptides and at heptad e-positions in a second alpha-helix, anti-parallel to the first alpha-helix, and optionally at heptad b-positions in the first alpha-helix of the Alphabody polypeptides and/or at heptad b-positions in the second alpha-helix of the Alphabody polypeptides,
      or
   (i3) at heptad g-positions in a first alpha-helix of the Alphabody polypeptides and at heptad g-positions in a second alpha-helix, anti-parallel to the first alpha-helix, and optionally at heptad c-positions in the first alpha-helix of the Alphabody polypeptides and/or at heptad c-positions in the second alpha-helix of the Alphabody polypeptides
   or
(ii) at heptad b-, c- and f-positions in one alpha-helix of the Alphabody polypeptides, or
(iii) at positions in a linker fragment connecting two consecutive alpha-helices of the Alphabody polypeptides.

For example, the production of a single-chain Alphabody library may involve the step of producing a nucleic acid or vector library of at least 100 different-sequence members, wherein each member encodes a polypeptide comprising a single-chain Alphabody and wherein the encoded different-sequence Alphabody polypeptides differ from each other in at least one of a defined set of 5 to 20 variegated amino acid residue positions. In particular embodiments, at least 70% but not all of these variegated amino acid residue positions are located either:
(i) at heptad e- or g-positions in a first alpha-helix of the Alphabody polypeptides and at heptad e- or g-positions in a second alpha-helix, and optionally at heptad b- or c-positions in the first alpha-helix of the Alphabody polypeptides and/or at heptad b- or c-positions in the second alpha-helix of the Alphabody polypeptides, or
(ii) at heptad b-, c- and f-positions in one alpha-helix of the Alphabody polypeptides, or
(iii) at positions in a linker fragment connecting two consecutive alpha-helices of the Alphabody polypeptides.
Upon expression of these Alphabody sequences in suitable host cells, an Alphabody polypeptide library is obtained. Thus, the production of a random library can be achieved in different ways, as will be known by the person skilled in the art.
For example, using a phagemid display (Kay et al., 'Phage Display of Peptides and Proteins. A Laboratory Manual, B.K. Kay et al. 1996, ISBN 0-12-402380-0) a given Alphabody library may be represented by a collection of phagemids each of which encodes for a fusion protein comprising a member of the Alphabody library fused to the minor coat protein pIII. These phagemids can be introduced into suitable E. coli cells (e.g. TG1) by electroporation or other means. Using infection with helper phage, phage are produced (packaging also the phagemid genome) that display the Alphabody-fusion protein. These phage can be used to select binders against a given target and the selected phage can be propagated by infecting E. coli TG1 (Stratagene),

Thus in addition, the methods for the production of at least one single-chain Alphabody having detectable binding affinity for, or detectable in vitro activity on, a cytokine or growth factor and/or cytokine or growth factor receptor of interest typically comprise the step of expressing said nucleic acid or vector library under conditions suitable for the production of said single-chain Alphabody library.

In certain embodiments of these production methods, the step of expressing the nucleic acid or vector library, comprises introducing the nucleic acid or vector library into host cells such that preferably each host cell contains at most one element or molecule of said nucleic acid or vector library and culturing the host cells in a medium under conditions suitable for the production of the single-chain Alphabody library.

### [SCREENING STEPS FOR GENERATING TARGET-SPECIFIC ALPHABODIES]

In addition, the methods of the present invention for the production of at least one single-chain Alphabody having detectable binding affinity for, or detectable in vitro activity on, a cytokine or growth factor and/or cytokine or growth factor receptor of interest at least comprise the further step of selecting from said single-chain Alphabody library at least one single-chain Alphabody having detectable binding affinity for, or detectable in vitro activity on, said cytokine or growth factor and/or cytokine or growth factor receptor of interest.

It will be understood that the selection step of the methods described herein can be performed by way of a method commonly known as a selection method or a by way of a method commonly known as a screening method. Both methods envisage the identification and subsequent isolation (i.e., the selection step) of desirable components (i.e., Alphabody , library members) from an original ensemble comprising both desirable and non-desirable components (i.e., an Alphabody library). In the case of a selection method, library members will typically be isolated by a step wherein the desired property is applied to obtain the desired goal; in such case, the desired property is usually restricted to the property of a high affinity for a given cytokine or growth factor and/or cytokine or growth factor receptor of interest and the desired goal is usually restricted to the isolation of such high-affinity library members from the others. Such method is generally known as an affinity selection method and, in the context of the present invention, such affinity selection method will be applied to a single-chain Alphabody library for the purpose of selecting Alphabodies having a high affinity for a cytokine or growth factor and/or cytokine or growth factor receptor of interest or a subdomain or subregion thereof. Equally possible is to select for kinetic properties such as e.g. high on-rate for binding to a given cytokine or growth factor and/or cytokine or growth factor receptor of interest, or low off-rate for library members bound to said target by adjusting the appropriate selection conditions (e.g. short incubation times or long wash cycles, or other conditions as is known by someone skilled in the art of library selection techniques). Alternatively, in the case of a screening method, library members will typically be isolated by a step wherein all library members, or at least a substantial collection of library members, are individually examined with respect to a given desired property, and wherein members having such desired property are retained whereas members not having such desired property are discarded; in such case, and in the context of the present invention, desired properties may relate to either a high affinity for a cytokine or growth factor and/or cytokine or growth factor receptor of interest or a subdomain or subregion thereof, or a functional activity such as an anti-cytokine or anti-growth factor activity, including the inhibition, reduction and/or prevention of the activity of a cytokine or growth factor and/or cytokine or growth factor receptor of interest. Accordingly, it is submitted that the selection step of the methods of the invention may be accomplished by either an (affinity) selection technique or by an affinity-based or activity-based functional screening technique, both techniques resulting in the selection of one or more single-chain Alphabodies having beneficial (favorable, desirable, superior) affinity or activity properties compared to the non-selected Alphabodies of the single-chain Alphabody library of the invention.

In particular embodiments, the selection step comprises contacting the single-chain Alphabody library of the invention or a mixture of single-chain Alphabody libraries of the invention with a cytokine or growth factor and/or cytokine or growth factor receptor of interest and determining binding between the target molecule and an Alphabody present in the library.

Thus, in particular embodiments, the methods for the production of target-specific Alphabodies comprise the step of identifying from the single-chain Alphabody library or mixture of single-chain Alphabody libraries being contacted with the target molecule of interest (i.e. cytokine or growth factor and/or cytokine or growth factor receptor or fragment thereof), the one or more single-chain Alphabodies having detectable binding affinity for the cytokine or growth factor and/or cytokine or growth factor receptor of interest. Specific binding of an Alphabody to a target molecule or protein of interest can be determined in any suitable manner known per se, including, for example biopanning, Scatchard analysis and/or competitive binding assays, such as radioimmunoassays (RIA), enzyme immunoassays (EIA) and sandwich competition assays, and the different variants thereof known in the art.

Biopanning is a well known iterative selection/screening method to enrich an initial population of different molecules (such as an Alphabody library) for molecules having an affinity for the target of choice. Most often the initial population of molecules is displayed on a typical display vehicle such as bacteriophage but the technique is certainly not limited to this type of display. The target is typically immobilized in the direct biopanning protocol. The immobilization of the target can be performed by many different methods known in the art. Examples of solid support are microtiter plates or tubes (e.g. Maxisorp plates, Maxisorp tubes, Nunc) or magnetic beads (Dynabeads, Invitrogen). The target can either be directly coated on the plastic or the beads (surface activated Dynabeads, e.g. Dynabeads M270 Epoxy, Invitrogen) or via streptavidin when the target is biotinylated (e.g. Dynabeads MyOne Streptavidin T1, Invitrogen).

Other tags can be used to capture the targets such as His-tags or alternatively, an antibody directed against the target can also be used to capture the target on the support. These alternative tags are also compatible with the Dynabeads (Dynabeads His-tag isolation and pull down, Invitrogen) and Protein A or Protein G coupled Dynabeads (Dynabeads-Protein A/G, Invitrogen). To immobilize the target on magnetic beads, the recommendations of the manufacturer are followed for each specific bead type.

In the soluble biopanning protocol, the target is captured on the solid support after incubation with the phage library. For the avoidance of doubt, this capturing can be indirect. For example, during the incubation phase in solution the target may be bound to e.g. a biotinylated compound that binds to a region of the target that is of no particular biological interest. The capturing step may then consists of trapping this biotinylated compound to streptavidin coated magnetic beads, thereby capturing indirectly phage bound to the target. The target-phage interaction is performed in solution. To be able to wash away the non-binding phage, the target needs to be immobilized on a solid support. The immobilization of the target in the soluble biopanning method is identical to the immobilization possibilities in the direct biopanning protocols.

A classical biopanning protocol consists of 2, 3 to 5 or more screening rounds, depending on the type of target and library. Each selection round consists of typically different steps: (1) immobilization of the target of choice to a support. This step is optional, as biopanning can also be performed in a format wherein the target is not-immobilized but kept in solution (in case of soluble target) or remains anchored on a cell (in case of e.g. a membrane anchored receptor), (2) incubation of the library with the target, (3) washing steps to eliminate non-specific binders, (4) optionally elution of the binders and (5) amplification of the eluted binders from step (4) or from step (3) (in case step (4)) was omitted in consecutive screening rounds). The steps 1 to 5 will be repeated two, three, four or more times to isolate from the initial library target-specific binders. After the biopanning, the target-specificity of the binders isolated from the different selection rounds is typically analyzed in ELISA assays or similar assays.
The most commonly used types of display libraries for selection or screening are libraries wherein the library members are displayed in a format where the displayed peptide or protein is attached, fused or anchored to a vehicle (phage, cell, RNA, etc) that contains the coding information of the displayed peptide or protein.

Thus, in particular embodiments, the Alphabody libraries used in the context of the invention are provided as a phage library and binding Alphabodies are identified by contacting the phage with the labeled target molecule, after which binding phages are retrieved by detection or selective collection of the labeled, bound target. Typically, a biotinylated target can be used, whereby phage which generate an Alphabody binding to the target are captured with a streptavidin-coated support (e.g. magnetic beads).

In particular embodiments of the present invention, the selection steps of the methods for producing one or more single-chain Alphabodies having detectable binding affinity (as defined herein) for a protein of interest, may comprise the (further) enrichment of the Alphabody library or the mixture of Alphabody libraries for single-chain Alphabodies having detectable binding affinity for the protein of interest by iterative execution of the steps of contacting a protein of interest with a single-chain Alphabody library or with a mixture of single-chain Alphabody libraries of the invention and subsequently identifying from the single-chain Alphabody library or mixture of single-chain Alphabody libraries being contacted with the protein, the one or more single-chain Alphabodies having detectable binding affinity for the protein of interest.

The step of selecting a single-chain Alphabody that has detectable in vitro activity by interacting with a target protein of interest typically comprises:
a) contacting a single-chain Alphabody library or a mixture of single-chain Alphabody libraries of the invention with the cytokine or growth factor or cytokine or growth factor receptor of interest, or a fragment thereof and
b) identifying from single-chain Alphabody library or mixture of single-chain Alphabody libraries, the one or more single-chain Alphabodies having detectable in vitro activity on the cytokine or growth factor or cytokine or growth factor receptor protein of interest.

More particularly the cytokine or growth factor receptor may be a membrane anchored receptor, a soluble receptor or a molecule comprising one or more ectodomains of said cytokine or growth factor receptor.

More particularly, in the context of the present invention, the effect on the activity of a cytokine or growth factor or on the activity of a cytokine or growth factor receptor can be measured by ways known in the art. More specifically this involves determining the effect of the Alphabody on a known cytokine-mediated or growth factor-mediated effect in vitro. For instance, the effect of an Alphabody directed to a chemokine of interest can be measured in a chemotaxis assay using chemotactic cells. Also, for example, the effect of an Alphabody directed to a cytokine or growth factor of interest may be measured by incubating said cytokine or growth factor with a given target cell which for its activity, growth or differentiation is dependent (positively or negatively) on said cytokine or growth factor and determining the target cell biological activity (metaboloic, growth, differentiation, apoptosis, or other functional property) in absence or presence of Alphabody.

It will be understood that the selection methods described herein can also be performed as screening methods. Accordingly the term 'selection' as used in the present description can comprise selection, screening or any suitable combination of selection and/or screening techniques.

### [ISOLATION]

In some cases, the methods of the present invention may further comprise the step of isolating from the single-chain Alphabody library at least one single-chain Alphabody having detectable binding affinity for, or detectable in vitro activity on, a cytokine or growth factor and/or cytokine or growth factor receptor of interest.

The methods of the present invention may further comprise the step of amplifying at least one single-chain Alphabody having detectable binding affinity for, or detectable in vitro activity on, a cytokine or growth factor and/or cytokine or growth factor receptor of interest. For example, a phage clone displaying a particular single-chain Alphabody, obtained from a selection step of a method of the invention, may be amplified by reinfection of a host bacteria and incubation in a growth medium.

In particular embodiments, the methods of the present invention encompass determining the sequence of the Alphabody or Alphabodies capable of binding to the cytokine or growth factor and/or cytokine or growth factor receptor.

Where an Alphabody polypeptide sequence, comprised in a set, collection or library of Alphabody polypeptide sequences, is displayed on a suitable cell or phage or particle, it is possible to isolate from said cell or phage or particle, the nucleotide sequence that encodes that Alphabody polypeptide sequence. In this way, the nucleotide sequence of the selected Alphabody library member(s) can be determined by a routine sequencing method.

In further particular embodiments, the methods of the invention comprise the step of expressing said nucleotide sequence(s) in a host organism under suitable conditions, so as to obtain the actual desired Alphabody polypeptide sequence(s). This step can be performed by methods known to the person skilled in the art.

In addition, the obtained Alphabody sequences having detectable binding affinity for, or detectable in vitro activity on, a cytokine or growth factor and/or cytokine or growth factor receptor of interest, may be synthesized as soluble protein construct, optionally after their sequence has been identified.

For instance, the Alphabodies obtained, obtainable or selected by the methods of the present invention can be synthesized using recombinant or chemical synthesis methods known in the art. Also, the Alphabodies obtained, obtainable or selected by the methods of the present invention can be produced by genetic engineering techniques. Thus, methods for synthesizing an Alphabody obtained, obtainable or selected by the methods of the present invention may comprise transforming or infecting a host cell with a nucleic acid or a vector encoding an Alphabody sequence having detectable binding affinity for, or detectable in vitro activity on, a cytokine or growth factor and/or cytokine or growth factor receptor of interest. Accordingly, the Alphabody sequences having detectable binding affinity for, or detectable in vitro activity on, a cytokine or growth factor and/or cytokine or growth factor receptor of interest can be made by recombinant DNA methods. DNA encoding the Alphabodies can be readily synthesized using conventional procedures. Once prepared, the DNA can be introduced into expression vectors, which can then be transformed or transfected into host cells such as E. coli or any suitable expression system, in order to obtain the expression of Alphabodies in the recombinant host cells and/or in the medium in which these recombinant host cells reside.

It should be understood, as known by someone skilled in the art of protein expression and purification, that the Alphabody produced from an expression vector using a suitable expression system may be tagged (typically at the N-terminal or C-terminal end of the Alphabody) with e.g. a Histidine or other sequence tag for easy purification.

Transformation or transfection of nucleic acids or vectors into host cells may be accomplished by a variety of means known to the person skilled in the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

Suitable host cells for the expression of the desired Alphabodies may be any eukaryotic or prokaryotic cell (e.g., bacterial cells such as E. coli, yeast cells, mammalian cells, avian cells, amphibian cells, plant cells, fish cells, and insect cells), whether located in vitro or in vivo. For example, host cells may be located in a transgenic animal.

Thus, the invention also relates to methods for the production of Alphabodies having detectable binding affinity for, or detectable in vitro activity on, a cytokine or growth factor and/or cytokine or growth factor receptor of interest comprising transforming, transfecting or infecting a host cell with nucleic acid sequences or vectors encoding such Alphabodies and expressing the Alphabodies under suitable conditions.

### [SEQUENCE RATIONALIZATION AND DEDICATED LIBRARY SCREENING]

The methods for the production of one or more target-specific Alphabodies may optionally comprise further steps or methods for improving or optimizing the binding specificity and/or efficacy of the target-specific Alphabodies obtainable by the methods of the invention.

In particular embodiments, the methods for the production of one or more target-binding Alphabodies, may further be followed by steps or methods involving the rationalization of the obtained or produced Alphabody sequences. Such a sequence rationalization process may include the identification or determination of particular amino acid residues, amino acid residue positions, stretches, motifs or patterns that are conserved between or among different Alphabodies against a specific target molecule of interest that have been produced using the methods of the invention. Accordingly, this rationalization process can be conducted by comparing different produced Alphabody sequences that are specific for a certain target molecule or protein of interest and identifying the sequence coherence between these sequences. Such a process can be optionally supported or performed by using techniques for molecular modeling, interactive ligand docking or biostatistical data mining.

The particular amino acid residues, amino acid residue positions, stretches, motifs or patterns that are identified as being conserved between or among different Alphabodies against a specific target molecule of interest may be considered as contributing to the binding or activity of the target-specific Alphabodies.

In particular embodiments, the process of sequence rationalization as described above may further be followed by the creation of a new library of Alphabody sequences starting from the set of different Alphabody sequences that have been identified as being specific for a target molecule of interest and that have been produced using the methods of the invention. In such a so-called, 'dedicated library' the set of different Alphabody sequences that have been identified as being specific for a certain target molecule of interest, the different Alphabody sequences are varied in a defined set of variegated amino acid residue positions. This defined set of variegated amino acid residue positions corresponds to those positions outside the positions where the amino acid residues, stretches, motifs or patterns are located that are conserved between or among different target-binding Alphabodies. The Alphabody libraries so obtained are referred to as 'dedicated libraries' of Alphabodies. These dedicated libraries are then again screened to identify the best target-binding Alphabody.

Thus, in the production of such dedicated libraries of Alphabody sequences, the amino acid residues, stretches, motifs or patterns that are conserved between or among different Alphabodies are kept constant during the production process of the library. From such dedicated libraries, Alphabody sequences having an improved or optimized binding specificity for and/or in vitro activity on the target molecule of interest may be identified and optionally isolated.

In particular embodiments, the process of sequence rationalization as described above may further be followed by the creation of a new library of Alphabody sequences starting from the set of different Alphabody sequences that have been identified as being specific for a target molecule of interest and that have been produced using the methods of the invention. In such a so-called, 'spiked library' the set of different Alphabody sequences that have been identified as being specific for a certain target molecule of interest, the different Alphabody sequences are varied by introducing at a limited number of randomly chosen positions, random amino acid substitutions. As is known by a person skilled in the art of library generation, error-prone PCR is a convenient method to generate 'spiked libraries', This can also be conveniently accomplished by a direct DNA synthesis method using spiked oligonucleotides as is known to someone skilled in the art of DNA synthesis.

Accordingly, the methods for the production of one or more target-binding Alphabodies, may further, after the identification of two or more target-binding Alphabodies from a random library, comprise the steps of:
- comparing the produced Alphabody sequences that bind the target protein of interest,
- identifying the amino acid residues, amino acid residue positions, stretches, motifs or patterns that are conserved between or among these different Alphabody sequences, and:
- starting from at least one of the two or more Alphabody sequences compared, producing a spiked library wherein the library comprises different Alphabody sequences that are variegated at a limited number of randomly chosen positions, or, producing a dedicated library wherein the library comprises different Alphabody sequences that are variegated in a set of amino acid positions which are not the amino acid residues, amino acid residue positions, stretches, motifs or patterns that are conserved between or among the different target-binding Alphabody sequences,
- selecting and/or identifying from the random library those Alphabody sequences having an improved or optimized binding specificity for and/or in vitro activity on the target molecule of interest, and optionally
- isolating these Alphabody sequences having an improved or optimized binding specificity for and/or in vitro activity on the target molecule of interest.

It will be understood that the steps involved in the methods for producing a dedicated or a spiked library and selecting, identifying and isolating Alphabody sequences having an improved or optimized binding specificity for and/or in vitro activity on the target molecule of interest, as described above, may be performed in a similar manner as described for the corresponding steps of the methods for producing target-binding Alphabodies of the invention.

As further described herein, the total number of amino acid residues in an Alphabody of the invention can be in the range of about 50 to about 210, depending mainly on the number of heptads per heptad repeat sequence and the length of the flexible linkers interconnecting the heptad repeat sequences. Parts, fragments, analogs or derivatives of an Alphabody, polypeptide or composition of the invention are not particularly limited as to their length and/or size, as long as such parts, fragments, analogs or derivatives still have the biological function of an Alphabody, polypeptide or composition of the invention from which they are derived and can still be used for the envisaged (pharmacological) purposes.

It should be remarked that directed evolution methods (such as DNA shuffling methods) may also be employed in building Alphabody libraries starting from one or more different Alphabody sequences that have been identified as being specific for a target molecule of interest. Such 'directed evolution' libraries can also be subjected to the selection and/or the identification of those Alphabody sequences having an improved or optimized binding specificity for and/or in vitro activity on the target molecule of interest.

### [POLYPEPTIDES COMPRISING ALPHABODIES]

In a further aspect, the present invention provides (Alphabody) polypeptides (also referred to herein as polypeptides of the invention) that comprise or essentially consist of at least one Alphabody of the present invention that specifically binds to a cytokine or growth factor and/or a cytokine or growth factor receptor. The polypeptides of the invention may comprise at least one Alphabody of the present invention and optionally one or more further groups, moieties, residues optionally linked via one or more linkers.

Accordingly, a polypeptide of the invention may optionally contain one or more further groups, moieties or residues for binding to other targets or target proteins of interest. It should be clear that such further groups, residues, moieties and/or binding sites may or may not provide further functionality to the Alphabodies of the invention (and/or to the polypeptide or composition in which it is present) and may or may not modify the properties of the Alphabody of the invention. Such groups, residues, moieties or binding units may also for example be chemical groups which can be biologically and/or pharmacologically active.

These groups, moieties or residues are, in particular embodiments, linked N- or C-terminally to the Alphabody. In particular embodiments however, one or more groups, moieties or residues are linked to the body of the Alphabody, e.g. to a free cysteine in an alpha-helix.

In particular embodiments, the polypeptides of the present invention comprise Alphabodies that have been chemically modified. For example, such a modification may involve the introduction or linkage of one or more functional groups, residues or moieties into or onto the Alphabody of the invention. These groups, residues or moieties may confer one or more desired properties or functionalities to the Alphabody of the invention. Examples of such functional groups will be clear to the skilled person.

For example, the introduction or linkage of such functional groups to an Alphabody of the invention can result in an increase in the half-life, the solubility and/or the stability of the Alphabody of the invention or in a reduction of the toxicity of the Alphabody of the invention, or in the elimination or attenuation of any undesirable side effects of the Alphabody of the invention, and/or in other advantageous properties.

In particular embodiments, the polypeptides of the present invention comprise Alphabodies that have been chemically modified to increase the biological or plasma half-life thereof, for example, by means of PEGylation. by means of the addition of a group which binds to or which is a serum protein (such as serum albumin) or, in general, by linkage of the Alphabody to a moiety that increases the half-life of the Alphabody of the invention. As an example, Alphabodies can be PEGylated at a solvent exposed cysteine using maleimide mPEG 40kD PEG (Jenkem Technology) or other PEG moieties of different molecular mass.

In particular embodiments, the polypeptides of the present invention comprise Alphabodies that have been fused to protein domains or peptides to increase the biological or plasma half-life thereof, for example, with a domain which binds to or which is a serum protein (such as serum albumin or to the Fc part of an immunoglobulin). Said protein domain may be an Alphabody which binds to a serum protein.

In particular embodiments, the polypeptides of the present invention comprise Alphabodies that in addition to their target binding (i.e., binding toward the cytokine or growth factor or cytokine or growth factor receptor of interest) bind also to a serum protein (such as serum albumin or to the Fc part of an immunoglobulin) to increase the biological or plasma half-life of said Alphabodies.

Typically, the polypeptides of the invention with increased half-life have a half-life (in human or in an animal model used for PK evaluation such as rat, dog, monkey, mouse, horse, pig, cat, etc) of more than 1 week, equally preferably more than 2 weeks as compared to the half-life of the corresponding Alphabody of the invention lacking the above described equipment for half life extension.

A particular modification of the Alphabodies of the invention may comprise the introduction of one or more detectable labels or other signal-generating groups or moieties, depending on the intended use of the labeled Alphabody.

Yet a further particular modification may involve the introduction of a chelating group, for example to chelate one or more metals or metallic cations.

A particular modification may comprise the introduction of a functional group that is one part of a specific binding pair, such as the biotin-(strept)avidin binding pair.

For some applications, in particular for those applications in which it is intended to kill a cell that expresses the target against which the Alphabodies of the invention specifically bind to (e.g., in the treatment of cancer), or to reduce or slow the growth and/or proliferation of such a cell, the Alphabodies of the invention may also be linked to a toxin or to a toxic residue or moiety.

Other potential chemical and enzymatic modifications will be clear to the skilled person.

In particular embodiments, the one or more groups, residues, moieties are linked to the Alphabody via one or more suitable linkers or spacers.

In further particular embodiments, the polypeptides of the invention comprise two or more target-specific Alphabodies. In such particular embodiments, the two or more target-specific Alphabodies may be linked (coupled, concatenated, interconnected, fused) to each other either in a direct or in an indirect way. In embodiments wherein the two or more Alphabodies are directly linked to each other, they are linked without the aid of a spacer or linker fragment or moiety. Alternatively, in embodiments wherein the two or more Alphabodies are indirectly linked to each other, they are linked via a suitable spacer or linker fragment or linker moiety.

In embodiments wherein two or more Alphabodies are directly linked, they may be produced as single-chain fusion constructs (i.e., as single-chain protein constructs wherein two or more Alphabody sequences directly follow each other in a single, contiguous amino acid sequence). Alternatively, direct linkage of Alphabodies may also be accomplished via cysteines forming a disulfide bridge between two Alphabodies (i.e., under suitable conditions, such as oxidizing conditions, two Alphabodies comprising each a free cysteine may react with each other to form a dimer wherein the constituting monomers are covalently linked through a disulfide bridge).

Alternatively, in embodiments wherein two or more Alphabodies are indirectly linked, they may be linked to each other via a suitable spacer or linker fragment or linker moiety. In such embodiments, they may also be produced as single-chain fusion constructs (i.e., as single-chain protein constructs wherein two or more Alphabody sequences follow each other in a single, contiguous amino acid sequence, but wherein the Alphabodies remain separated by the presence of a suitably chosen amino acid sequence fragment acting as a spacer fragment). Alternatively, indirect linkage of Alphabodies may also be accomplished via amino acid side groups or via the Alphabody N- or C-termini. For example, under suitably chosen conditions, two Alphabodies comprising each a free cysteine may react with a homo-bifunctional chemical compound, yielding an Alphabody dimer wherein the constituting Alphabodies are covalently cross-linked through the said homo-bifunctional compound. Analogously, one or more Alphabodies may be cross-linked through any combination of reactive side groups or termini and suitably chosen homo- or heterobifunctional chemical compounds for cross-linking of proteins.

In particular embodiments of linked Alphabodies, the two or more linked Alphabodies can have the same amino acid sequence or different amino acid sequences. The two or more linked Alphabodies can also have the same binding specificity or a different binding specificity. The two or more linked Alphabodies can also have the same binding affinity or a different binding affinity.

Suitable spacers or linkers for use in the coupling of different Alphabodies of the invention will be clear to the skilled person and may generally be any linker or spacer used in the art to link peptides and/or proteins. In particular, such a linker or spacer is suitable for constructing proteins or polypeptides that are intended for pharmaceutical use.

Some particularly suitable linkers or spacers for coupling of Alphabodies in a single-chain amino acid sequence include for example, but are not limited to, polypeptide linkers such as glycine linkers, serine linkers, mixed glycine/serine linkers, glycine- and serine-rich linkers or linkers composed of largely polar polypeptide fragments. Some particularly suitable linkers or spacers for coupling of Alphabodies by chemical cross-linking include for example, but are not limited to, homo-bifunctional chemical cross-linking compounds such as glutaraldehyde, imidoesters such as dimethyl adipimidate (DMA), dimethyl suberimidate (DMS) and dimethyl pimelimidate (DMP) or N-hydroxysuccinimide (NHS) esters such as dithiobis(succinimidylpropionate) (DSP) and dithiobis(sulfosuccinimidylpropionate) (DTSSP). Examples of hetero-bifunctional reagents for cross-linking include, but are not limited to, cross-linkers with one amine-reactive end and a sulfhydryl-reactive moiety at the other end, or with a NHS ester at one end and an SH-reactive group (e.g., a maleimide or pyridyl disulfide) at the other end.

A polypeptide linker or spacer for usage in single-chain concatenated Alphabody constructs may be any suitable (e.g., glycine-rich) amino acid sequence having a length between 1 and 50 amino acids, such as between 1 and 30, and in particular between 1 and 10 amino acid residues. It should be clear that the length, the degree of flexibility and/or other properties of the spacer(s) may have some influence on the properties of the final polypeptide of the invention, including but not limited to the affinity, specificity or avidity for a protein of interest, or for one or more other target proteins of interest. It should be clear that when two or more spacers are used in the polypeptides of the invention, these spacers may be the same or different. In the context and disclosure of the present invention, the person skilled in the art will be able to determine the optimal spacers for the purpose of coupling Alphabodies of the invention without any undue experimental burden.

The linked Alphabody polypeptides of the invention can generally be prepared by a method which comprises at least one step of suitably linking the one or more Alphabodies of the invention to the one or more further groups, residues, moieties and/or other Alphabodies of the invention, optionally via the one or more suitable linkers, so as to provide a polypeptide of the invention.

Also, the polypeptides of the present invention can be produced by methods at least comprising the steps of: (i) expressing, in a suitable host cell or expression system, the polypeptide of the invention, and (ii) isolating and/or purifying the polypeptide of the invention. Techniques for performing the above steps are known to the person skilled in the art.

### [PARTS/FRAGMENTS/ANALOGS/DERIVATIVES]

The present invention also encompasses parts, fragments, analogs, mutants, variants, and/or derivatives of the Alphabodies and polypeptides of the invention and/or polypeptides comprising or essentially consisting of one or more of such parts, fragments, analogs, mutants, variants, and/or derivatives, as long as these parts, fragments, analogs, mutants, variants, and/or derivatives are suitable for the prophylactic, therapeutic and/or diagnostic purposes envisaged herein.

Such parts, fragments, analogs, mutants, variants, and/or derivatives according to the invention are still capable of specifically binding to cytokines or growth factors and/or cytokine or growth factor receptors.

### [ORIGIN AND FORM OF ALPHABODIES, POLYPEPTIDES AND COMPOSITIONS OF INVENTION]

It should be noted that the invention is not limited as to the origin of the Alphabodies, polypeptides or compositions of the invention (or of the nucleotide sequences of the invention used to express them). Furthermore, the present invention is also not limited as to the way that the Alphabodies, polypeptides or nucleotide sequences of the invention have been generated or obtained. Thus, the Alphabodies of the invention may be synthetic or semi-synthetic amino acid sequences, polypeptides or proteins.

The Alphabodies, polypeptides and compositions provided by the invention can be in essentially isolated form (as defined herein), or alternatively can form part of a polypeptide or composition of the invention, which may comprise or essentially consist of at least one Alphabody of the invention and which may optionally further comprise one or more other groups, moieties or residues (all optionally linked via one or more suitable linkers).

### [TARGET SPECIES AND CROSS-REACTIVITY]

It will be appreciated based on the disclosure herein that for prophylactic, therapeutic and/or diagnostic applications, the Alphabodies, polypeptides and compositions of the invention will in principle be directed against or specifically bind to human cytokines or growth factors and/or human cytokine or growth factor receptors. However, where the Alphabodies, polypeptides and compositions of the invention are intended for veterinary purposes, they will be directed against or specifically bind to cytokines or growth factors and/or cytokine or growth factor receptors from the species intended to be treated, or they will be at least cross-reactive with cytokines or growth factors and/or cytokine or growth factor receptors from the species to be treated. Accordingly, Alphabodies, polypeptides and compositions that specifically bind to cytokines or growth factors and/or cytokine or growth factor receptors from one subject species may or may not show cross-reactivity with cytokines or growth factors and/or cytokine or growth factor receptors from one or more other subject species. Of course it is envisaged that, in the context of the development of Alphabodies for use in humans or animals, Alphabodies may be developed which bind to a cytokine or growth factor and/or a cytokine or growth factor receptor from another species than that which is to be treated, for use in research and laboratory testing.

It is also expected that the Alphabodies and polypeptides of the invention will bind to a number of naturally, occurring or synthetic analogs, variants, mutants, alleles, parts and fragments of cytokines or growth factors and/or cytokine or growth factor receptors. More particularly, it is expected that the Alphabodies and polypeptides of the invention will bind to at least those analogs, variants, mutants, alleles, parts and fragments of cytokines or growth factor and/or cytokine or growth factor receptors that (still) contain the binding site, part or domain of the (natural/wild-type) cytokine or growth factor and/or the cytokine or growth factor receptor to which those Alphabodies and polypeptides bind.

### [NUCLEIC ACID SEQUENCES]

In yet a further aspect, the invention provides nucleic acid sequences encoding single-chain Alphabodies or Alphabody polypeptides, which are obtainable by the methods according to the invention (also referred to herein as 'nucleic acid sequences of the invention') as well as vectors and host cells comprising such nucleic acid sequences.

In a further aspect, the present invention provides nucleic acid sequences encoding the Alphabodies or the polypeptides of the invention (or suitable fragments thereof). These nucleic acid sequences are also referred to herein as nucleic acid sequences of the invention and can also be in the form of a vector or a genetic construct or polynucleotide. The nucleic acid sequences of the invention may be synthetic or semi-synthetic sequences, nucleotide sequences that have been isolated from a library (and in particular, an expression library), nucleotide sequences that have been prepared by PCR using overlapping primers, or nucleotide sequences that have been prepared using techniques for DNA synthesis known per se.

The genetic constructs of the invention may be DNA or RNA, and are preferably double-stranded DNA. The genetic constructs of the invention may also be in a form suitable for transformation of the intended host cell or host organism in a form suitable for integration into the genomic DNA of the intended host cell or in a form suitable for independent replication, maintenance and/or inheritance in the intended host organism. For instance, the genetic constructs of the invention may be in the form of a vector, such as for example a plasmid, cosmid, YAC, a viral vector or transposon. In particular, the vector may be an expression vector, i.e., a vector that can provide for expression in vitro and/or in vivo (e.g. in a suitable host cell, host organism and/or expression system). The genetic constructs of the invention may comprise a suitable leader sequence to direct the expressed Alphabody to an intended intracellular or extracellular compartment. For example, the genetic constructs of the invention may be inserted in a suitable vector at a pelB leader sequence site to direct the expressed Alphabody to the bacterial periplasmic space. Also the vector may be equipped with a suitable promoter system to, for example, optimize the yield of the Alphabody.

In a further aspect, the invention provides vectors comprising nucleic acids encoding single-chain Alphabodies, which are obtainable by the methods according to the invention.

In yet a further aspect, the present invention provides host cells comprising nucleic acids encoding single-chain Alphabodies or Alphabody polypeptides, which are obtainable by the methods according to the invention or vectors comprising these nucleic acids. Accordingly, a particular embodiment of the invention is a host cell transfected or transformed with a vector comprising the nucleic acid sequence encoding the Alphabodies or Alphabody polypeptides obtainable by the methods of the invention and which is capable of expressing them. Suitable examples of hosts or host cells for expression of the Alphabodies or polypeptides of the invention will be clear to the skilled person and include any suitable eukaryotic or prokaryotic cell (e.g., bacterial cells such as E. coli, yeast cells, mammalian cells, avian cells, amphibian cells, plant cells, fish cells, and insect cells), whether located in vitro or in vivo.

### [INHIBITING ALPHABODIES, POLYPEPTIDES AND COMPOSITIONS]

In particular embodiments, the Alphabodies or polypeptides of the invention that specifically bind to cytokines or growth factors or a cytokine or growth factor receptor, are capable of specifically inhibiting, preventing or decreasing the activity of the cytokines or growth factors or cytokine or growth factor receptor, and/or of inhibiting, preventing or decreasing the signaling and biological mechanisms and pathways in which these cytokines or growth factor and/or receptors play a role.

By binding to one or more particular cytokines or growth factors and/or cytokine or growth factor receptors, the Alphabodies, polypeptides and pharmaceutical compositions of the present invention can be used to prevent or inhibit the interaction between one or more cytokines or growth factors and their corresponding cytokine or growth factor receptors, thereby preventing, inhibiting or reducing the signalling pathways that are mediated by those cytokines or growth factors and/or their receptors and/or modulating the biological pathways and mechanisms in which those cytokines or growth factors and/or their receptors are involved. Accordingly, the Alphabodies, polypeptides and pharmaceutical compositions of the present invention can be used to affect, change or modulate the immune system and/or one or more specific immune responses in a subject in which the cytokines or growth factors and/or cytokine or growth factor receptors to which the one or more of the Alphabodies, polypeptides and compositions of the present invention bind, are involved.

Thus, in particular embodiments, the Alphabodies, polypeptides and compositions of the invention, specifically bind to a cytokine or growth factor, and more particularly to the receptor binding site on the cytokine or growth factor.

More particularly, 'inhibiting', 'reducing' and/or 'preventing' using an Alphabody, polypeptide or composition of the invention may mean either inhibiting, reducing and/or preventing the interaction between a target protein of interest and its natural binding partner, or, inhibiting, reducing and/or preventing the activity of a target protein of interest, or, inhibiting, reducing and/or preventing one or more biological or physiological mechanisms, effects, responses, functions pathways or activities in which the target protein of interest is involved, such as by at least 10%, but preferably at least 20%, for example by at least 50%, at least 60%, at least 70%, at least 80%, at least 90%, at least 95% or more, as measured using a suitable in vitro, cellular or in vivo assay, compared to the activity of the target protein of interest in the same assay under the same conditions but without using the Alphabody, polypeptide or composition of the invention. In addition, 'inhibiting', 'reducing' and/or 'preventing' may also mean inducing a decrease in affinity, avidity, specificity and/or selectivity of a target protein of interest for one or more of its natural binding partners and/or inducing a decrease in the sensitivity of the target protein of interest for one or more conditions in the medium or surroundings in which the target protein of interest is present (such as pH, ion strength, the presence of co-factors, etc.), compared to the same conditions but without the presence of the Alphabody, polypeptide or composition of the invention. In the context of the present invention, 'inhibiting', 'reducing' and/or 'preventing' may also involve allosteric inhibition, reduction and/or prevention of the activity of a target protein of interest.

Accordingly, in particular embodiments, the Alphabodies, polypeptides and compositions of the invention are directed against a cytokine or growth factor (as further described herein). The result of the binding of the Alphabodies to the cytokine or growth factor can be such that, upon binding to that cytokine or growth factor, it prevents, reduces or inhibits binding of that cytokine or growth factor to its receptor or to at least one subunit thereof compared to the binding of the cytokine or growth factor to its receptor in the absence of such Alphabodies, polypeptides or pharmaceutical compositions of the invention, and this by at least 20%, for example by at least 50%, as at least 70%, at least 80%, at least 90%, at least 95% or more, as determined by a suitable assay known in the art. Alternatively, the binding of the Alphabodies or Alphabody polypeptides to the cytokine or growth factor is such that it still allows the cytokine or growth factor to bind to its receptor, but prevents, reduces or inhibits the signalling that would be triggered by binding of the cytokine or growth factor to its receptor or at least one subunit thereof compared to the signalling upon binding of the cytokine or growth factor to its receptor in the absence of such Alphabodies, polypeptides or pharmaceutical compositions of the invention, and this by at least 20%, for example by at least 50%, as at least 70%, at least 80%, at least 90%, at least 95% or more, as determined by a suitable assay known in the art. In further particular embodiments, the binding of the Alphabodies or Alphabody polypeptides to the cytokine or growth factor is such that it prevents, reduces or inhibits activation and/or association of the receptor, and in particular cytokine-mediated or growth factor-mediated association of the receptor (i.e. compared to the cytokine- or growth factor-mediated association of the receptor without the presence of such Alphabodies, polypeptides and compositions of the invention), and this by at least 20%, for example by at least 50%, as at least 70%, at least 80%, at least 90%, at least 95% or more, as determined by a suitable assay known in the art.

In other particular embodiments, the Alphabodies or polypeptides of the invention specifically bind to a cytokine or growth factor receptor, and more particularly to the cytokine or growth factor binding site on the cytokine or growth factor receptor.

Accordingly, in particular embodiments, the Alphabodies, polypeptides and compositions of the invention are directed against a cytokine or growth factor receptor or at least one subunit thereof (as further described herein) such that, upon binding to that cytokine or growth factor receptor, it prevents, reduces or inhibits binding of that cytokine or growth factor receptor to its natural cytokine or growth factor ligand compared to the binding of the cytokine or growth factor receptor to its natural cytokine or growth factor ligand in the absence of such Alphabodies, polypeptides or pharmaceutical compositions of the invention, and this by at least 20%, for example by at least 50%, as at least 70%, at least 80%, at least 90%, at least 95% or more, as determined by a suitable assay known in the art.

Alternatively, binding of the Alphabodies or Alphabody polypeptides to the cytokine or growth factor receptor or to at least one subunit thereof (as further described herein) is such that it still allows the cytokine or growth factor receptor to bind to its natural cytokine or growth factor ligand, but prevents, reduces or inhibits the signalling that would be triggered by binding of the cytokine or growth factor receptor to its natural cytokine or growth factor ligand compared to the signalling upon binding of the cytokine or growth factor receptor to its natural cytokine or growth factor ligand in the absence of such Alphabodies, polypeptides or pharmaceutical compositions of the invention, and this by at least 20%, for example by at least 50%, as at least 70%, at least 80%, at least 90%, at least 95% or more, as determined by a suitable assay known in the art. According to particular embodiments, the Alphabodies, polypeptides and compositions of the invention specifically bind to a cytokine or growth factor receptor (as further described herein) such that it prevents, reduces or inhibits activation and/or association of the receptor, and in particular cytokine-mediated or growth factor-mediated association of the receptor (i.e. compared to the cytokine- or growth factor-mediated association of the receptor without the presence of such Alphabodies, polypeptides and compositions of the invention), and this by at least 20%, for example by at least 50%, as at least 70%, at least 80%, at least 90%, at least 95% or more, as determined by a suitable assay known in the art.

As will be known to the skilled person, the above Alphabodies, polypeptides and compositions of the invention will generally act as antagonists of cytokine- or growth factor-mediated signalling , i.e. the signalling that is caused by binding of a cytokine or growth factor to its receptor, as well as the biological mechanisms and effects that are induced by such signalling.

### [AGONIZING ALPHABODIES, POLYPEPTIDES AND COMPOSITIONS]

In certain non-limiting embodiments, an Alphabody, polypeptide or composition according to the invention may specifically bind to a cytokine or growth factor and/or a cytokine or growth factor receptor thereby enhancing, increasing and/or activating the interaction between that cytokine or growth factor and/or its receptor. Such an agonizing Alphabody, polypeptide or composition according to the invention may specifically bind to a cytokine or growth factor and/or a cytokine or growth factor receptor thereby enhancing, increasing and/or activating the biological activity and/or one or more biological or physiological mechanisms, effects, responses, functions or pathways of that cytokine or growth factor and/or that cytokine or growth factor receptor, as measured using a suitable in vitro, cellular or in vivo assay. As will be clear to the skilled person, the Alphabodies, polypeptides and compositions of the invention according to this particular embodiment, will generally act as agonists of cytokine-mediated or growth factor-mediated signalling, i.e. the signalling that is caused by binding of a cytokine or growth factor to its receptor, as well as the biological mechanisms and effects that are induced by such signalling.

Accordingly, in these particular embodiments, the Alphabodies, polypeptides and pharmaceutical compositions of the present invention can be used to increase one or more specific immune responses in a subject in which the cytokines or growth factors and/or cytokine or growth factor receptors to which the one or more of the Alphabodies, polypeptides and compositions of the present invention bind, are involved. Agonistic Alphabodies, polypeptides or pharmaceutical compositions of the invention binding to certain cytokines or growth factors and/or cytokine or growth factor receptors can be used to stimulate or enhance one or more immune responses in a subject, for example for the prevention and/or treatment of diseases that are characterized by a weakened immune system or that may occur as a result of having a weakened immune system.

### [PHARMACEUTICAL COMPOSITIONS]

In yet a further aspect, the present invention provides pharmaceutical compositions comprising one or more Alphabodies, polypeptides and/or nucleic acid sequences according to the invention and optionally at least one pharmaceutically acceptable carrier (also referred to herein as pharmaceutical compositions of the invention). According to certain particular embodiments, the pharmaceutical compositions of the invention may further optionally comprise at least one other pharmaceutically active compound.

The pharmaceutical compositions of the present invention can be used in the diagnosis, prevention and/or treatment of diseases and disorders associated with cytokines or growth factors and/or cytokine or growth factor receptors.

In particular, the present invention provides pharmaceutical compositions comprising Alphabodies and polypeptides of the invention that are suitable for prophylactic, therapeutic and/or diagnostic use in a warm-blooded animal, and in particular in a mammal, and more in particular in a human being.

The present invention also provides pharmaceutical compositions comprising Alphabodies and polypeptides of the invention that can be used for veterinary purposes in the prevention and/or treatment of one or more diseases, disorders or conditions associated with and/or mediated by cytokines or growth factors and/or cytokine or growth factor receptors.

Generally, for pharmaceutical use, the polypeptides of the invention may be formulated as, a pharmaceutical preparation or compositions comprising at least one Alphabody or polypeptide of the invention and at least one pharmaceutically acceptable carrier, diluent or excipient and/or adjuvant, and optionally one or more further pharmaceutically active polypeptides and/or compounds. Such a formulation may be suitable for oral, parenteral, topical administration or for administration by inhalation. Thus, the Alphabodies, or polypeptides of the invention and/or the compositions comprising the same can for example be administered orally, intraperitoneally, intravenously, subcutaneously, intramuscularly, transdermally, topically, by means of a suppository, by inhalation, again depending on the specific pharmaceutical formulation or composition to be used. The clinician will be able to select a suitable route of administration and a suitable pharmaceutical formulation or composition to be used in such administration.

The pharmaceutical compositions may also contain suitable binders, disintegrating agents, sweetening agents or flavoring agents. Tablets, pills, or capsules may be coated for instance with gelatin, wax or sugar and the like. In addition, the Alphabodies and polypeptides of the invention may be incorporated into sustained-release preparations and devices.

The pharmaceutical dosage forms suitable for injection or infusion can include sterile aqueous solutions or dispersions or sterile powders comprising the active ingredient which are adapted for the extemporaneous preparation of sterile injectable or infusible solutions or dispersions, optionally encapsulated in liposomes. In all cases, the ultimate dosage form must be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols; and the like), vegetable oils, nontoxic glyceryl esters, and suitable mixtures thereof. Antibacterial and antifungal agents and the like can optionally be added.

Useful dosages of the Alphabodies and polypeptides of the invention can be determined by determining their in vitro activity, and/or in vivo activity in animal models. Methods for the extrapolation of effective dosages in mice, and other animals, to humans are known to the skilled person.

The amount of the Alphabodies and polypeptides of the invention required for use in prophylaxis and/or treatment may vary not only with the particular Alphabody or polypeptide selected but also with the route of administration, the nature of the condition being treated and the age and condition of the patient and will be ultimately at the discretion of the attendant physician or clinician. Also the dosage of the Alphabodies and polypeptides of the invention may vary depending on the target cell, tumor, tissue, graft, or organ.

The Alphabodies or polypeptides of the invention and/or the compositions comprising the same are administered according to a regimen of treatment that is suitable for preventing and/or treating the disease or disorder to be prevented or treated. The clinician will generally be able to determine a suitable treatment regimen. Generally, the treatment regimen will comprise the administration of one or more Alphabodies and/or polypeptides of the invention, or of one or more compositions comprising the same, in one or more pharmaceutically effective amounts or doses.

The desired dose may conveniently be presented in a single dose or as divided doses (which can again be sub-dosed) administered at appropriate intervals. An administration regimen could include long-term (i.e., at least two weeks, and for example several months or years) or daily treatment.

The Alphabodies and polypeptides of the present invention will be administered in an amount which will be determined by the medical practitioner based inter alia on the severity of the condition and the patient to be treated. Typically, for each disease indication an optical dosage will be determined specifying the amount to be administered per kg body weight per day, either continuously (e.g. by infusion), as a single daily dose or as multiple divided doses during the day. The clinician will generally be able to determine a suitable daily dose, depending on the factors mentioned herein. It will also be clear that in specific cases, the clinician may choose to deviate from these amounts, for example on the basis of the factors cited above and his expert judgment.

In particular, the Alphabodies and polypeptides of the invention may be used in combination with other pharmaceutically active compounds or principles that are or can be used for the prevention and/or treatment of the diseases and disorders cited herein, as a result of which a synergistic effect may or may not be obtained. Examples of such compounds and principles, as well as routes, methods and pharmaceutical formulations or compositions for administering them will be clear to the clinician.

### [PROPHYLACTIC, THERAPEUTIC AND/OR DIAGNOSTIC APPLICATIONS]

According to a further aspect, the present invention provides the use of Alphabodies or polypeptides of the invention that specifically bind to a cytokine or growth factor and/or a cytokine or growth factor receptor for the preparation of a medicament for the prevention and/or treatment of at least one cytokine-mediated or growth factor-mediated disease and/or disorder in which said cytokine or growth factor and/or said cytokine or growth factor receptor are involved. Accordingly, the invention provides Alphabodies, polypeptides and pharmaceutical compositions specifically binding to a cytokine or growth factor and/or a cytokine or growth factor receptor for use in the prevention and/or treatment of at least one cytokine-mediated or growth factor-mediated disease and/or disorder in which said cytokine or growth factor and/or said cytokine or growth factor receptor are involved. In particular embodiments, the present invention also provides methods for the prevention and/or treatment of at least one cytokine- or growth factor-mediated disease and/or disorder, comprising administering to a subject in need thereof, a pharmaceutically active amount of one or more Alphabodies, polypeptides and/or pharmaceutical compositions of the invention. In particular, the pharmaceutically active amount may be an amount that is sufficient (to create a level of the Alphabody or polypeptide in circulation) to inhibit, prevent or decrease (or in the case of agonistic Alphabodies and polypeptides of the invention: enhance, promote or increase) the function of cytokines or growth factors and/or their receptors or their biological or pharmacological activity and/or the biological pathways or signalling in which they are involved.

The subject or patient to be treated with the Alphabodies or polypeptides of the invention may be any warm-blooded animal, but is in particular a mammal, and more in particular a human suffering from, or at risk of, diseases and disorders in which the cytokines or growth factors and/or cytokine or growth factor receptors to which the Alphabodies or polypeptides of the invention specifically bind are involved.

'Diseases and disorders associated with cytokines or growth factors and/or cytokine or growth factor receptors' can be defined as diseases and disorders in which the activity of one or more cytokines or growth factors plays a detrimental role. Thus these are diseases or disorders that can be prevented and/or treated, respectively, by suitably administering to a subject in need thereof (i.e., having the disease or disorder or at least one symptom thereof and/or being at risk of attracting or developing the disease or disorder), either an Alphabody, a polypeptide or composition of the invention (and in particular, of a pharmaceutically active amount thereof). Examples of such diseases and disorders associated with cytokines or growth factors and/or cytokine or growth factor receptors will be clear to the skilled person, and include the following diseases and disorders: inflammation and inflammatory disorders such as bowel diseases (colitis, Crohn's disease, IBD), infectious diseases, psoriasis, and other autoimmune diseases (such as rheumatoid arthritis, Multiple Sclerosis, Spondyloarthritis, Sarcoidosis, Lupus, Behcet's disease), transplant rejection, cystic fibrosis, asthma, chronic obstructive pulmonary disease, cancer, viral infection, common variable immunodeficiency.

In particular embodiments, the disease or disorder is an inflammatory disorder and/or an autoimmune disease. In further particular embodiments, the disease is selected from the group consisting of asthma, multiple sclerosis, inflammatory bowel disease, rheumatoid arthritis or psoriasis.

In further particular embodiments, the invention provides for Alphabodies or Alphabody polypeptides against IL-23 or the IL-23 receptor for use in a method for treatment of an inflammatory and/or autoimmune disease or for use in a method of treatment of transplant rejection, cystic fibrosis, asthma, chronic obstructive pulmonary disease, cancer, viral infection, or common variable immunodeficiency.

In further particular embodiments, the invention provides for Alphabodies or Alphabody polypeptides against FIt3L (i.e., FIt3 ligand) or against FIt3R (i.e., FIt3 receptor) for use in a method for treatment of an inflammatory and/or autoimmune disease or for use in a method of treatment of transplant rejection, cystic fibrosis, asthma, chronic obstructive pulmonary disease, cancer, viral infection, or common variable immunodeficiency.

The efficacy of the Alphabodies and polypeptides of the invention, and of compositions comprising the same, can be tested using any suitable in vitro assay, cell-based assay, in vivo assay and/or animal model known per se, or any combination thereof, depending on the specific disease or disorder involved. Suitable assays and animal models will be clear to the skilled person. For example the efficacy of an anti-IL-23 Alphabody or Alphabody polypeptide can be easily determined in an in vitro assay which measures the IL-23-dependent production of IL-17 in mouse splenocytes. In this assay, splenocytes from balb/c mice are isolated, and IL-23 (mouse or human) is added together with (or without) the selected Alphabodies or Alphabody polypeptides at various concentrations. The appearance of IL-17A in the supernatans will then be detected via an ELISA from which the inhibitory potential of the anti-IL-23 Alphabodies can be easily determined.

For example, the efficacy of an anti-IL-23 or anti-FIt3L Alphabody or polypeptide can be determined in a mouse psoriasis model, using for example TNO's (The Netherlands) xenografted mice model which comprises following steps as described in TNO's web publication ('Humanized_mouse_model_psoriasis-Pharma19B1.pdf' located at http://www.tno.nl/downloads/): (a) Select patients and obtain Medical Ethical Committee approval, (b) Patient donate biopsies, based on informed consent, (c) Biopsies are transplanted onto immune-deficient mice, (d) Peripheral blood mononucleated cells (PBMC) are isolated from patients, (e) The autologous activated PBMC are injected into the xenograft to induce psoriasis, (f) Treatment with investigational drug and determining the inhibition of epidermal (acanthosis) by the anti-IL-23 drug in this humanized mouse model. The results are then compared to a topical treatment with 0.05% betamethasone. This study takes about 7 weeks from the transplantation of the skin to the end of the in vivo treatment.

Depending on the cytokine(s) or growth factor(s) and/or receptor(s) involved, the skilled person will generally be able to select a suitable in vitro assay, cellular assay or animal model to test the Alphabodies and polypeptides of the invention for binding to a cytokine or growth factor and/or a cytokine or growth factor receptor or for their capacity to affect the activity of these cytokines or growth factor and receptors, and/or the biological mechanisms in which these are involved; as well as for their therapeutic and/or prophylactic effect in respect of one or more diseases and disorders that are associate with a cytokine or growth factor and/or a cytokine or growth factor receptor.

The invention will now be further described by means of the following non-limiting Examples and Figures, in which the FIGURES show:

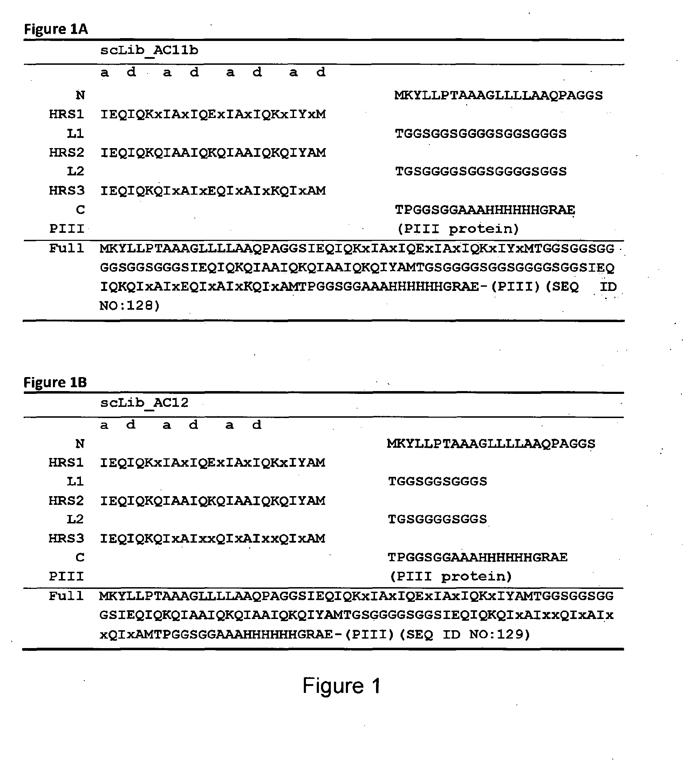

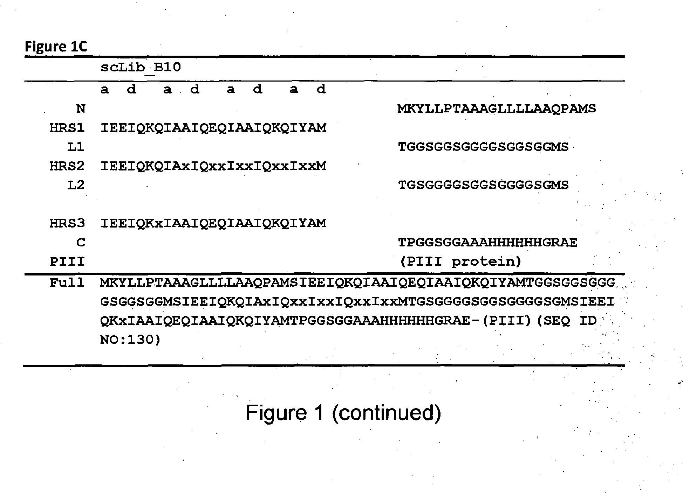

**Figure 1****.** Definition of the single-chain Alphabody libraries scLib_AC11b (Figure 1A), scLib_AC12 (Figure 1 B) and scLib_B10 (Figure 1 C). These libraries are also named by their short names, respectively AC11b, AC12 and B10. The libraries are C-terminally connected to the N-terminus of the phage coat protein pIII. The name of each library is indicated at the top row of each of the three panels ('scLib_AC11b', 'scLib_AC12' and 'scLib_B10'). Their full amino acid sequences (SEQ ID NO: 128, 129, 130) are listed (in single-letter notation) at the bottom of each table panel, to the right of the label 'Full'. The symbol 'x' is used at positions that are randomized. 'PIII' denotes the phage pIII coat protein. Specific segments within the same sequences are also shown on top, to facilitate identification of N- and C-terminal flanking segments (labeled 'N' and 'C', respectively), linker segments (labeled 'L1' and 'L2', respectively) and the actual heptad repeat sequences (labeled 'HRS1', 'HRS2' and 'HRS3'). Heptad a- and d-positions are provided at the top row to facilitate their identification within the heptad repeat sequences.

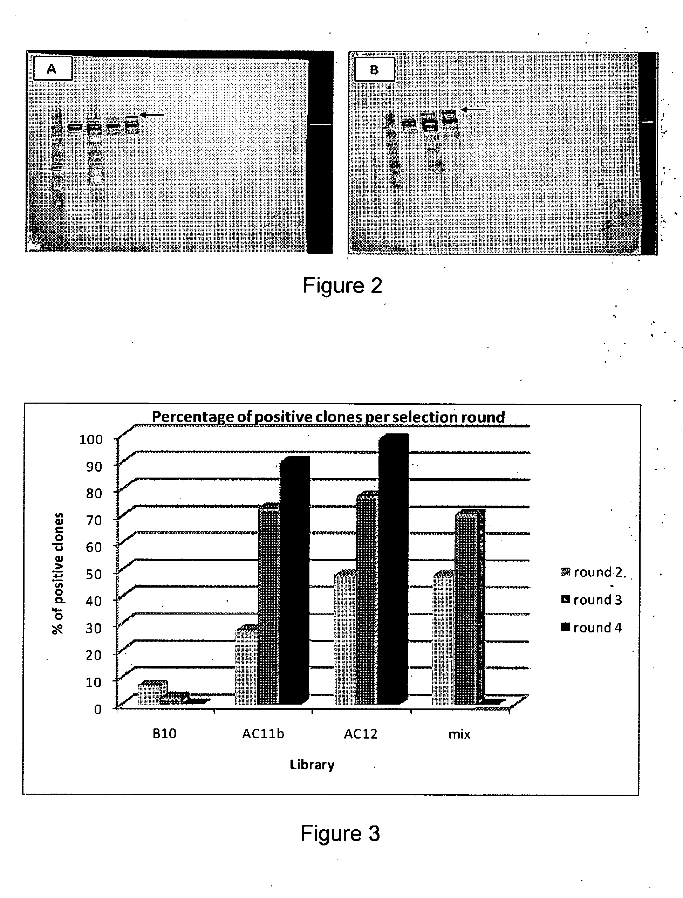

**Figure 2****.** Western blot of Alphabody libraries. Phage were run on a 12% SDS-PAGE gel and transferred on a PVDF membrane for Western blot analysis. The pIII protein was visualized with an anti-pIII antibody. Panel A: The first two lanes are respectively, a size ladder (ColorPlus Pre-stained protein Ladder broad range 10-230 kDa, NEB) and an empty phage. The empty phage displayed only wild type pIII protein. The next three lanes are a reference Alphabody library, the scLib_AC12 and the scLib_AC11b library, respectively. Panel B: The first two lanes are respectively, a size ladder (ColorPlus Pre-stained protein Ladder broad range 10-230 kDA, NEB) and an empty phage. The next two lanes are a reference Alpliabody library and the scLib_B10 library, respectively. The arrow indicates the Alphabody fusion pIII protein displaying a higher molecular weight than the wild type pIII protein.
**Figure 3****.** Evolution of the percentage of positive clones as determined in ELISA during the selection rounds 2, 3 and 4 for the 4 biopanning campaigns with the scLib_B10, scLib_AC11b, scLib_AC12 and mix of these libraries. The percentage of positive clones was not determined for the library scLib_B10 and the mix of libraries at round 4.

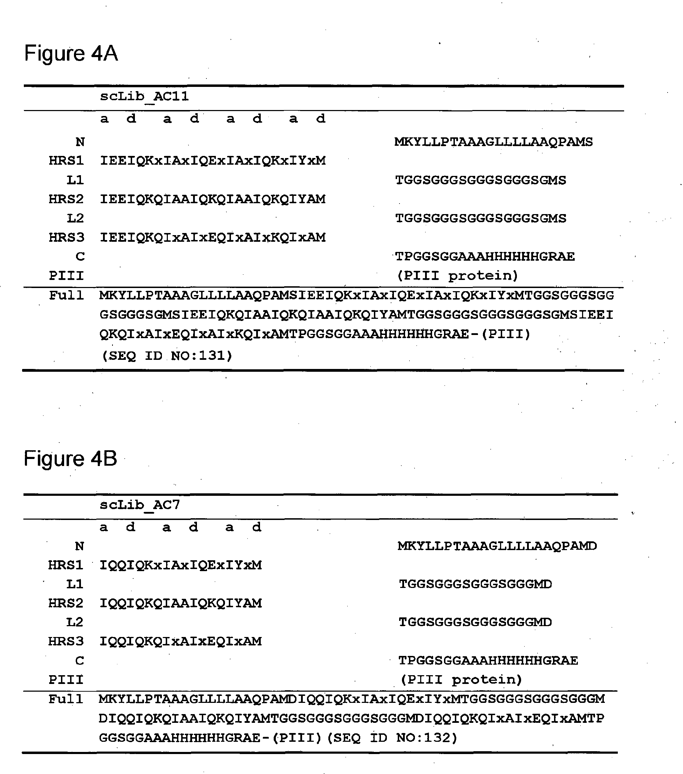

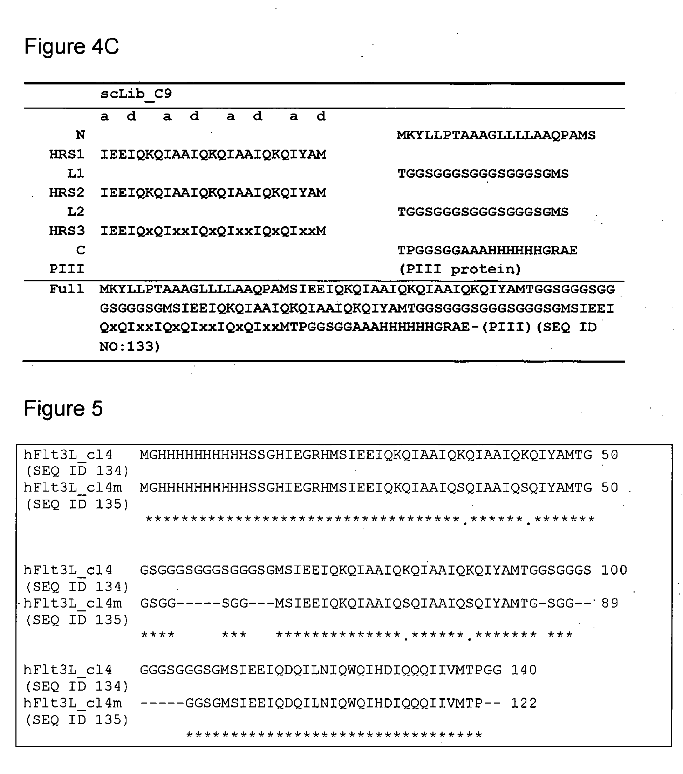

**Figure 4****.** Definition of the single-chain Alphabody libraries scLib_AC11 (Figure 4A), scLib_AC7 (Figure 4B) and scLib_C9 (Figure 4C). These libraries are also named by their short names AC11, AC7 and C9, respectively. Their full amino acid sequences (SEQ ID NO: 131, 132, 133) are listed (in single-letter notation) at the bottom of each table panel, to the right of the label 'Full'. The symbol 'x' is used at positions that are randomized. 'PIII' denotes the phage pIII coat protein. Specific segments within the same sequences are also shown on top, to facilitate identification of N- and C-terminal flanking segments (labeled 'N' and 'C', respectively), linker segments (labeled 'L1' and 'L2', respectively) and the actual heptad repeat sequences (labeled 'HRS1', 'HRS2' and 'HRS3'). Heptad a- and d-positions are provided at the top row to facilitate their identification within the heptad repeat sequences.
**Figure 5****.** Alignment of the full amino acid sequences of hFIt3L cl4 (SEQ ID No: 134) and hFlt3L_cl4m (SEQ ID No: 135).

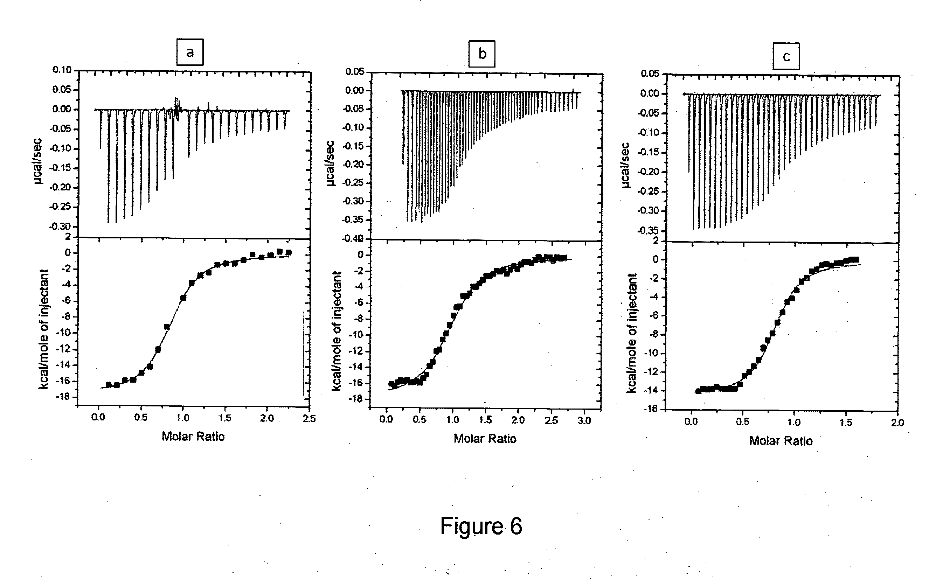

**Figure 6****.** Isothermal titration calorimetry of hFIt3L (cell) with hFlt3L_cl4 (syringe). The experiment was performed three times (panels a-c). Upper panels show the raw thermograms recorded as heat output (in µcal/sec); each peak corresponds to one injection. Lower panels show the enthalpy changes (in kcal/mole of hFlt3L_cl4) integrated per peak.

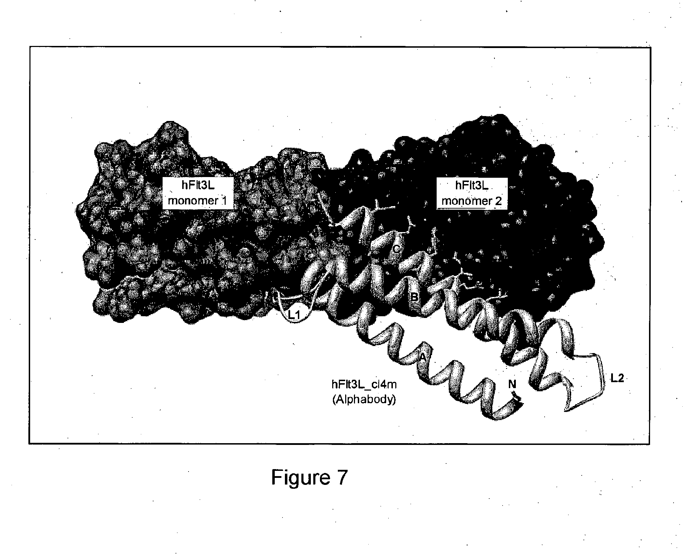

**Figure 7****.** Crystallographically determined structure of the hFlt3L_cl4m:(hFlt3L)₂ complex. The dimeric hFIt3L structure is rendered in space filling mode, with each monomer in different shades of gray. The Alphabody hFlt3L_cl4m is shown as a ribbon and its N-terminal end is labeled 'N'. Alphabody helices A, B and C and linker segments L1 and L2 are labeled accordingly. Linker segment L2 was not well visible in the X-ray structure (in contrast to L1) and was completed by molecular modeling. All contact residues from the Alphabody (having one or more side-chain atoms within 4 A distance from hFIt3L) are represented by sticks. All such residues are located in the C-helix, although some additional contacts are observed in L1 as well.

### EXAMPLES

### EXAMPLE 1. Production of Alphabodies specifically binding to the p19 subunit of IL-23

### 1. Production of Alphabody libraries

In one library (referred to as 'scLib_B10', also denoted 'B10') randomized positions where introduced in an Alphabody B-helix and in two other libraries (referred to as 'scLib_AC11b' or 'AC11b' and 'scLib_AC12' or 'AC12', respectively) residues in the A- and C-helices were randomized. The AC11b library comprised Alphabodies with 11 variegated residue positions within the A- and C-helices, the majority of these positions being located at heptad c- and g-positions in the A-helix and at b- and e-positions in the C-helix. The randomized amino acid sequence of the AC11b library is shown in Figure 1A.

The AC12 library comprised Alphabodies with 12 variable residue positions in the groove formed by the A- and C-helices, similar to the AC11b library, but modified with the specific aim to make the variable positions more 'patch-like'. For this purpose, two heptad f-positions in the C-helix were randomized as well. In this library, the L1 and L2 linkers comprise 8-residue Gly/Ser sequences. The randomized amino acid sequence of the AC12 library is shown in Figure 1B.

The B10 library comprised Alphabodies with 9 variable residue positions in the B-helix located at heptad b-, c-, f- and g-positions primarily near the end of helix B. As Alphabody position B3g was included as a variable residue, its H-bonding partner at position C1g was varied too; Alphabody positions are herein referred to using a 3-character notation wherein the first character denotes the helix, the second character denotes the heptad number, and the third character denotes the heptad position (e.g., 'C1g' refers to C-helix, 1st heptad, g-position). Hence, in total 10 residues were variegated in the B10 library. The randomized amino acid sequence of the B10 library is shown in Figure 1C.

These libraries were ordered from GeneArt AG (Germany) and delivered as transformed E. coli cells (strain ER2738, supE strain).

### 2. Production of Phage libraries

The three phage-displayed Alphabody libraries described in this Example were used individually to perform biopanning campaigns on IL-23. In addition, also a mixture of these libraries, referred to as 'mix', was used in a separate biopanning campaign. The phage libraries displayed randomized Alphabody sequences as fusion proteins with the viral pIII protein. The system used for display was a phagemid system resulting in monovalent display, although other display systems can be envisaged as well. The size of the Alphabody libraries corresponded to about 1 E9 (one billion) unique clones (u.c.). It is important that during the rescue of the library the complexity (i.e., diversity) of the library is conserved and that all Alphabody variants are represented in the rescued phage library.

Based on the size of each library and the concentration of the transformed bacteria provided by GeneArt, the inoculum and the start volume for the bacterial culture for phage rescue were calculated. It was aimed to rescue about 10 times more phage than the actual size of the library to ensure preservation of the library diversity.

ScLib_AC12 (AC12) library had a size of 1.4E9 u.c. and the provided stock had 2.3E10 transformed bacteria per ml. 1.4E10 phage were to be rescued, hence 1.4E10/2.3E10 = 0.608 ml stock was used as inoculum. In order not to exceed an OD600nm of 0.05 (containing 1.5E7 transformed bacteria/ml), 933 ml (933=1.4E10/1.5E7) of liquid growth medium was inoculated with 0.608 ml of the transformed bacteria stock.

The same type of calculations were performed for the scLib_B10 library. This library had a size of 2.2E9 u.c. (stock of transformed bacteria: 1.9E10/ml). 2.2E10 phage were rescued, hence 2.2E10/1.9E10= 1.157 ml stock was used as inoculum. In order not to exceed an OD600nm of 0.05 (containing 1.5E7 transformed bacteria/ml), 1466 ml (1466=2.2E10/1.5E7) of liquid growth medium was inoculated with 1.157 ml of the transformed bacteria stock.

The same type of calculations were performed for the scLib_AC11b library. This library had a size of 1.9E9 u.c. (stock of transformed bacteria: 3E10/ml). 1.9E10 phage were rescued, hence 1.9E10/3E10= 0.633 ml stock was used as inoculum. In order not to exceed an OD600nm of 0.05 (containing 1.5E7 transformed bacteria/ml), 1266 ml (1266=1.9E10/1.5E7) of liquid growth medium was inoculated with 0.633 ml of the transformed bacteria stock.

The inoculums (0.608 ml, 1.157 ml and 0.633 ml) of the library stock were transferred to the calculated volumes (respectively 933 ml, 1466 ml and 1266 ml) of growth medium consisting of 2xTY supplemented with 0.1 mg/ml ampicillin and 2% of glucose (2xTY-AG). Of note, the baffle flask containing the growth medium should preferably not be overfilled to maintain good aeration.

The bacterial cultures were grown at 37°C while shaking (300 rpm) in baffle flasks to reach an OD600nm of 0.5 (mid-log phase). The bacterial culture was used for superinfection with helper phage M13K07 (GE Healthcare) to produce phage particles. Before adding the helper phage, a sample of the bacterial culture was taken to determine the total number of viable cells at this stage by titration on 2xTY-AG agar plates. The ratio helper phage/bacteria applied for the superinfection was 20:1. To calculate the concentration of helper phage to be added, the following formula was used: 3E8 x 0.5 x culture volume x 20 = concentration of helper phage to be added to the cultures.

The infection culture was incubated at 37°C for 30 min without shaking to allow infection of the bacteria. After incubation, a sample of the infection culture was taken for titration on 2xTY-AG agar plates and 2xTY-AGK agar plates (K standing for kanamycin (25 microgram/ml) since helper phage are kanamycin resistant) to determine the number of viable and infected bacteria, respectively. The infection culture was then incubated for an additional 30 min, at 37°C while shaking.

After incubation, bacterial cell pellets were obtained by centrifugation of the cultures at 4000 rpm for 10 min at room temperature. The bacterial pellets were resuspended in the same volume of pre-warmed 2xTY-AK medium as the initial startvolume. Cultures were grown overnight at 30°C while shaking (300 rmp).

After overnight incubation, a sample of the culture was taken for titration on 2xTY-AGK agar plates to determine viable cells. The cultures were cooled for 5 min on ice and bacteria were pelleted at 7000 rpm for 20 min. The supernatant was collected to precipitate phage by adding 1/5th of the volume of a solution of 20% PEG 4000/2.5 M NaCl and incubated 1 to 2 hours on ice. After incubation, phage were recovered by centrifugation at 4°C, 7000 rpm for 20 min. Phage pellet was dissolved in 35 ml Phosphate Buffered Saline (PBS)/900 ml of bacterial culture and bacterial debris was removed by 10 min centrifugation at 12000 rpm. A second PEG/NaCl precipitation was performed for 1 hr followed by removal of bacterial debris. Finally, purified phage displaying the library were obtained (10ml/900 ml initial bacterial culture). 15% of glycerol was added to the phage to store at -80°C.

The titer of the phage preparation displaying to the library was determined by infecting bacteria (E. coli TG1 strain) with serial dilutions of purified phage and plating out these dilutions on 2xTY-AG agar plates. This titration allowed to determine the number of infectious phage particles (colony forming units (cfu) or transducing units (TU)). Although the libraries were provided as transformed E. coli ER 2738 bacteria, all further phage propagations were performed using a E. coli TG1 strain (Stratagene), a common strain used in phage display technology.

The expression of Alphabodies was evaluated in Western Blot analysis using an anti-pIII antibody to determine the presence of wild type pIII protein and the Alphabody fusion pIII protein. The molecular weight of the fusion pIII protein is higher than that of the wild type pIII protein and will thus migrate slower in the SDS-PAGE gel. When fusion protein is present, two bands should be visualized using the anti-pIII antibody.

The titers of the rescued libraries of this particular example are shown in Table 1.

**Table 1**

| **Library** | **Size of the library** | **Stock (GeneArt AG)** | **Titer after rescue** |
|---|---|---|---|
| scLib_AC12 | 1.4 x 10⁹ | 2.3 x 10¹⁰/ml | 2 x 10¹⁰, 14 times library size |
| scLib_AC11b | 1.9 x 10⁹ | 3.0 x 10¹⁰/ml | 3.4 x 10¹⁰, 17 times library size |
| scLib_B10 | 2.2 x 10⁹ | 1.9 x 10¹⁰/ml | 1.9 x 10¹⁰, 8 times library size |

For the libraries scLib_AC11b, scLib_AC12 and scLib_B10, titers of 17, 14 and 8 times the size of the library, respectively, were obtained. Western blot analysis showed that for all libraries Alphabody-fusion pIII protein was present (Figure 2). Two bands were visualized. The lower band corresponded to wild type pIII and the upper band, with higher molecular weight, to fusion pIII protein (Figure 2). In conclusion, the rescue of the libraries was successful. Titers of a factor 10 above the initial library size were obtained and, according the Western Blot, all libraries displayed fusion pIII proteins.

Other systems such as dot blots can be used to evaluate the presence of the fusion protein but additionally can be used to determine the display level (i.e., the average number of fusion protein per phage) in case one wants to assess this feature. Two-fold dilutions of phage are typically spotted in duplicate (one for wild type pIII and the other for fusion pIII visualization) on nitrocellulose membranes. The wild type pIII and fusion pIII proteins are visualized using an anti-pIII antibody and an anti-His antibody, respectively. Other anti-tag antibodies can be used depending on the tag displayed by the foreign insert (Myc-tag, HA tag,...). A secondary antibody conjugated to Alkaline Phosphatase detecting both the anti-pIII and anti-His tag antibody allows the colorimetric read-out. Spots of the same intensity in both the wild type pIII and the fusion pIII detection are determined and the ratio of the two phage concentrations of the spots displaying the same intensity in both detection systems will give the display number of the library.

### 3. Biopanning of Alphabody libraries against IL-23

Four separate biopanning campaigns were conducted using each of the individual libraries AC11 b, AC12 and B10, as well as an equal mixture of the three. The capturing of the target IL-23 after incubation with the phage library was performed using a biotinylated anti-p40 IL-23 antibody (Biolegend, 508802; clone C8.6) recognizing the subunit p40 of the cytokine IL-23. Prior to incubation with the library, the cytokine IL-23 (eBioscience, 34-8239-85, lot E034049) was incubated with the anti-p40 antibody. This strategy was developed to drive the selections towards binders of the p19 subunit of IL-23 by (partially) blocking the p40 subunit with an antibody. This particular antibody was then also used to immobilize IL-23 on solid support for washing purposes.

Concretely, variable concentrations of IL-23 were incubated at twice the concentration of biotinylated anti-IL-23 p40 antibody in 0.1% BSA in PBS (phosphate buffered saline, pH 7.2) buffer for 1 hr. The concentrations of IL-23 varied as a function of the selection round, in order to modulate the selection stringency so as to obtain more target-specific phage. Five rounds were performed using 200, 100, 50, 25 and 12.5 nM of IL-23, respectively. In contrast, the concentration of input phage, i.e. phage added to the target in each selection round, remained constant and corresponded to 1E12 phage. To avoid the selection of IgG-Fc binders (in view of the fact that an anti-p40 antibody was used to capture IL-23), from the second round on, 20 micromolar of whole IgG (Sanguin, The Netherlands) was added to the IL-23/anti-p40 antibody/phage mixture. The phage were incubated with the target (anti-p40+IL-23) for 1 hr at room temperature followed by capturing on 0.1 ml streptavidin-coated magnetic Dynabeads (Dynabeads M280 Streptavidin, Invitrogen) for 30 min. Prior to their use, the magnetic beads were washed as recommended by the manufacturer and blocked with 0.1% BSA in PBS for 1 hr at room temperature on a rotating wheel.

After capturing, the magnetic beads were then washed 10 times with 1 ml of PBS containing 0.1 % of Tween 20 to eliminate non-specific phage. Magnetic beads can be easily washed by using a magnet as known to anyone skilled in the art of biopanning.

Target-specific phage were eluted from the beads using 0.2 ml glycine-HCl buffer pH 2 for 5 min., followed by neutralization with 0.05 ml of Tris buffer pH 8. A supplementary fraction was recovered by adding directly mid-log bacteria to the magnetic beads.

A sample of the eluted phage was used to prepare a ten-fold dilution series (in the range dilutions (1 E-1 to 1E-4) in 2xTY for titration by infection of E. coli TG1 bacteria grown to mid-log phase (OD600nm = 0.5). Ten-fold dilutions (in the range 1E-7 to 1E-9) were also prepared from a small sample of the phage used as input in the selection round. These titration results allow the calculation of the yield of the selection round using the formula described earlier.

In this example, 0.9 ml bacteria were added to the 0.1 ml phage dilutions and incubated for 30 min at 37°C. 0.1 ml of the infection culture is plated on 2xTY-AG agar plates for colony counting and titer determination.

The eluted phage were used to infect 20 ml of mid-log phage E. coli TG1 bacteria during 30 min at 37°C. After incubation, a bacterial pellet was obtained by centrifugation at 4000 rpm for 10 min. The pellet was resuspended in 2 ml of 2xTY and plated out on two big 2xTY-AG agar plates (25x25cm).

0.25 ml of mid-log phase bacteria were added directly to the magnetic beads and incubated for 30 min at 37°C. A sample was taken for titration as previously described. The infection culture was plated out on a big 2xTY-AG agar plate.

The next day, colonies on each plate were counted and the titer of the input and output phage was determined for yield calculations. Bacteria on the big agar plates corresponding to the large infection culture were scraped from the plate using LB, the number of bacteria was determined by measuring the OD600nm and 15% glycerol was added to store at -80°C. Phage were rescued from these glycerol stocks as previously described to obtain purified phage for the consecutive selections rounds. 1 E12 phage were taken for the next selection round.

The titers obtained for the 5 selection rounds with the individual and pooled libraries on IL-23 are shown in Table 2.

**Table 2**

| **A** | **Library scLib_AC11b** | | |
|---|---|---|---|
| **Round** | **IL-23 concentration (nM)** | **INPUT titer** | **OUTPUT titer** |
| 1 | 200 | 1x10¹² | 15x10⁵ |
| 2 | 100 | 1x10¹² | 13x10⁵ |
| 3 | 50 | 1x10¹² | 11x10⁶ |
| 4 | 25 | 1x10¹² | 10x10⁷ |
| 5 | 12.5 | 1x10¹² | 4x10⁷ |

| **B** | **Library scLib_AC12** | | |
|---|---|---|---|
| **Round** | **IL-23 concentration (nM)** | **INPUT titer** | **OUTPUT titer** |
| 1 | 200 | 1x10¹² | 9x10⁵ |
| 2 | 100 | 1x10¹² | 3x10⁶ |
| 3 | 50 | 1x10¹² | 24x10⁶ |
| 4 | 25 | 1x10¹² | 43x10⁶ |
| 5 | 12.5 | 1x10¹² | 17x10⁶ |

| **C** | **Library scLib_B10** | | |
|---|---|---|---|
| **Round** | **IL-23 concentration (nM)** | **INPUT titer** | **OUTPUT titer** |
| 1 | 200 | 1x10¹² | 3x10⁵ |
| 2 | 100 | 1x10¹² | 6x10⁵ |
| 3 | 50 | 1x10¹² | 9x10⁶ |
| 4 | 25 | 1x10¹² | 28x10⁶ |
| 5 | 12.5 | 1x10¹² | 2x10⁷ |

| **D** | **Mixture of libraries** | | |
|---|---|---|---|
| **Round** | **IL-23 concentration (nM)** | **INPUT titer** | **OUTPUT titer** |
| 1 | 200 | 1x10¹² | 9x10⁵ |
| 2 | 100 | 1x10¹² | 3x10⁶ |
| 3 | 50 | 1x10¹² | 23x10⁶ |
| 4 | 25 | 1x10¹² | 9x10⁷ |
| 5 | 12.5 | 1x10¹² | 22x10⁶ |

For biopanning with the AC11b and AC12 library, the highest yield was obtained after four selection rounds (Table 2). Biopanning with the library B10 resulted in the highest yield after 5 rounds (Table 2). For all biopanning campaigns, enrichments were observed since the output titer increased between 48 and 100 times.

To isolate target-positive clones from the different selection rounds and to further determine the efficacy of the biopanning, screening by ELISA assays was performed. In this assay, supernatant from small volume bacterial cultures was tested. These bacterial cultures corresponded to individual clones (i.e., individual phage) randomly picked from the titration plates from the different selection rounds. These bacterial clones were grown in 96 deep-well plates in 0.12 ml 2xTY-AG at 30°C overnight while shaking (180 rpm) (MASTERPLATE). The next day, 0.002 ml of this plate was used to inoculate 0.1 ml/well of 2xTY-A without glucose and M13K07 helper phage were added (2E9 plaque-forming units/0.02 microliter/well) immediately. After 2.5 hours of incubation at 37°C while shaking (180 rpm), 0.030 ml 2xTY-AK (Amp: 0.1 mg/ml and Kan: 0.05 mg/ml) was added to the cultures and further incubated overnight for phage propagation at 30°C while shaking (180 rpm).

For the masterplate, 0.020 ml of 80% glycerol was added for storage at -80°C. The masterplate serves to grow individual positive clones and subsequent phage purification for further characterization of their target interaction.

In this example, 44 clones per selection round from the 4 biopanning campaigns were screened. No clones were screened from the first selection round since the expectations to isolate target-specific clones from this round are low.

The set-up of the ELISA was as follows. Neutravidin was immobilized on the plate at a concentration of 0.010 mg/ ml in PBS (0.100 ml) for 1 hour at room temperature (RT). Plates were washed 5 times with PBS containing 0.05% Tween 20 (PBST) for 5 min. Subsequently, 0.1 ml Biotinylated anti-p40 antibody (100 nM) was added to the plates in PBS containing 0.1% BSA and incubated overnight at 4°C or at RT for 1 hour. After incubation, plates were washed 5 times with PBST and blocked with PBS containing 0.1 % BSA and 0.5% gelatin (0.120 ml /well) for at least 1 hour at room temperature or overnight at 4°C. After washing 5 times with PBST, 100 nM IL-23 (target) was added to 0.1 ml PBS, 0.1 % BSA. For individual negative controls no IL-23 was added (background).

In the meantime, the bacteria plates were centrifuged at 1700 rpm for 10 min to pellet the bacteria. Plates with the immobilized IL-23 were washed 5 times with PBST and 0.050 ml of PBS with 0.2% BSA were added to the plate together with 0.050 ml of the bacterial culture supernatant containing phage. Plates were incubated for 1 to 1.5 hours at room temperature while shaking. Shaking of the plates enhanced the ELISA signals.

After the incubation, the plates were washed 5 times with PBST and incubated with an anti-M13 antibody conjugated to HRP diluted 1:5000 in 2% PBS containing 0.1% BSA (0.1 ml /well) for 40 min at room temperature while rocking. Plates were washed 5 times and TMB (substrate of HRP) solution (0.1 ml /well) was added and plates were incubated in the dark between 5 to 30 min. The reaction was stopped by adding 0.05 ml 2N H2SO4 to each well and the plates were read at 450 nm.

Clones were considered positive when the signal on the target was at least 3 times above background.

In this example, 3 biopanning campaigns (using the AC11b, AC12 and mixed libraries) resulted in a positive correlation between the percentage of positive clones determined by ELISA and the consecutive selection rounds (Figure 3). The library B10 performed less well and only a weak number of positive clones was retrieved from this library. Clearly, this indicates that the Alphabodies tend to preferably recognize the IL-23 target in a groove-binding mode. For the AC11b and AC12 libraries, 90% or more positive clones were obtained after 4 selection rounds (Figure 3).

This ELISA can also be performed using soluble Alphabodies instead of Alphabodies displayed on phage. The production of soluble Alphabodies is based on the catabolic repression of the lacZ promoter by using glucose free conditions and the isopropylthio-beta-galactosidase (IPTG) induction of transcription by inactivating the laclq repressor on the bacterial genome. The gene coding for Alphabody is transcribed and soluble Alphabody is produced. More precisely, bacterial colonies are randomly picked from the titration plates of the selection rounds and grown in 96 well plates in 0.12 ml 2xTY-AG at 30°C, overnight while shaking (180 rpm). The next day, 0.002 ml of this plate is used to inoculate 0.1 ml /well of 2xTY-A with 0.1% glucose and further grown at 37°C to reach an OD600 nm of 0.9 (approximately 2 to 3 hours). Then, 0.03 ml 2xTY-A with 3.3 mM IPTG is added to the cultures and further incubated at 30°C while shaking (180 rpm) for 16 to 18 hours. After the induction of the expression with IPTG, 0.014 ml of freshly prepared B-per (Pierce) is added per well and incubated for 15 min at room temperature while mixing. The supernatant is then used in ELISA assays.

The Alphabody sequences were determined for all positive clones binding to IL-23 from the AC12 and AC11b biopanning campaigns (clones were picked from different biopanning rounds). These sequences were determined by the standard DNA sequencing service of the VIB Genetic Service Facility, University of Antwerp (Belgium) using Sanger sequencing and M13RS sequencing primer. Table 3 shows a multiple alignment of 77 Alphabody sequences resulting from the AC12 library against IL-23.

Table 4 shows a multiple alignment of 50 Alphabody sequences resulting from the AC11b library against IL-23.

These Alphabodies can also be readily made as soluble Alphabodies (i.e., outside the phage format) as described above, and can be subsequently purified by a standard Ni-NTA/SEC procedure as known to anyone skilled in the art of protein purification. The Ni-NTA purification is a straightforward first purification step for the soluble Alphabodies containing a His-tag either at their C-terminal end (as is the case in the Alphabody libraries AC12 and B10) or at their N-terminus as a result of a recloning step wherein a given Alphabody gene is excised from its phage context (by standard molecular biology techniques) and inserted into a suitable expression vector such as e.g. pET16 wherein a His-tag and protease cleavage site precedes the Alphabody gene. To determine the Kd (dissociation constant) for binding to IL-23, the soluble Alphabodies are subjected to a (kinetic) Biacore analysis or to Friguet (indirect ELISA) analysis (Friguet et al., 1985, J. Immunol. Methods 77:305-319) or to another appropriate method as known to anyone skilled in the art of measuring binding strengths.

### 4. Testing of cross-reactivity

The cross-reactivity with mouse IL-23 was studied using ELISA assays in which mouse IL-23 was captured by a mouse anti-p40 antibody in analogy with the human IL-23 strategy. The ELISA assays were performed as previously described and clones were considered positive when their signal on target was at least 2.5 times above the signal on background. It was observed that for the IL-23 positive clones resulting from the AC11b and AC12 library respectively 34% and 26% of the analyzed clones were cross-reactive with mouse IL-23 (ratio target over background, T/B, > 2.5).

### 5. Domain specificity

The domain specificity of the 127 different clones was also tested on human IL-12.The ELISA was performed as described for IL-23. Human IL-12 was captured on the plate via an anti-human p40 antibody and the ELISA was performed as previously described. The ELISA results showed that 3/50, 5/76 and 0 clones from respectively the AC11b, AC12 and B10 libraries were cross-reactive with human IL-12. For the AC12 library, 4 out of the 5 human IL-12-positive clones also cross-reacted with mouse IL-23.

**Table 3**

| | |
|---|---|
| SEQ_ID1 | GSIEQIQKW+A*IQEWIAR+QKSIYAMTGGSGGSGGGSIEQIQKQIAAIQKQIAAIQKQIYAMTGSGGGGSGGSIEQIQKQIRAISEQIVAIMLQIMAMTP |
| SEQ_ID2 | GSIEQIQKGIARIQEVIAKIQKGIYAMTGGSGGSGGGSIEQIQKQIAAIQKQIAAIQKQIYAMTGSGGGGSGGSIEQIQKQIVAITHQITAIIWQIWAMTP |
| SEQ_ID3 | GSIEQIQKRIAFIQETIAWIQKNIYAMTGGSGGSGGGSIEQIQKQIAAIQKQIAAIQKQIYAMTGSGGGGSGGSIEQIQKQISAIARQIRAILGQIFAMTP |
| SEQ_ID4 | GSIEQIQKTIAMIQEYIAWIQKKIYAMTGGSGGSGGGSIEQIQKQIAAIQKQIAAIQKQIYAMTGSGGGGSGGSIEQIQKQIRAIVGQIMAILRQITAMTP |
| SEQ_ID5 | GSIEQIQKFIANIQELIACIQKNIYAMTGGSGGSGGGSIEQIQKQIAAIOKOIAAIQKQIYAMTGSGGGGSGGSIEQIQKQITAIASQIYAIVAQITAMTP |
| SEQ_ID6 | GSIEQIQKGIALIQEWIAWIQKSIYAMTGGSGGSGGGSIEQIQKQIAAIQKQIAAIQKQIYAMTGSGGGGSGGSIEQIQKQILAISLQIMAILEQIMAMTP |
| SEQ_ID7 | GSIEQIQKIIAGIQEGIASIQK*IYAMTGGSGGSGGGSIEQIQKQIAAIQKQIAAIQKQIYAMTGSGGGGSGGSIEQIQKQISAIVQQIMAIFAQITAMTP |
| SEQ_ID8 | GSIEQIQKYIAPIQEIIAKIQKLIYAMTGGSGGSGGGSIEQIQKQIAAIQKQIAAIQKQIYAMTGSGGGGSGGSIEQIQKQIGAIISQIGAILGQIYAMTP |
| SEQ_ID9 | GSIEQIQKKIATIQEYIAYIQKFIYAMTGGSGGSGGGSIEQIQKQIAAIQKQIAAIQKQIYAMTGSGGGGSGGSIEQIQKQIKAILGQIGAIIGQIWAMTP |
| SEQ_ID10 | GSIEQIQKKIAVIQEVIAGIQKGIYAMTGGSGGSGGGSIEQIQKQIAAIQKQIAAIQKQIYAMTGSGGGGSGGSIEQIQKQISAIIIQITAIVKQIMAMTP |
| SEQ_ID11 | GSIEQIQKYIAMIQE*IALIQKSIYAMTGGSGGSGGGSIEQIQKQIAAIQKQIAAIQKQIYAMTGSGGGGSGGSIEQIQKQIAAIARQIFAIINQITAMTP |
| SEQ_ID12 | GSIEQIQK+IA+IQE+IANIQKRIYAMTGGSGGSGGGSIEQIQKQIAAIQKQIAAIQKQIYAMTGSGGGGSGGSIEQIQKQIRAI+EQIAAIF+QIFAMTP |
| SEQ_ID13 | GSIEQIQKRIAPIQECIAFIQKLIYAMTGGSGGSGGGSIEQIQKQIAAIQKQIAAIQKQIYAMTGSGGGGSGGSIEQIQKQITAIGRQIMAIFIQIWAMTP |
| SEQ_ID14 | GSIEQIQKRIARIQEPIA*IQKGIYAMTGGSGGSGGGSIEQIQKQIAAIQKQIAAIQKQIYAMTGSGGGGSGGSIEQIQKQIWAISQQITAIVIQIFAMTP |
| SEQ_ID15 | GSIEQIQK+IA+IQEWIAQIQK+IYAMTGGSGGSGGGSIEQIQKQIAAIQKQIAAIQKQIYAMTGSGGGGSG6SIEQIQKQIWAIVSQI+AILVQI+AMTP |
| SEQ_ID16 | GSIEQIQKVIAYIQEKIAVIQKSIYAMTGGSGGSGGGSIEQIQKQIAAIQKQIAAIQKQIYAMTGSGGGGSGGSIEQIQKQI*AIGSQITAIVRQILAMTP |
| SEQ_ID17 | GSIEQIQKRIAGIQERIA+IQK+IYAMTGGSGGSGGGSIEQIQKQIAAIQKQIAAIQKQIYAMTGSGGGGSGGSIEQIQKQIEAIS+QIVAIIGQILAMTP |
| SEQ_ID18 | GSIEQIQKTIASIQEVIAAIQKYIYAMTGGSGGSGGGSIEQIQKQIAAIQKQIAAIQKQIYAMTGSGGGGSGGSIEQIQKQIAAIGSQIIAIVRQIRAMTP |
| SEQ_ID19 | GSIEQIQKTIAAIQECIARIQKAIYAMTGGSGGSGGGSIEQIQKQIAAIQKQIAAIQKQIYAMTGSGGGGSGGSIEQIQKQIRAIVSQISAILIQIGAMTP |
| SEQ_ID20 | GSIEQIQKVIARIQEVIASIQKYIYAMTGGSGGSGGGSIEQIQKQIAAIQKQIAAIQKQIYAMTGSGGGGSGGSIEQIQKQIGAIVTQILAIISQITAMTP |
| SEQ_ID21 | GSIEQIQKSIARIQEGIAPIQKMIYAMTGGSGGSGGGSIEQIQKQIAAIQKQIAAIQKQIYAMTGSGGGGSGGSIEQIQKQIMAIAGQIGAIL*QIRAMTP |
| SEQ_ID22 | GSIEQIQKMIAPIQELIARIQKDIYAMTGGSGGSGGGSIEQIQKQIAAIQKQIAAIQKQIYAMTGSGGGGSGGSIEQIQKQIGAITRQILAILVQIGAMTP |
| SEQ_ID23 | GSIEQIQKFIASIQECIARIQKTIYAMTGGSGGSGGGSIEQIQKQIAAIQKQIAAIQKQIYAMTGSGGGGSGGSIEQIQKQIKAIRTQIFAIFRQIYAMTP |
| SEQ_ID24 | GSIEQIQKPIALIQESIA*IQKYIYAMTGGSGGSGGGSIEQIQKQIAAIQKQIAAIQKQIYAMTGSGGGGSGGSIEQIQKQIFAI+RQIMAILRQINAMTP |
| SEQ_ID25 | GSIEQIQKYIARIQEKIAYIQKMIYAMTGGSGGSGGGSI-EQIQKQIAAIQKQIAAIQKQIYAMTGSGGGGSGGSIEQIQKQIIAIGSQILAILDQIYAMTP |
| SEQ_ID26 | GSIEQIQKLIAVIQEYIALIQKKIYAMTGGSGGSGGGSIEQIQKQlAAIQKQlAAIQKQIYAMTGSGGGGSGGSIEQIQKQlKAIATQISAIIRQIFAMTP |
| SEQ_ID27 | GSIEQIQKWIAQIQENIADIQKLIYAMTGGSGGSGGGSIEQIQKQIAAIQKQIAAIQKQIYAMTGSGGGGSGGSIEQIQKQIPAIAYQILAIIRQISAMTP |
| SEQ_ID28 | GSIEQIQKWIAGIQEAIA*IQKLIYAMTGGSGGSGGGSIEQIQKQIAAIQKQIAAIQKQIYAMTGSGGGGSGGSIEQIQKQIRAIRSQIRAILSQIIAMTP |
| SEQ_ID29 | GSIEQIQKLIARIQESIAMIQKKIYAMTGGSGGSGGGSIEQIQKQIAAIQKQIAAIQKQIYAMTGSGGGGSGGSIEQIQKQIIAIAKQILAIVSQIKAMTP |
| SEQ_ID30 | GSIEQIQKLIAFIQEGIASIQK*IYAMTGGSGGSGGGSIEQIQKQIAAIQKQIAAIQKQIYAMTGSGGGGSGGSIEQIQKQIAAIGNQIMAILQQIKAMTP |
| SEQ_ID31 | GSIEQIQKTIARIQEGIAVIQKLIYAMTGGSGGSGGGSIEQIQKQIAAIQKQIAAIQKQIYAMTGSGGGGSGGSIEQIQKQIRAIVRQITAIMTQIFAMTP |
| SEQ_ID32 | GSIEQIQKAIARIQE*IAIIQKKIYAMTGGSGGSGGGSIEQIQKQIAAIQKQIAAIQKQIYAMTGSGGGGSGGSIEQIQKQIVAIIAQIAAIIPQIIAMTP |
| SEQ_ID33 | GSIEQIQKGIAPIQEMIASIQKVIYAMTGGSGGSGGGSIEQIQKQIAAIQKQIAAIQKQIYAMTGSGGGGSGGSIEQIQKQIMAIAFQIFAIMRQILAMTP |
| SEQ_ID34 | GSIEQIQKPIA*IQERIAWIQKRIYAMTGGSGGSGGGSIEQIQKQIAAIQKQIAAIQKQIYAMTGSGGGGSGGSIEQIQKQIEAITGQIVAIVFQIYAMTP |
| SEQ_ID35 | GSIEQIQK*IAKIQEFIARIQKVIYAMTGGSGGSGGGSIEQIQKQIAAIQKQIAAIQKQIYAMTGSGGGGSGGSIEQIQKQICAIAVQIDAILGQILAMTP |
| SEQ_ID36 | GSIEQIQKFIAPIQEYIAAIQKIIYAMTGGSGGSGGGSIEQIQKQIAAIQKQIAAIQKQIYAMTGSGGGGSGGSIEQIQKQIAAIASQIKAIVTQIVAMTP |
| SEQ_ID37 | GSIEQIQKIIAGIQE*IALIQKAIYAMTGGSGGSGGGSIEQIQKQIAAIQKQIAAIQKQIYAMTGSGGGGSGGSIEQIQKQIYAIGLQILAIMNQIWAMTP |
| SEQ_ID38 | GSIEQIQKFIASIQESIARIQKSIYAMTGGSGGSGGGSIEQIQKQIAAIQKQIAAIQKQIYAMTGSGGGGSGGSIEQIQKQITAIARQIVAIIVQITAMTP |
| SEQ_ID39 | GSIEQIQKFIAAIQEYIATIQK*IYAMTGGSGGSGGGSIEQIQKQIAAI+KQIAAIQKQIYAMTGSGGGGSGGSIEQIQKQIGAIAYQIIAIVNQIKAMTP |
| SEQ_ID40 | GSIEQIQKGIAIIQETIAYIQKSIYAMTGGSGGSGGGSIEQIQKQIAAIQKQIAAIQKQIYAMTGSGGGGSGGSIEQIQKQITAIARQITAIIAQIFAMTP |
| SEQ_ID41 | GSIEQIQKTIA*IQEPIAIIQKRIYAMTGGSGGSGGGSIEQIQKQIAAIQKQIAAIQKQIYAMTGSGGGGSGGSIEQIQKQIKAITSQISAIMSQIWAMTP |
| SEQ_ID42 | GSIEQIQKVIAPIQEYIAIIQKYIYAMTGGSGGSGGGSIEQIQKQIAAIQKQIAAIQKQIYAMTGSGGGGSGGSIEQIQKQISAIMRQIYAIISQIQAMTP |
| SEQ_ID43 | GSIEQIQKLIASIQEYIATIQKLIYAMTGGSGGSGGGSIEQIQKQIAAIQKQIAAIQKQIYAMTGSGGGGSGGSIEQIQKQISAIMIQINAILGQIFAMTP |
| SEQ_ID44 | GSIEQIQKGIAVIQETIA*IQKIIYAMTGGSGGSGGGSIEQIQKQIAAIQKQIAAIQKQIYAMTGSGGGGSGGSIEQIQKQILAIAQQIHAIVSQIVAMTP |
| SEQ_ID45 | GSIEQIQK+IA+IQEAIA+IQKVIYAMTGGSGGSGGGSIEQIQKQIAAIQKQIAAIQKQIYAMTGSGGGGSGGSIEQIQKQI*AIWEQIAAILKQIVAMTP |
| SEQ_ID46 | GSIEQIQKRIAYIQEAIARIQKWIYAMTGGSGGSGGGSIEQIQKQIAAIQKQIAAIQKQIYAMTGSGGGGSGGSIEQIQKQIGAI+MQILAIF+QI+AMTP |
| SEQ_ID47 | GSIEQIQKKIAGIQEVIALIQKFIYAMTGGSGGSGGGSIEQIQKQIAAIQKQIAAIQKQIYAMTGSGGGGSGGSIEQIQKQIRAISSQIQAIVLQILAMTP |
| SEQ_ID48 | GSIEQIQKFIAAIQEYIATIQK*IYAMTGGSGGSGGGSIEQIQKQIAAIQKQIAAIQKQIYAMTGSGGGGSGGSIEQIQKQIGAIAYQIIAIVNQIKAMTP |
| SEQ_ID49 | GSIEQIQKGIAPIQEPIA*IQKLIYAMTGGSGGSGGGSIEQIQKQIAAIQKQIAAIQKQIYAMTGSGGGGSGGSIEQIQKQI*AIANQIRAIINQIMAMTP |
| SEQ_ID50 | GSIEQIQKAIAKIQETIAFIQKSIYAMTGGSGGSGGGSIEQIQKQIAAIQKQIAAIQKQIYAMTGSGGGGSGGSIEQIQKQITAIAGQIYAILQQIFAMTP |
| SEQ_ID51 | G+IE+I+K*IAGIQEWIAPI+KRIYAMTGGSGGSGGG+IEQIQKQIAAIQKQIAAIQK+IYAMTGSGGGGSGGSIEQI+KQISAIIDQI+AIFAQIIAMTP |
| SEQ_ID52 | GSIEQIQK*IAGIQEWIAPIQKRIYAMTGGSGGSGGGSIEQIQKQIAAIQKQIAAIQKQIYAMTGSGGGGSGGSIEQIQKQISAIIDQIRAIFAQIIAMTP |
| SEQ_ID53 | GSIEQIQK+IAWIQEYIA+IQK+IYAMTGGSGGSGGGSIEQIQKQlAAIQKQlAAIQKQIYAMTGSGGGGSGGSIEQIQKQI+AIATQILAIV+QIVAMTP |
| SEQ_ID54 | GSIEQIQKFIAMIQEVIA-IQKNIYAMTGGSGGSGGGSIEQIQKQIAAIQKQIAAIQKQIYAMTGSGGGGSGGSIEQIQKQIPAIREQIKAILHQITAMTP |
| SEQ_ID55 | GSIEQIQKRIA*IQEPIANIQKRIYAMTGGSGGSGGGSIEQIQKQIAAIQKQIAAIQKQIYAMTGSGGGGSGGSIEQIQKQIEAITNQIWAIITQIWAMTP |
| SEQ_ID56 | GSIEQIQKVIA+IQEYIAGIQK+IYAMTGGSGGSGGG+IE+IQKQIAAI+KQIAAIQKQIYAMTG+GGGGS+GS+EQI+KQIRAITSQIWAII+QIIAMTP |
| SEQ_ID57 | GSIEQIQKYIA+IQETIA+IQKLIYAMTGGSGGSGGGSIEQIQK+IA+IQKQIAAIQKQIYAMTGSG+GGSGGSIEQIQKQISAII+QI+AIL+QIPAMTP |
| SEQ_ID58 | GSIEQIQKVIAQIQE*IAMIQKAIYAMTGGSGGSGGGSIEQIQKQIAAIQKQIAAIQKQIYAMTG.SGGGGSGGSIEQIQKQIRAIIMQISAIFNQIFAMTP |
| SEQ_ID59 | GSIEQIQKWIALIQEKIARIQKDIYAMTGGSGGSGGGSIEQIQKQIAAIQKQIAAIQKQIYAMTGSGGGGSGGSIEQIQKQINAIAGQILAIATQIMAMTP |
| SEQ_ID60 | GSIEQIQKVIASIQERIA+IQKRIYAMTGGSGGSGGGSIEQIQKQIAAIQKQIAAIQKQIYAMTGSGGGGSGGSIEQIQKQIGAIMLQIMAIIRQIPAMTP |
| SEQ_ID61 | GSIEQIQKRIAGIQEYIAKIQKSIYAMTGGSGGSGGGSI8QIQKQIAAIQKQIAAIQKQIYAMTGSGGGGSGGSIEQIQKQISAISRQIVAIVSQILAMTP |
| SEQ_ID62 | GSIEQIQKNIAPIQEVIARIQKCIYAMTGGSGGSGGGSIEQIQKQlAAIQKQlAAIQKQIYAMTGSGGGGSGGSIEQIQKQI*AISSQlRAILTQILAMTP |
| SEQ_ID63 | GSIEQIQKTIAWIQESIANIQKGIYAMTGGSGGSGGGSIEQIQKQlAAIQKQlAAIQKQIYAMTGSGGGGSGGSIEQIQKQlRAIG*QIIAIVEQIWAMTE |
| SEQ_ID64 | GSIEQIQKVIARIQEPIAVIQKMIYAMTGGSGGSGGGSIEQIQKQIAAIQKQIAAIQKQIYAMTGSGGGGSGGSIEQIQKQISAIRSQILAIIRQIFAMTI |
| SEQ_ID65 | GSIEQIQKTIAKIQERIAWIQKVIYAMTGGSGGSGGGSIEQIQKQIAAIQKQIAAIQKQIYAMTGSGGGGSGGSIEQIQKQIKAISYQIIAIMRQILAMTP |
| SEQ_ID66 | GSIEQIQKHIA*IQEKIAWIQKLIYAMTGGSGGSGGGSIEQIQKQIAAIQKQIAAIQKQIYAMTGSGGGGSGGSIEQIQKQISAIIGQIYAIASQIMAMTP |
| SEQ_ID67 | GSIEQIQKVIAWIQESIASIQKRIYAMTGGSGGSGGGSIEQIQKQIAAIQKQIAAIQKQIYAMTGSGGGGSGGSIEQIQKQIQAIANQITAIVRQIIAMTP |
| SEQ_ID68 | GSIEQIQKVIA*IQEYIAWIQKSIYAMTGGSGGSGGGSIEQIQKQIAAIQKQIAAIQKQIYAMTGSGGGGSGGSIEQIQKQIRAIAMQIEAIITQIRAMTP |
| SEQ_ID69 | GSIEQIQKGIAAIQ++IAMI+KSIYAMTGGSGGSGGGSIEQI++QIA+IQKQIAAIQKQIYAMTG++GGGSGGSIE+I+KQITAI+TQI+AI+SQIL+MT+ |
| SEQ_ID70 | GSIEQIQKCIAFIQERIAGIQKRIYAMTGGSGGSGGGSIEQIQKQIAAIQKQIAAIQKQIYAMTGSGGGGSGGSIEQIQKQIFAIGKQIFAIVKQILAMTP |
| SEQ_ID71 | GSIEQIQKPIAAIQEKIARIQKRIYAMTGGSGGSGGGSIEQIQKQIAAIQKQIAAIQKQIYAMTGSGGGGSGGSIEQIQKQIMAINRQILAILRQILAMTP |
| SEQ_ID72 | GSIEQIQKYIA*IQEKIAWIQKMIYAMTGGSGGSGGGSIEQIQKQIAAIQKQIAAIQKQIYAMTGSGGGGSGGSIEQIQKQIVAISFQIWAIVRQITAMTP |
| SEQ_ID73 | GSIEQIQKFIASIQECIASIQKVIYAMTGGSGGSGGGSIEQIQKQIAAIQKQIAAIQKQIYAMTGSGGGGSGGSIEQIQKQ.IGAIAAQIIAIVEQIVAMTP |
| SEQ_ID74 | GSIEQIQKKIAYIQEMIALIQKGIYAMTGGSGGSGGGSIEQIQKQIAAIQKQIAAIQKQIYAMTGSGGGGSGGSIEQIQKQISAIAAQISAIIKQIMAMTP |
| SEQ_ID75 | GSIEQIQKNIAWIQERIAMIQKLIYAMTGGSGGSGGGSIEQIQKQlAAIQKQlAAIQKQIYAMTGSGGGGSGGSIEQIQKQI*AIIYQIVAIIRQIPAMTP |
| SEQ_ID76 | GSIEQIQKLIARIQE*IALIQKRIYAMTGGSGGSGGGSIEQIQKQIAAIQKQIAAIQKQIYAMTGSGGGGSGGSIEQIQKQIVAIMYQIYAIIKQIWAMTP |
| SEQ_ID77 | GSIEQIQKGIAAIQEWIATIQKRIYAMTGGSGGSGGGSIEQIQKQIAAIQKQIAAIQKQIYAMTGSGGGGSGGSIEQIQKQIRAITIQIIAIIQQIWAMTP |

**Table 4**

| |
|---|
| SEQ_ID78 |
| GSIEQIQKKIASIQE+IAGIQKAIYSMTGGSGGSGGGGSGGSGGGSIEQIQKQIAAIQKQIAAIQKQIYAMTGSGGGGSGGSGGGGSGGSIEQIQKQILAISEQIMAIVKQITAMTP |
| SEQ_ID79 |
| GSIEQIQKK+ARIQEAIAVIQKTIYGMTGGSGGSGGGGSGGSGGGSIEQIQKQIAAIQKQIAAIQKQIYAMTGSGGGGSGGSGGGGSGGSIEQIQKQIRAISEQIIAIVKQISAMTP |
| SEQ_ID80 |
| GSIEQIQKSIAMIQETIA*IQKKIYMMTGGSGGSGGGGSGGSGGGSIEQIQKQIAAIQKQIAAIQKQIYAMTGSGGGGSGGSGGGGSGGSIEQIQKQITAIAEQIFAIVKQIQAMTP |
| SEQ_ID81 |
| GSI*QIQKFIAHIQEQ+AV+QKGIYKMTGGSGGSGGGGSGGSGGGSIEQIQKQIAAIQKQIAAIQKQIYAMTGSGGGGSGGSGGGGSGGSIEQIQKQISAIREQITAIIKQIWAMTP |
| SEQ_ID82 |
| GSIEQIQKK+ARIQEHIA*+QKLIYSMTGGSGGSGGGGSGGSGGGSIEQIQKQIAAIQKQlAAIQKQIYAMTGSGGGGSGGSGGGGSGGSIEQIQKQlAAIAEQITAIIKQIAAMTP |
| SEQ_ID83 |
| GSIEQIQKRIARIQEPIANIQKHIY*MTGGSGGSGGGGSGGSGGGSIEQIQKQIAAIQKQlAAIQKQIYAMTGSGGGGSGGSGGGGSGGSIEQIQKQIYAIGEQIFAIIKQILAMTP |
| SEQ_ID84 |
| GSIEQIQKKIAWIQEYIATIQK+IY+MTGGSGGSGGGGSGGSGGGSIEQIQKQIAAIQKQIAAIQKQIYAMTGSGGGGSGGSGGGGSGGSIEQIQKQI+AIAEQIWAILKQITAMTP |
| SEQ_ID85 |
| GSIEQIQKGIAGIQENIA*+QKPIY*MTGGSGGSGGGGSGGSGGGSIEQIQKQIAAIQKQIAAIQKQIYAMTGSGGGGSGGSGGGGSGGSIEQIQKQIRAIAEQIVAITKQIFAMTP |
| SEQ_ID86 |
| GSIEQIQKIIAYIQERIASIQKLIYSMTGGSGGSGGGGSGGSGGGSIEQIQKQIAAIQKQIAAIQKQIYAMTGSGGGGSGGSGGGGSGGSIEQIQKQIFAIAEQI*AIVKQIFAMTP |
| SEQ_ID87 |
| GSIEQIQKPIAKIQETIANIQKYIYRMTGGSGGSGGGGSGGSGGGSIEQIQKQIAAIQKQIAAIQKQIYAMTGSGGGGSGGSGGGGSGGSIEQIQKQIWAIAEQI*AIMKQIVAMTP |
| SEQ_ID88 |
| GSIEQIQKSIARIQEVIAQIQKCIYFMTGGSGGSGGGGSGGSGGGSIEQIQKQIAAIQKQIAAIQKQIYAMTGSGGGGSGGSGGGGSGGSIEQIQKQILAIREQI*AILKQIWAMTP |
| SEQ_ID89 |
| GSIEQIQKPIATIQEYIA*+QKSIYTMTGGSGGSGGGGSGGSGGGSIEQIQKQIAAIQKQIAAIQKQIYAMTGSGGGGSGGSGGGGSGGSIEQIQKQI+AISEQIYAIVKQIVAMTP |
| SEQ_ID90 |
| GSIEQIQKR+A+IQEPIANIQKRIY+MTGGSGGSGGGGSGGSGGGSIEQIQKQIAAIQKQIAAIQKQIYAMTGSGGGGSGGSGGGGSGGSIEQIQKQIGAIAEQIMAILKQIRAMTP |
| SEQ_ID91 |
| GSIEQIQKPIA*IQELIASIQKIIYGMTGGSGGSGGGGSGGSGGGSIEQIQKQIAAIQKQIAAIQKQIYAMTGSGGGGSGGSGGGGSGGSIEQIQKQIAAIREQIGAIIKQISAMTP |
| SEQ_ID92 |
| GSIEQIQK*IANIQEYIAR+QKNIYVMTGGSGGSGGGGSGGSGGGSIEQIQKQIAAIQKQIAAIQKQIYAMTGSGGGGSGGSGGGGSGGSIEQIQKQIWAISEQIWAIIKQITAMTP |
| SEQ_ID93 |
| GSIEQIQKPIA+IQEYIAMIQKAIYLMTGGSGGSGGGGSGGSGGGSIEQIQKQIAAIQKQIAAIQKQIYAMTGSGGGGSGGSGGGGSGGSIEQIQKQIWAIGEQINAI+KQI+AMTP |
| SEQ_ID94 |
| GSIEQIQKRIA*IQEGIAMIQKGIYVMTGGSGGSGGGGSGGSGGGSIEQIQKQIAAIQKQIAAIQKQIYAMTGSGGGGSGGSGGGGSGGSIEQIQKQIRAISEQILAIVKQITAMTP |
| SEQ_ID95 |
| GSIEQIQKPIALIQERIATIQKLIYGMTGGSGGSGGGGSGGSGGGSIEQIQKQIAAIQKQIAAIQKQIYAMTGSGGGGSGGSGGGGSGGSIEQIQKQISAIAEQILAISKQIWAMTP |
| SEQ_ID96 |
| GSIEQIQKWIACIQECIAGIQKEIYIMTGGSGGSGGGGSGGSGGGSIEQIQKQIAAIQKQIAAIQKQIYAMTGSGGGGSGGSGGGGSGGSIEQIQKQIRAISEQIYAIIKQISAMTP |
| SEQ_ID97 |
| GSIEQIQKGIAKIQETIA*IQKVIYWMTGGSGGSGGGGSGGSGGGSIEQIQKQIAAIQKQIAAIQKQIYAMTGSGGGGSGGSGGGGSGGSIEQIQKQIIAISEQIRATVKQIIAMTP |
| SEQ_ID98 |
| GSIEQIQK+IARIQETIAL+QKSIYRMTGGSGGSGGGGSGGSGGGSIEQIQKQIAAIQKQIAAIQKQIYAMTGSGGGGSGGSGGGGSGGSIEQIQKQIGAIREQIYAIIKQIFAMTP |
| SEQ_ID99 |
| GSIEQIQKPIAEIQEIIARIQKKIYIMTGGSGGSGGGGSGGSGGGSIEQIQKQIAAIQKQIAAIQKQIYAMTGSGGGGSGGSGGGGSGGSIEQIQKQISAIGEQIFAIVKQIYAMTP |
| SEQ_ID100 |
| GSIEQIQKSIA*IQEPIAYIQKTIYSMTGGSGGSGGGGSGGSGGGSIEQIQKQIAAIQKQIAAIQKQIYAMTGSGGGGSGGSGGGGSGGSIEQIQKQI*AIAEQIMAIAKQIWAMTP |
| SEQ_ID101 |
| GSIEQIQKSIA*IQEPIARIQKLIYRMTGGSGGSGGGGSGGSGGGSIEQIQKQIAAIQKQIAAIQKQIYAMTGSGGGGSGGSGGGGSGGSIEQIQKQILAIftEQISAIVKQITAMTP |
| SEQ_IDI02 |
| GSIEQIQKIIASIQEPIARIQKGIYRMTGGSGGSGGGGSGGSGGGSIEQIQKQIAAIQKQIAAIQKQIYAMTGSGGGGSGGSGGGGSGGSIEQIQKQIGAIAEQILAIFKQIRAMTP |
| SEQ_ID103 |
| GSIEQIQKPIASIQELIAMIQKAIYWMTGGSGGSGGGGSGGSGGGSIEQIQKQIAAIQKQIAAIQICQIYAMTGSGGGGSGGSGGGGSGGSIEQIQKQIVAISEQIMAIIKQIGAMTP |
| SEQ_ID104 |
| GSIEQIQKNIAYIQERIATIQKKIYRMTGGSGGSGGGGSGGSGGGSIEQIQKQIAAIQKQIAAIQKQIYAMTGSGGGGSGGSGGGGSGGSIEQIQKQIGAIAEQILAIVKQIRAMTP |
| SEQ_ID105 |
| GSIEQIQKRIARIQEKIAWIQKPIYQMTGGSGGSGGGGSGGSGGGSIEQIQKQIAAIQKQIAAIQKQIYAMTGSGGGGSGGSGGGGSGGSIEQIQKQIRAIREQIGAILKQIKAMTP |
| SEQ_IDI06 |
| GSIEQIQKPIANIQE*IACIQKRIYVMTGGSGGSGGGGSGGSGGGSIEQIQKQIAAIQKQIAAIQKQIYAMTGSGGGGSGGSGGGGSGGSIEQIQKQISAISEQIWAILKQIWAMTP |
| SEQ_ID107 |
| GSIEQIQKPIARIQETIAIIQKTIYRMTGGSGGSGGGGSGGSGGGSIEQIQKQIAAIQKQIAAIQKQIYAMTGSGGGGSGGSGGGGSGGSIEQIQKQITAIREQIRAIWKQIPAMTP |
| SEQ_ID108 |
| GSIEQIQK*IAAIQEYIASIQKAIYRMTGGSGGSGGGGSGGSGGGSIEQIQKQIAAIQKQIAAIQKQIYAMTGSGGGGSGGSGGGGSGGSIEQIQKQITAIAEQIWAILKQIPAMTP |
| SEQ_ID109 |
| GSIEQIQKPIAGIQEGIARIQK*IYRMTGGSGGSGGGGSGGS+GGSIEQIQKQIAAIQKQIAAIQKQIYAMTGSGGGGSGGSGGGGSGGSI+QIQKQIAAISEQIKAIVKQIWAMTP |
| SEQ_ID110 |
| GSIEQIQKGIAGIQEAIAPIQKRIY*MTGGSGGSGGGGSGGSGGGSIEQIQKQIAAIQKQIAAIQKQIYAMTGSGGGGSGGSGGGGSGGSIEQIQKQISAIAEQISAIVKQILAMTP |
| SEQ_ID111 |
| GSIEQIQKMIA*IQEYIAGIQKVIYKMTGGSGGSGGGGSGGSGGGSIEQIQKQIAAIQKQIAAIQKQIYAMTGSGGGGSGGSGGGGSGGSIEQIQKQIIAISEQINAIFKQIWAMTP |
| SEQ_ID112 |
| GSIEQIQKFIAGIQESIA*IQKLIYRMTGGSGGSGGGGSGGSGGGSIEQIQKQIAAIQKQIAAIQKQIYAMTGSGGGGSGGSGGGGSGGSIEQIQKQIRAIAEQITAIFKQIMAMTP |
| SEQ_ID113 |
| GSIEQIQK*IA+IQEPIAPIQK+IYMMTGGSGGSGGGGSGGSGGGSIEQIQKQIAAIQKQIAAIQKQIYAMTGSGGGGSGGSGGGGSGGSIEQIQKQIGAITEQINAIFKQIWAMTP |
| SEQ_ID114 |
| GSIEQIQKRIARIQEPIAGIQKRIYMMTGGSGGSGGGGSGGSGGGSIEQIQKQIAAIQKQIAAIQKQIYAMTGSGGGGSGGSGGGGSGGSIEQIQKQIRAIAEQIVAIAKQIVAMTP |
| SEQ_ID115 |
| GSIEQIQKPIA*IQETIAVIQKWIYRMTGGSGGSGGGGSGGSGGGSIEQIQKQIAAIQKQIAAIQKQIYAMTGSGGGG8GGSGGGGSGGSIEQIQKQIRAIAEQITAIVKQIFAMTP |
| SEQ_ID116 |
| GSIEQIQKCIAGIQECIA*IQKWIYNMTGGSGGSGGGGSGGSGGGSIEQIQKQIAAIQKQIAAIQKQIYAMTGSGGGGSGGSGGGGSGGSIEQIQKQIAAIAEQIGAIIKQITAMTP |
| SEQ_ID117 |
| GSIEQIQKGIAPIQERIASIQKKIYRMTGGSGGSGGGGSGGSGGGSIEQIQKQIAA+QKQIAAIQKQIYAMTGSGGG+SGGSGGGGSGGSIEQIQKQIDAISEQIKAIVKQIIAMTP |
| SEQ_ID118 |
| GSIEQIQKPIAPIQERIATIQKFIYRMTGGSGGSGGGGSGGSGGGSIEQIQKQIAAIQKQIAAIQKQIYAMTGSGGGGSGGSGGGGSGGSIEQIQKQI*AITEQIWAIVKQIFAMTP |
| SEQ_ID119 |
| GSIEQIQKGIA*IQERIAQIQKPIY*MTGGSGGSGGGGSGGSGGGSIEQIQKQIAAIQKQIAAIQKQIYAMTGSGGGGSGGSGGGGSGGSIEQIQKQIRAIREQITAIIKQIFAMTP |
| SEQ_ID120 |
| GSIEQIQKKIAKIQEPIA*IQKVIYSMTGGSGGSGGGGSGGSGGGSIEQIQKQIAAIQKQIAAIQKQIYAMTGSGGGGSGGSGGGGSGGSIEQIQKQIRAISEQIRAIIKQIYAMTP |
| SEQ_ID121 |
| GSIEQIQKFIAKIQERIA*IQKNIYTMTGGSGGSGGGGSGGSGGGSIEQIQKQIAAIQKQIAAIQKQIYAMTGSGGGGSGGSGGGGSGGSIEQIQKQIYAISEQIYAIIKQIVAMTP |
| SEQ_ID122 |
| GSIEQIQKRIAPIQESIAGIQKRIYRMTGGSGGSGGGGSGGSGGGSIEQIQKQIAAIQKQIAAIQKQIYAMTGSGGGGSGGSGGGGSGGSIEQIQKQIRAITEQIGAILKQIFAMTP |
| SEQ_ID123 |
| GSIEQIQK*IAPIQEYIAWIQKTIYKMTGGSGGSGGGGSGGSGGGSIEQIQKQIAAIQKQIAAIQKQIYAMTGSGGGGSGGSGGGGSGGSIEQIQKQIYAIGEQILAIFKQIAAMTP |
| SEQ_ID124 |
| GSIEQIQKDIAFIQEPIANIQK*IYRMTGGSGGSGGGGSGGSGGGSIEQIQKQIAAIQKQIAAIQKQIYAMTGSGGGGSGGSGGGGSGGSIEQIQKQIKAIREQIIAIMKQIFAMTP |
| SEQ_ID125 |
| GSIEQIQK*IAPIQEAIAGIQKRIYRMTGGSGGSGGGGSGGSGGGSIEQIQKQIAAIQKQIAAIQKQIYAMTGSGGGGSGGSGGGGSGGSIEQIQKQIAAIGEQIVAILKQILAMTP |
| SEQ_ID126 |
| GSIEQIQK*IATIQEPIALIQKRIYRMTGGSGGSGGGGSGGSGGGSIEQIQKQIAAIQKQIAAIQKQIYAMTGSGGGGSGGSGGGGSGGSIEQIQKQTGAIAEQIGAILKQIWAMTP |
| SEQ_ID127 |
| GSIEQIQKPIAWIQE*IAEIQKRIYTMTGGSGGSGGGGSGGSGGGSIEQIQKQIAAIQKQIAAIQKQIYAMTGSGGGGSGGSGGGGSGGSIEQIQKQIRAIREQIFAIMKQIFAMTP |

### EXAMPLE 2. Production of Alphabodies specifically binding to Flt3L and Flt3R

In the present example the Alphabody platform technology was used to obtain binders against the Flt3L cytokine and on a soluble form of the ectodomain of Flt3R.

Biopanning campaigns were conducted against both human Flt3L (hFlt3L) and soluble human Flt3R ectodomain (hFlt3Re). Recombinant hFlt3L target was produced from E. coli as described in Verstraete et al., Protein J., 2009, 28:57-65. Recombinant hFlt3Re target was produced in mammalian cells as described in Verstraete et al., Acta Cryst F, 2011, 67:325-331. Essentially the same selection and screening protocols were followed as in Example 1. However, three phage-displayed Alphabody libraries, different from the ones in Example 1, were used. The Alphabody library sequences are shown in Figure 4. The first library, referred to as 'scLib_AC11' or 'AC11', shown in Figure 4A, comprised sequences that are highly similar to the AC11 b library of Figure 1b. Exactly the same 11 variable positions in alpha-helices A and C were maintained, but the linker sequences and N-terminal capping motifs were chosen differently. The second library, referred to as 'scLib_AC7' or 'AC7', was based on a smaller Alphabody scaffold wherein in each alpha-helix 7 residues (one heptad) were deleted. This library comprised 7 variegated residue positions located in the A- and C-helices (Figure 4B). Both of the AC11 and AC7 libraries are of the type 'groove library' because amino acid variegation is essentially confined to the groove formed by parallel helices A and C. The third library, referred to as 'scLib_C9' or 'C9' was again based on the 'long' Alphabody scaffold and comprised 9 variable residues located at solvent-oriented positions in the C-helix (Figure 4C). In view of the variable surface region being restricted to a single helix, and the variable positions being highly solvent exposed, this library is also referred to as a 'helix surface' library.

Prior to starting the biopanning experiments, equal amounts of phage from the three libraries AC11, AC7 and C9 were mixed, so the campaigns were executed using library mixtures.

Biopanning was performed against biotinylated forms of the targets hFlt3L and hFlt3Re. Each of these campaigns consisted of at least 5 selection rounds. In each round, 1E12 phage were incubated for 1 hr at R.T. with 500 nM target in 0.1% BSA in PBS, followed by capturing on streptavidin-coated magnetic Dynabeads. Capturing and elution protocols were as in Example 1. Contrary to the panning protocol of Example 1, the target concentration was kept constant over the different rounds. Despite the constant stringency, clear enrichment of phage was seen as of round 3 (R3) for both targets. Output/input phage ratio (O/I ratio) raised by a factor 90 in R3 and a factor 600 in R4 compared to R2 for hFlt3L selections. Round 5 gave no further significant enrichment and this campaign was therefore stopped after R5. The biopanning campaign against hFlt3Re showed slow increases in O/I ratios in consecutive rounds 3 to 5 and therefore the campaign was continued up to round 6. This last round, R6, suddenly gave a steep rise in enrichment, with an O/I ratio 20000 times higher than that of R2.

Individual phage clones were randomly picked from rounds 4 and 5 of both campaigns and were further analyzed in standard phage ELISA according to the method described in Example 1. Four 96-well plates were coated with biotinylated hFlt3L or hFlt3Re (two plates each), except 8 wells in each plate that were left empty for the purpose of signal over background calculations. The phage clones selected from the two campaigns were tested both on a positive plate (i.e., coated with the target they were selected against) and on a negative plate (i.e., coated with the other target) for the purpose of checking target specificity.

Within the population of hFlt3L-selected clones, 57% of them were deemed positive (target over background ratio, T/B > 3). For the clones selected against hFlt3Re, 40% had a T/B > 3. Importantly, not any of the clones selected against any of the targets was found positive on the control plate, suggesting that target recognition was significant and highly specific.

Next, 20 clones having the highest T/B ratio were picked from each of the positive plates and were submitted for sequencing. Surprisingly, all sequences from both campaigns turned out to exclusively arise from the helix surface library C9. The absence of sequences from the groove libraries AC11b or AC12 was not expected because exactly the contrary was observed in the biopanning experiments on IL-23 described in Example 1.

Tables 5a and 5b show the amino acids at each variable position in the Alphabodies selected on hFlt3L and hFlt3Re, respectively. As there were no unintended mutations, only the variable positions are shown. For the hFlt3L and hFlt3Re selected clones, 18 and 19 sequences could be successfully determined, respectively. The hFlt3L set comprised 10 unique sequences, one of which appeared 8 times, another one twice, and the rest only once. The number of times each sequence was found in a given biopanning round (N_Rx) is indicated to the right of Tables 5a and 5b (e.g., N_R4 = 6 means that this sequence appeared 6 times within the set selected from biopanning round R4). The hFlt3Re set only comprised 2 unique sequences and was dominated by a single sequence appearing 18 times.

The hFlt3Re data set was too small to permit further sequence analysis, but the hFlt3L set showed clear positional preferences. Overall, a full preservation of tryptophan was observed at position 2f (i.e., 2nd heptad, f-position, in the C-helix of Alphabodies from the C9 library). In addition, position 1f showed a strong preference for either arginine or glycine, position 2b a high frequency of aromatic/aliphatic residues, and position 3c a high occurrence of acidic residues aspartic or glutamic acid. Such high degree of sequence conservation may be indicative of a common binding site on the target molecule hFlt3L. However, the first and second half of the data set in Table 5a also differed at specific positions. For example, a strict conservation of glutamic acid at position 2c was observed in the clones numbered 6-10 and not in 1-5. Clones 7-10 seemed to prefer basic residues (arginine, lysine) at position 3b. Clones 1-5 had exclusively glutamine at position 3f. Finally, clones 1-6 showed only non-polar residues at positions 4b and 4c, whereas the others mainly had polar residues. This situation may be indicative of a common binding region on hFlt3L albeit with different Alphabody binding modes in the complex. Finally, the almost unique sequence of clones selected from round 4 on Flt3Re (Table 5b) suggests a single dominant binding mode to the Flt3 receptor, and explains the high O/I ratios in later rounds, as discussed above. The second sequence, which was found only once, showed an unexpected similarity with Flt3L-selected clones and is perhaps the result of an artefact (contamination) during preparation of clones for sequencing.

**Table 5a**

| # | 1f | 2b | 2c | 2f | 3b | 3c | 3f | 4b | 4c | N_R4 | N_R5 | N_tot |
|---|---|---|---|---|---|---|---|---|---|---|---|---|
| 1 | D | R | V | W | W | D | Q | F | L | 1 | | 1 |
| 2 | R | F | V | W | F | D | Q | I | V | 6 | 2 | 8 |
| 3 | G | Y | R | W | F | D | Q | I | V | | 1 | 1 |
| 4 | D | L | N | W | H | D | Q | I | V | | 1 | 1 |
| 5 | G | L | S | W | F | D | Q | L | V | | 2 | 2 |
| 6 | G | L | E | W | Q | D | M | L | Y | 1 | | 1 |
| 7 | G | I | E | W | R | E | N | S | G | 1 | | 1 |
| 8 | G | I | E | W | R | D | G | D | V | 1 | | 1 |
| 9 | G | I | E | W | K | D | N | K | K | 1 | | 1 |
| 10 | G | L | E | W | R | G | T | V | Q | | 1 | 1 |
| **Consensus** | **R** | **F** | **V** | **W** | **F** | **D** | **Q** | **I** | **V** | | | |

**Table 5b**

| # | 1f | 2b | 2c | 2f | 3b | 3c | 3f | 4b | 4c | N_R4 | N_R5 | N_tot |
|---|---|---|---|---|---|---|---|---|---|---|---|---|
| 1 | W | R | L | L | K | Q | E | F | I | 18 | - | 18 |
| 2 | D | V | E | W | R | V | L | K | R | 1 | - | 1 |

### EXAMPLE 3. Structural basis for the interaction between an Alphabody and hFlt3L

Two his-tagged variants of the Alphabody sequence corresponding to #4 in Table 5a were recombinantly produced in E. coli. Synthetic genes for these constructs were subcloned into the pET16b vector (Novagen) in frame with the N-terminal 10-His tag between the Ndel and BamHI sites. The first variant, herein denoted 'hFlt3L_cl4' and further provided as SEQ ID No: 134, consisted of the native sequence as identified for phage clone #4 of Example 2, N-terminally appended with the 10-His tag sequence of the vector. The second variant, herein denoted 'hFlt3L_cl4m' and further provided as SEQ ID No: 135, consisted of basically the same sequence but with shortened linkers between the alpha-helices and comprising two lysine to serine mutations in both the A- and B-helices of the Alphabody. The full amino acid sequences of hFlt3L_cl4 (SEQ ID No: 134) and hFlt3L_cl4m (SEQ ID No: 135) are aligned Figure 5.

The gene constructs were transformed into host E. coli cells (BL21(DE3)-strain) harboring a chromosomal copy of the T7 polymerase gene under control of the lacUV5 promoter (DE3 lysogens). The cells were grown in standard Luria-Bertani medium (10 l) at 37 °C, in the presence of carbenicillin (0.1 mg/ml). At an optical density of 0.6 - 0.7 at 600 nm, protein expression was induced by the addition of 1 mM isopropyl β-D-1-thiogalactopyranoside (IPTG). The expression culture was incubated overnight. Expression cultures were then centrifuged at 6000 g for 10 min (at 4 °C). The cell pellet was resuspended in 20 mM Tris, 100 mM NaCl, pH 7.5. Next, the cell suspension was subjected to sonication. The insoluble fraction was isolated by centrifugation (20000 g, 20 min, 4 °C) and washed with buffer containing 1% Triton X-100. This wash step was repeated once. Insoluble proteins were solubilized in 50 mM NaH₂PO₄, 300 mM NaCl, 4 M guanidinium hydrochloride, pH 8.0 and incubated on a rotatory-shaker for 2 hours at R.T. Cells debris and other unsolubilized material was removed by centrifugation (1 h, 100000 g, 4 °C). The supernatant was filtered through a 0.22 µm-filter and loaded onto a prepacked Ni Sepharose Fast Flow column (10 mL) (GE Healthcare) connected to an AKTA-system and equilibrated with 50 mM NaH₂PO₄, 300 mM NaCl, 4 M guanidinium hydrochloride, pH 8.0. After loading the sample, the column was washed with equilibration buffer. Next, the running buffer was exchanged for 50 mM NaH₂PO₄, 300 mM NaCl, pH 8.0. Bound protein was eluted using a stepwise gradient with imidazole (with 500 mM as the final concentration). Elution fractions corresponding to the Alphabody were pooled and desalted into trypsin digestion buffer (20 mM Tris, 50 mM Tris, pH 8.0). To remove the N-terminal His-tag, the protein solution was incubated with TPCK-trypsin immobilized on magnetic beads (Clontech) on a rotary shaker for two hours at 37 °C. Next, the magnetic beads were removed by low-speed centrifugation (500 g) and the protein solution was filtered through a 0.22 µm-filter and loaded onto a prepacked Ni Sepharose Fast Flow column. Non-bound protein was collected and concentrated by ultracentrifugation using concentrators with a pore size of 5 kDa. As a polishing step, the Alphabody was injected onto a Superdex 75 column (GE Healthcare) connected to an AKTA-system and equilibrated with 20 mM Tris, 150 mM NaCl, pH 7.4. Fractions corresponding to the Alphabody were stored at -80 °C.

The binding of Alphabody hFlt3L_cl4 to the target hFlt3L was tested by isothermal titration calorimetry (ITC) in order to determine its dissociation constant (K_{D}), thermodynamic parameters (ΔH, ΔS) and binding stoichiometry. Prior to the measurements, Alphabody and target samples were dialyzed against 20 mM HEPES buffer containing 150 mM NaCl, pH 7.4. The ITC measurements were performed in triplicate at a temperature of 37 °C. In each experiment, hFlt3L, which exists as a stable dimer in solution, was loaded into the cell (at concentrations 3.0 µM, 6.5 µM and 7.0 µM of hFlt3L dimer, respectively) and hFlt3L_cl4 was loaded in the syringe (at concentrations of 41.35 µM, 89.62 µM and 89.62 µM, respectively). Figure 6 shows the recorded thermograms. The enthalpy changes were indicative of an exothermic binding reaction, with an average ΔH of -16.6 ± 1.9 kcal/mol. The average binding free energy (ΔG) was derived from the fitted affinity constants, K_{A}, of each thermogram using the equation ΔG = -RT In(K_{A}) and was found to be ΔG = -9.5 ± 0.4 kcal/mol. The average entropy change of binding (ΔS) was derived from the free energy and enthalpy changes using the equation ΔG = ΔH - TΔS and was found to be ΔS = -22.9 ± 6.8 cal/(mol.K). The final affinity and dissociation constants for the three measurements were calculated back from the averaged ΔG value and were found to be K_{A} = 4.95 x 10⁶ M⁻¹ and K_{D} = 202 nM. Finally, the thermograms showed a binding stoichiometry (n) of 0.88 ± 0.10. This implies that, since the concentrations of hFlt3L were given for the dimeric complex (hFlt3L)₂, only one hFlt3L_cl4 Alphabody binds to one hFlt3L dimer.

In a further attempt to characterize Alphabody:hFlt3L binding, steps were undertaken to crystallize complexes and to determine their 3-D structure by X-ray crystallography. Concretely, hFlt3L_cl4m:hFlt3L complexes were formed by incubating purified recombinant hFlt3L (Verstraete et al., Protein J, 2009) with a molar excess of Alphabody. The complex was separated from excess Alphabody by gel filtration using a Superdex 75 column equilibrated with 20 mM Tris, 150 mM NaCl, pH 7.4. Fractions corresponding to the complex were pooled and concentrated using a Vivaspin concentrator to a final concentration of 8 mg/ml. The concentrated protein solution was aliquoted and flash frozen into liquid nitrogen and subsequently stored at -80 °C. Subsequently, initial crystal screening was carried out with a Mosquito crystallization robot (TTP Labtech), using commercially available sparse matrix screens. Crystallization drops were set up at both 277 K and 293 K in 96-well sitting-drop crystallization plates by mixing 100 nl of reservoir solution (45 nl) with 100 nl of protein solution at a concentration of 8 mg/ml. Crystals of the hFlt3L_cl4m:hFlt3L complex suitable for X-ray diffraction experiments appeared overnight in several conditions of the PEG ION 2 screen (Hampton Research) at 277 K. Crystallization of the hFlt3L_cl4m:hFlt3L complex was confirmed by running a silver-stained SDS-PAGE gel of washed crystals. Crystals were then transferred to a drop of mother liquor (5 µl) with the use of a nylon loop (Hampton Research) mounted onto SPINE standard cryocaps (Molecular Dimensions). The concentration of cryoprotectant (PEG 400) was gradually adjusted by adding increasing amounts of mother liquor containing a higher concentration of cryoprotectant to the drop until the desired concentration of cryoprotectant (20%) was reached. A 3-minute equilibration time was allowed between subsequent addictions Subsequently, the crystals were flash-frozen into liquid nitrogen and loaded into SPINE/ESRF pucks for storage and transport. Diffraction experiments were conducted at the X06SA (PXI) beamline of the Swiss Light Source (Paul Scherrer Institute, Villigen, Switzerland). As a result, diffraction data to 1.7 A resolution, for a single hFlt3L_cl4m:hFlt3L crystal grown in 8% v/v Tacsimate pH 4.0, 20% PEG 3350, were obtained and were used for further structural analysis. The data were integrated and scaled using the XDS suite (Kabsch, 2010). The structure was solved using molecular replacement as implemented in Phaser (McCoy et al., 2007) using the high resolution structure of hFlt3L (pdb entry code 1ETE, Savvides et al., 2000) as a search model. The resulting maps showed clear 2FO-FC and positive FO-FC difference density for the hFlt3L_cl4m Alphabody. Model (re)building was carried out manually in Coot (Emsley et al., Acta Cryst D, 2010). Crystallographic refinement was carried out in PHENIX (Adams et al., 2010). Figure 7 shows a ribbon representation of the 3-D structure of the hFlt3L_cl4m:hFlt3L complex. It was observed that one Alphabody molecule binds to one hFlt3L dimer, in agreement with the stoichiometric data obtained previously by ITC. The binding or 'interface' residues on the Alphabody, defined as those amino acid residues having at least one side-chain atom within 4 A from the hFlt3L structure, included all residues located at positions that were randomized in the original Alphabody library (i.e., all x-positions shown in Figure 4C or all positions shown in Table 5a, see EXAMPLE 2). This suggests that all of these library positions contribute to some extent to the Alphabody-hFlt3L interaction. Also, all of these library residues seem to interact with a single monomer (monomer 2 in Figure 7) of the hFlt3L dimer. Central in the interface of the complex is the tryptophan at position 2f in the Alphabody C-helix, which is buried in a relatively hydrophobic pocket on hFlt3L. This confirms the presumed dominant role of this residue which was strictly conserved among the sequences identified from the biopanning campaign (Table 5a). In addition to the library residues, two other residues that had not been randomized are identified as contact residues (according to the 4 A criterion), i.e., the glutamines at positions 2e and 3g in the C-helix. Finally, the linker segment L1 also seems to interact favorably with hFlt3L, albeit with a distinct monomer (monomer 1 in Figure 7).

## Claims

1. An Alphabody polypeptide comprising an Alphabody having the general formula HRS1-L1-HRS2-L2-HRS3, wherein
- each of HRS1, HRS2 and HRS3 is independently a heptad repeat sequence (HRS) consisting of 2 to 7 consecutive heptad repeat units, at least 50% of all heptad a- and d-positions are occupied by isoleucine residues, each HRS starts and ends with an aliphatic or aromatic amino acid residue located at either a heptad a- or d-position, and HRS1, HRS2 and HRS3 together form a triple-stranded, alpha-helical, coiled coil structure; and
- each of L1 and L2 is independently a linker fragment, which covalently connects HRS1 to HRS2 and HRS2 to HRS3, respectively;
wherein said Alphabody specifically binds to a member of a cytokine - cytokine receptor or growth factor - growth factor receptor complex.

2. The Alphabody polypeptide according to claim 1, which is **characterized in that** the binding site is located primarily either
- on the helix surface of the Alphabody,
- in a groove of the Alphabody, involving at least one e or g residue of the Alphabody helix
- in the loop or linker region of the Alphabody.

3. The Alphabody polypeptide according to claim 1, wherein the binding site is formed primarily either:
(i) by heptad e-positions in a first alpha-helix of the Alphabody and by heptad g-positions in a second alpha-helix, and optionally by heptad b-positions in said first alpha-helix of the Alphabody and/or by heptad c-positions in said second alpha-helix of the Alphabody, or
(ii) by heptad b-, c- and f-positions in one alpha-helix of the Alphabody, or
(iii) by positions in a linker fragment connecting two consecutive alpha-helices of the Alphabody.

4. The Alphabody polypeptide according to any one of claims 1 to 3, which specifically binds to a cytokine or growth factor.

5. The Alphabody polypeptide according to claim 4, which specifically binds to the receptor binding site on said cytokine or growth factor.

6. Alphabody polypeptide according to any one of claims 1 to 3, which specifically binds to a cytokine receptor or growth factor receptor.

7. The Alphabody polypeptide according to claim 6, wherein said Alphabody polypeptide specifically binds to the cytokine or growth factor binding site on said cytokine receptor or growth factor receptor.

8. The Alphabody polypeptide according to any of claims 1 to 6, wherein said Alphabody polypeptide inhibits the interaction between said cytokine or growth factor and said cytokine receptor or growth factor receptor.

9. The Alphabody polypeptide according to any one of claims 1 to 8, wherein said Alphabody polypeptide specifically binds to said cytokine or growth factor and/or to said cytokine receptor or growth factor receptor with a dissociation constant (KD) of at least 1 micromolar.

10. The Alphabody polypeptide according to any one of claims 1, 2, 3, 4, 5, 8 or 9, wherein said cytokine or growth factor is chosen from the group consisting of cardiotrophin 1, cardiotrophin-like cytokine factor 1, cardiotrophin-like cytokine factor 1, ciliary neurotrophic factor, interleukin 11, interleukin 6 (interferon, beta 2), leukemia inhibitory factor (cholinergic differentiation factor), oncostatin M, interleukin 4, isoform 1, interleukin 13, interleukin 12A, interleukin 23, alpha subunit p19, colony stimulating factor 2, interleukin 3, interleukin 5, interleukin 2, interleukin 4 isoform 1, interleukin 7, interleukin 9, interleukin 15, preproprotein, interleukin 21, colony stimulating factor 3 isoform a, erythropoietin, thymic stromal lymphopoietin isoform 1, interleukin 1 alpha proprotein and interleukin 1 beta proprotein, interleukin 10, interleukin 19 isoform 2, interleukin 20, interleukin 22, interleukin 24 isoform 1, interleukin 28A, interleukin 28B and interleukin 29, interleukin 17, interleukin 17B and interleukin 17E isoform 1, interferon alpha 1, interferon beta 1, interferon kappa, interferon epsilon 1, interferon omega 1, interferon gamma, tumor necrosis factor ligand superfamily member 7, tumor necrosis factor ligand superfamily member 14 isoform 1 precursor, tumor necrosis factor ligand superfamily member 13 isoform alphaproprotein, tumor necrosis factor ligand superfamily, member 11 isoform 1, tumor necrosis factor alpha, tumor necrosis factor (ligand) superfamily member 9, tumor necrosis factor (ligand) superfamily member 8, tumor necrosis factor (ligand) superfamily member 4, tumor necrosis factor (ligand) superfamily member 18, tumor necrosis factor (ligand) superfamily member 13b, tumor necrosis factor (ligand) superfamily member 12 isoform 1 precursor, tumor necrosis factor (ligand) superfamily member 10, lymphotoxin-beta isoform a, lymphotoxin alpha, fas ligand, ectodysplasin A isoform EDA-A2, nerve growth factor, CD27 ligand, CD30 ligand, CD40 ligand, colony stimulating factor 1 isoform a, epidermal growth factor (beta-urogastrone), fms-related tyrosine kinase 3 ligand, hepatocyte growth factor isoform 1 preproprotein, KIT ligand isoform b, PH domain-containing protein, platelet-derived growth factor beta isoform 1 preproprotein, platelet-derived growth factor C, vascular endothelial growth factor B, vascular endothelial growth factor C preproprotein, vascular endothelial growth factor isoform a, transforming growth factor beta 3, transforming growth factor beta 2, transforming growth factor beta 1, inhibin beta C chain preproprotein, inhibin beta B subunit, inhibin beta A, growth differentiation factor 5 preproprotein, bone morphogenetic protein 7, bone morphogenetic protein 2, activin beta E, chemokine (C motif) ligand 1, chemokine (C motif) ligand 2, chemokine (C-C motif) ligand 14 isoform 1, chemokine (C-C motif) ligand 15, chemokine (C-C motif) ligand 20, chemokine (C-C motif) ligand 26, chemokine (C-C motif) ligand 3, chemokine (C-C motif) ligand 4, chemokine (C-C motif) ligand 7, chemokine (C-C motif) ligand 1, chemokine (C-C motif) ligand 11, chemokine (C-C motif) ligand 13, chemokine (C-C motif) ligand 16, chemokine (C-C motif) ligand 17, chemokine (C-C motif) ligand 19, chemokine (C-C motif) ligand 2, chemokine (C-C motif) ligand 21, chemokine (C-C motif) ligand 22, chemokine (C-C motif) ligand 23 isoform CKbeta8-1, chemokine (C-C motif) ligand 24, chemokine (C-C motif) ligand 25 isoform 1, chemokine (C-C motif) ligand 27, chemokine (C-C motif) ligand 28, chemokine (C-C motif) ligand 5, chemokine (C-C motif) ligand 8, chemokine (C-X-C motif) ligand 1, chemokine (C-X-C motif) ligand 12 (stromal cell-derived factor 1)isoform beta, chemokine (C-X-C motif) ligand 13 (B-cell chemoattractant), chemokine (C-X-C motif) ligand 16, chemokine (C-X-C motif) ligand 2, chemokine (C-X-C motif) ligand 3, chemokine (C-X-C motif) ligand 5, chemokine (C-X-C motif) ligand 6 (granulocyte chemotactic protein2), interleukin 8, pro-platelet basic protein, platelet factor (PF)4, groa, MIG, ENA-78, macrophage inflammatory protein (MIP) I a, MIP I monocyte chemoattractant protein (MCP)- 1, 1-3 09, HC 14, C 10, Regulated on Activation, Normal T- cell Expressed, Secreted (RANTES), chemokine (C-X-C motif) ligand 10, chemokine (C-X-C motif) ligand 11, chemokine (C-X-C motif) ligand 9 and chemokine (C-X3-C motif) ligand 1.

11. The Alphabody polypeptide according to claims 6 to 9, wherein said cytokine or growth factor receptor is chosen from the group consisting of type 1 interleukin receptor, erythropoietin receptor, GM-CSF receptor, G-CSF receptor, oncostatin M receptor, leukemia inhibitory factor receptor, type II interleukin receptors, interferon-alpha/beta receptor, interferon-gamma receptor, interleukin-1 receptor, CSF1, C-kit receptor, interleukin-18 receptor, CD27, CD30, CD120, CD40, lymphotoxin beta receptor, interleukin-8 receptor, CCR1, CCR5, CXCR4, CXCR7, MCAF receptor, NAP-2 receptor, CC chemokine receptors, CXC chemokine receptors, CX3C chemokine receptors, XC chemokine receptor (XCR1), TGF beta receptor 1 and TGF beta receptor 2.

12. The Alphabody polypeptide according to any one of claims 1 to 11, wherein said cytokine or growth factor is a heterodimeric cytokine or growth factor and/or wherein said cytokine or growth factor receptor is a receptor for a heterodimeric cytokine or growth factor.

13. The Alphabody polypeptide according to claim 12, wherein said heterodimeric cytokine is chosen from the group consisting of IL-12, IL-23, IL-27 and IL-35 and/or said receptor for a heterodimeric cytokine is chosen from the group consisting of IL-12 receptor, the IL-23 receptor, the IL-27 receptor and the IL-35 receptor.

14. The Alphabody polypeptide according to any one of claims 12 or 13, wherein said heterodimeric cytokine is IL-23.

15. The Alphabody polypeptide according claim 14, wherein said Alphabody polypeptide binds to the p19 subunit of IL-23.

16. The Alphabody polypeptide according to any one of claims 13 to 15, wherein said Alphabody polypeptide binds to the p19 subunit and not to the p40 subunit of IL-23.

17. The Alphabody polypeptide according to any of claims 1 to 16 having at least 80% sequence identity with at least one of the amino acid sequences corresponding to any one of SEQ ID NO: 1 to SEQ ID NO: 127 and SEQ ID NO: 134 and SEQ ID NO: 135.

18. The Alphabody polypeptide according to claim 10 or 11, wherein said Alphabody polypeptide binds to Flt3 ligand or Flt3 receptor.

19. The Alphabody polypeptide according to any of claims 1 to 18, optionally comprising, in addition to said Alphabody one or more further groups, optionally linked via one or more linkers.

20. Nucleic acid sequence encoding an Alphabody polypeptide according to any one of claims 1 to 19.

21. Pharmaceutical composition comprising at least one Alphabody polypeptide according to any of claims 1 to 19 or nucleic acid sequence according to claim 20 and optionally at least one pharmaceutically acceptable carrier.

22. A method for the production of at least one Alphabody polypeptide according to any one of claims 1 to 19, having detectable binding affinity for a cytokine or growth factor and/or for a receptor of said cytokine or growth factor, said method at least comprising the steps of:
a) producing a single-chain Alphabody library comprising at least 100 different-sequence single-chain Alphabody polypeptides, wherein said Alphabody polypeptides differ from each other in at least one of a defined set of 5 to 20 variegated amino acid residue positions, and wherein at least 70% of said variegated amino acid residue positions are located either:
(i) at heptad e-positions in a first alpha-helix of the Alphabody polypeptides and at heptad g-positions in a second alpha-helix, and optionally at heptad b-positions in said first alpha-helix of the Alphabody polypeptides and/or at heptad c-positions in said second alpha-helix of the Alphabody polypeptides, or
(ii) at heptad b-, c- and f-positions in one alpha-helix of the Alphabody polypeptides, or
(iii) at positions in a linker fragment connecting two consecutive alpha-helices of the Alphabody polypeptides, and
b) selecting from said single-chain Alphabody library at least one single-chain Alphabody polypeptide having detectable binding affinity for said cytokine or growth factor and/or for a receptor of said cytokine or growth factor.

23. An Alphabody polypeptide according to any one of claims 1 to 19, specifically binding to a cytokine or growth factor and/or a cytokine receptor or growth factor receptor for use in the prevention and/or treatment of an inflammatory and/or auto-immune disease, transplant rejection, cystic fibrosis, asthma, chronic obstructive pulmonary disease, cancer, a viral infection or common variable immunodeficiency.

## Patentansprüche

1. Alphabody-Polypeptid, umfassend einen Alphabody mit der allgemeinen Formel HRS1-L1-HRS2-L2-HRS3, wobei
- es sich bei HRS1, HRS2 und HRS3 jeweils unabhängig um eine HRS (Heptad Repeat Sequence), die aus 2 bis 7 aufeinander folgenden Heptad-Repeat-Einheiten besteht, handelt, wenigstens 50% aller Heptad-Positionen a und d von Isoleucin-Resten besetzt sind, die HRS jeweils mit einem entweder an einer a- oder d-Heptad-Position lokalisierten aliphatischen oder aromatischen Aminosäurerest beginnt und endet und HRS1, HRS2 und HRS3 zusammen eine tripelsträngige, alpha-helikale Coiled-Coil-Struktur bilden; und
- es sich bei L1 und L2 jeweils unabhängig um ein Linker-Fragment handelt, das HRS1 mit HRS2 bzw. HRS2 mit HRS3 kovalent verbindet;
wobei der Alphabody an ein Mitglied eines Cytokin-Cytokinrezeptor- oder Wachstumsfaktor-Wachstumsfaktorrezeptor-Komplexes spezifisch bindet.

2. Alphabody-Polypeptid nach Anspruch 1, das **dadurch gekennzeichnet ist, dass** die Bindungsstelle in erster Linie entweder
- auf der Helixoberfläche des Alphabody,
- in einer Furche des Alphabody, unter Beteiligung wenigstens eines e- oder g-Rests der Alphabody-Helix,
- in der Loop- oder Linker-Region des Alphabody lokalisiert ist.

3. Alphabody-Polypeptid nach Anspruch 1, wobei die Bindungsstelle in erster Linie entweder
(i) von Heptad-Positionen e in einer ersten Alpha-Helix des Alphabody und von Heptad-Positionen g in einer zweiten Alpha-Helix und gegebenenfalls von Heptad-Positionen b in der ersten Alpha-Helix des Alphabody und/oder von Heptad-Positionen c in der zweiten Alpha-Helix des Alphabody oder
(ii) von Heptad-Positionen b, c und f in einer Alpha-Helix des Alphabody oder
(iii) von Positionen in einem zwei aufeinander folgende Alpha-Helices des Alphabody verbindenden Linker-Fragment
gebildet wird.

4. Alphabody-Polypeptid nach einem der Ansprüche 1 bis 3, das an ein Cytokin oder einen Wachstumsfaktor spezifisch bindet.

5. Alphabody-Polypeptid nach Anspruch 4, das an die Rezeptorbindungsstelle auf dem Cytokin oder Wachstumsfaktor spezifisch bindet.

6. Alphabody-Polypeptid nach einem der Ansprüche 1 bis 3, das an einen Cytokinrezeptor oder Wachstumsfaktorrezeptor spezifisch bindet.

7. Alphabody-Polypeptid nach Anspruch 6, wobei das Alphabody-Polypeptid an die Cytokin- oder Wachstumsfaktorbindungsstelle auf dem Cytokinrezeptor oder Wachstumsfaktorrezeptor spezifisch bindet.

8. Alphabody-Polypeptid nach einem der Ansprüche 1 bis 6, wobei das Alphabody-Polypeptid die Wechselwirkung zwischen dem Cytokin oder Wachstumsfaktor und dem Cytokinrezeptor oder Wachstumsfaktorrezeptor hemmt.

9. Alphabody-Polypeptid nach einem der Ansprüche 1 bis 8, wobei das Alphabody-Polypeptid an das Cytokin bzw. den Wachstumsfaktor und/oder an den Cytokinrezeptor oder Wachstumsfaktorrezeptor mit einer Dissoziationskonstante (KD) von wenigstens 1 mikromolar spezifisch bindet.

10. Alphabody-Polypeptid nach einem der Ansprüche 1, 2, 3, 4, 5, 8 oder 9, wobei das Cytokin bzw. der Wachstumsfaktor aus der aus Cardiotrophin 1, CLCF1 (Cardiotrophin-Like Cytokin Factor 1), Cardiotrophin-Like Cytokine Factor 1, CNTF (Ciliary Neurotrophic Factor), Interleukin 11, Interleukin 6 (Interferon, beta 2), LIF/CDF (Leukemia Inhibitory Factor/Cholinergic Differentiation Factor), Onkostatin M, Interleukin 4, Isoform 1, Interleukin 13, Interleukin 12A, Interleukin 23, Alpha-Untereinheit p19, CSF2 (Colony Stimulating Factor 2), Interleukin 3, Interleukin 5, Interleukin 2, Interleukin-4-Isoform 1, Interleukin 7, Interleukin 9, Interleukin 15, Präproprotein, Interleukin 21, CSF3(Colony Stimulating Factor 3)-Isoform a, Erythropoietin, TSLP(Thymic Stromal Lymphopoietin)-Isoform 1, Interleukin-1-alpha-Proprotein und Interleukin-1-beta-Proprotein, Interleukin 10, Interleukin-19-Isoform 2, Interleukin 20, Interleukin 22, Interleukin-24-Isoform 1, Interleukin 28A, Interleukin 28B und Interleukin 29, Interleukin 17, Interleukin 17B und Interleukin-17E-Isoform 1, Interferon-alpha 1, Interferon-beta 1, Interferon-kappa, Interferonepsilon 1, Interferon-omega 1, Interferon-gamma, TNF(Tumor Necrosis Factor)-Ligand-Superfamilie-Mitglied 7, TNF-Ligand-Superfamilie-Mitglied-14-Isoform-1-Vorstufe, TNF-Ligand-Superfamilie-Mitglied-13-Isoform-Alphaproprotein, TNF-Ligand-Superfamilie, Mitglied-11-Isoform 1, TNF-alpha, TNF-(Ligand)-Superfamilie-Mitglied 9, TNF-(Ligand)-Superfamilie-Mitglied 8, TNF-(Ligand)-Superfamilie-Mitglied 4, TNF-(Ligand)-Superfamilie-Mitglied 18, TNF-(Ligand)-Superfamilie-Mitglied 13b, TNF-(Ligand)-Superfamilie-Mitglied-12-Isoform-1-Vorstufe, TNF-(Ligand)-Superfamilie-Mitglied 10, Lymphotoxin-beta-Isoform a, Lymphotoxin-alpha, fas-Ligand, Ectodysplasin-A-Isoform EDA-A2, NGF (Nerve Growth Factor), CD27-Ligand, CD30-Ligand, CD40-Ligand, CSF(Colony Stimulating Factor)-1-Isoform a, EGF (Epidermal Growth Factor) (Beta-Urogastron), fms-Verwandte-Tyrosinkinase-3-Ligand, HGF(Hepatocyte Growth Factor)-Isoform-1-Präproprotein, KIT-Ligand-Isoform b, PH-Domäne enthaltendem Protein, PDGF(Platelet-Derived Growth Factor)-beta-Isoform-1-Präproprotein, PDGF C, VEGF (Vascular Endothelial Growth Factor) B, VEGF-C-Präproprotein, VEGF-Isoform a, TGF(Transforming Growth Factor)-beta 3, TGF-beta 2, TGF-beta 1 , Inhibin-beta-C-Kette-Präproprotein, Inhibin-beta-B-Untereinheit, Inhibin-beta A, GDF(Growth Differentiation Factor)-5-Präproprotein, BMP (Bone Morphogenetic Protein) 7, BMP 2, Anti-Müller-Hormon, Activin-beta E, Chemokin-(C-Motiv)-Ligand 1 , Chemokin-(C-Motiv)-Ligand 2, Chemokin-(C-C-Motiv)-Ligand-14-Isoform 1 , Chemokin-(C-C-Motiv)-Ligand 15, Chemokin-(C-C-Motiv)-Ligand 20, Chemokin-(C-C-Motiv)-Ligand 26, Chemokin-(C-C-Motiv)-Ligand 3, Chemokin-(C-C-Motiv)-Ligand 4, Chemokin-(C-C-Motiv)-Ligand 7, Chemokin-(C-C-Motiv)-Ligand 1, Chemokin-(C-C-Motiv)-Ligand 11, Chemokin-(C-C-Motiv)-Ligand 13, Chemokin-(C-C-Motiv)-Ligand 16, Chemokin-(C-C-Motiv)-Ligand 17, Chemokin-(C-C-Motiv)-Ligand 19, Chemokin-(C-C-Motiv)-Ligand 2, Chemokin-(C-C-Motiv)-Ligand 21, Chemokin-(C-C-Motiv)-Ligand 22, Chemokin-(C-C-Motiv)-Ligand-23-Isoform CKbeta8-1, Chemokin-(C-C-Motiv)-Ligand 24, Chemokin-(C-C-Motiv)-Ligand-25-Isoform 1, Chemokin-(C-C-Motiv)-Ligand 27, Chemokin-(C-C-Motiv)-Ligand 28, Chemokin-(C-C-Motiv)-Ligand 5, Chemokin-(C-C-Motiv)-Ligand 8, Chemokin-(C-X-C-Motiv)-Ligand 1, Chemokin-(C-X-C-Motiv)-Ligand-12(SDF-1 (Stromal Cell-Derived Factor 1))-Isoform beta, Chemokin-(C-X-C-Motiv)-Ligand 13 (BLC (B-Cell Chemoattractant)), Chemokin-(C-X-C-Motiv)-Ligand 16, Chemokin-(C-X-C-Motiv)-Ligand 2, Chemokin-(C-X-C-Motiv)-Ligand 3, Chemokin-(C-X-C-Motiv)-Ligand 5, Chemokin-(C-X-C-Motiv)-Ligand 6 (GCP-2 (Granulocyte Chemotactic Protein2)), Interleukin 8, PPBP (Pro-Platelet Basic Protein), PF(Platelet Factor)4, groa, MIG, ENA-78, MIP (Macrophage Inflammatory Protein) I a, MIP I MCP(Monocyte Chemoattractant Protein)- 1, 1-3 09, HC 14, C 10, RANTES (Regulated on Activation, Normal T-cell Expressed, Secreted), Chemokin-(C-X-C-Motiv)-Ligand 10, Chemokin-(C-X-C-Motiv)-Ligand 11, Chemokin-(C-X-C-Motiv)-Ligand 9 und Chemokin-(C-X3-C-Motiv)-Ligand 1 bestehenden Gruppe gewählt wird.

11. Alphabody-Polypeptid nach Anspruch 6 bis 9, wobei der Cytokin- oder Wachstumsfaktorrezeptor aus der aus Typ-1-Interleukin-Rezeptor, Erythropoietin-Rezeptor, GM-CSF-Rezeptor, G-CSF-Rezeptor, Oncostatin-M-Rezeptor, LIF(Leukemia Inhibitory Factor)-Rezeptor, Type-II-Interleukin-Rezeptoren, Interferon-alpha/beta-Rezeptor, Interferon-gamma-Rezeptor, Interleukin-1-Rezeptor, CSF1, C-kit-Rezeptor, Interleukin-18-Rezeptor, CD27, CD30, CD120, CD40, Lymphotoxin-beta-Rezeptor, Interleukin-8-Rezeptor, CCR1, CCR5, CXCR4, CXCR7, MCAF-Rezeptor, NAP-2-Rezeptor, CC-Chemokin-Rezeptoren, CXC-Chemokin-Rezeptoren, CX3C-Chemokin-Rezeptoren, XC-Chemokin-Rezeptor (XCR1), TGF-beta-Rezeptor 1 und TGF-beta-Rezeptor 2 bestehenden Gruppe gewählt wird.

12. Alphabody-Polypeptid nach einem der Ansprüche 1 bis 11, wobei es sich bei dem Cytokin oder Wachstumsfaktor um ein heterodimeres Cytokin bzw. einen heterodimeren Wachstumsfaktor handelt und/oder wobei es sich bei dem Cytokin- oder Wachstumsfaktorrezeptor um einen Rezeptor für ein heterodimeres Cytokin bzw. einen heterodimeren Wachstumsfaktor handelt.

13. Alphabody-Polypeptid nach Anspruch 12, wobei das heterodimere Cytokin aus der aus IL-12, IL-23, IL-27 und IL-35 bestehenden Gruppe gewählt wird und/oder der Rezeptor für ein heterodimeres Cytokin aus der aus IL-12-Rezeptor, dem IL-23-Rezeptor, dem IL-27-Rezeptor und dem IL-35-Rezeptor bestehenden Gruppe gewählt wird.

14. Alphabody-Polypeptid nach einem der Ansprüche 12 oder 13, wobei es sich bei dem heterodimeren Cytokin um IL-23 handelt.

15. Alphabody-Polypeptid nach Anspruch 14, wobei das Alphabody-Polypeptid an die p19-Untereinheit von IL-23 bindet.

16. Alphabody-Polypeptid nach einem der Ansprüche 13 bis 15, wobei das Alphabody-Polypeptid an die p19-Untereinheit und nicht an die p40-Untereinheit von IL-23 bindet.

17. Alphabody-Polypeptid nach einem der Ansprüche 1 bis 16 mit einer Sequenzidentität von wenigstens 80% mit wenigstens einer der einer von SEQ ID NO: 1 bis SEQ ID NO: 127 und SEQ ID NO: 134 und SEQ ID NO: 135 entsprechenden Aminosäuresequenzen.

18. Alphabody-Polypeptid nach Anspruch 10 oder 11, wobei das Alphabody-Polypeptid an Flt3-Ligand oder Flt3-Rezeptor bindet.

19. Alphabody-Polypeptid nach einem der Ansprüche 1 bis 18, gegebenenfalls umfassend neben dem Alphabody eine oder mehrere weitere Gruppen, gegebenenfalls verknüpft über einen oder mehrere Linker.

20. Nukleinsäuresequenz, codierend ein Alphabody-Polypeptid nach einem der Ansprüche 1 bis 19.

21. Pharmazeutische Zusammensetzung, umfassend wenigstens ein Alphabody-Polypeptid nach einem der Ansprüche 1 bis 19 oder wenigstens eine Nukleinsäuresequenz nach Anspruch 20 und gegebenenfalls wenigstens einen pharmazeutisch unbedenklichen Trägerstoff.

22. Verfahren zur Herstellung wenigstens eines Alphabody-Polypeptids nach einem der Ansprüche 1 bis 19 mit nachweisbarer Bindungsaffinität für ein Cytokin oder einen Wachstumsfaktor und/oder für einen Rezeptor des Cytokins bzw. Wachstumsfaktors, wobei das Verfahren wenigstens die folgenden Schritte umfasst:
a) Herstellen einer Einzelkette-Alphabody-Bibliothek, die wenigstens 100 Einzelkette-Alphabody-Polypeptide mit unterschiedlicher Sequenz umfasst, wobei sich die Alphabody-Polypeptide voneinander in wenigstens einer von einem definierten Satz von 5 bis 20 variegierten Aminosäurerestpositionen unterscheiden und wobei wenigstens 70% der variegierten Aminosäurerestpositionen entweder
(i) an Heptad-Positionen e in einer ersten Alpha-Helix der Alphabody-Polypeptide und an Heptad-Positionen g in einer zweiten Alpha-Helix und gegebenenfalls an Heptad-Positionen b in der ersten Alpha-Helix der Alphabody-Polypeptide und/oder an Heptad-Positionen c in der zweiten Alpha-Helix der Alphabody-Polypeptide oder
(ii) an Heptad-Positionen b, c und f in einer Alpha-Helix der Alphabody-Polypeptide oder
(iii) an Positionen in einem zwei aufeinander folgende Alpha-Helices der Alphabody-Polypeptide verbindenden Linker-Fragment lokalisiert sind, und
b) Auswählen wenigstens eines Einzelkette-Alphabody-Polypeptids mit nachweisbarer Bindungsaffinität für das Cytokin bzw. den Wachstumsfaktor und/oder für einen Rezeptor des Cytokins bzw. Wachstumsfaktors aus der Einzelkette-Alphabody-Bibliothek.

23. Alphabody-Polypeptid nach einem der Ansprüche 1 bis 19 mit spezifischer Bindung an ein Cytokin oder einen Wachstumsfaktor und/oder einen Cytokinrezeptor oder Wachstumsfaktorrezeptor zur Verwendung bei der Vorbeugung und/oder Behandlung einer Entzündungs- und/oder Autoimmunkrankheit, von Transplantatabstoßung, zystischer Fibrose, Asthma, chronisch obstruktiver Lungenerkrankung, Krebs, einer Virusinfektion oder von variablem Immundefektsyndrom.

## Revendications

1. Polypeptide d'Alphabody comprenant un Alphabody ayant la formule générale HRS1-L1-HRS2-L2-HRS3, dans lequel
- chacun de HRS1, HRS2 et HRS3 est indépendamment une séquence de répétition d'heptade (HRS) constituée de 2 à 7 motifs de répétition d'heptade consécutifs, au moins 50 % de toutes les positions a et d d'heptade sont occupées par des résidus isoleucine, chaque HRS commence et termine par un résidu d'acide aminé aliphatique ou aromatique situé à une position a ou d d'heptade, et HRS1, HRS2 et HRS3 forment conjointement une structure de superhélice, de type hélice alpha, tricaténaire ; et
- chacun de L1 et L2 est indépendamment un fragment de lieur, qui relie de façon covalente HRS1 à HRS2 et HRS2 à HRS3, respectivement ;
ledit Alphabody se liant spécifiquement à un membre d'un complexe cytokine - récepteur de cytokine ou facteur de croissance - récepteur de facteur de croissance.

2. Polypeptide d'Alphabody selon la revendication 1, qui est **caractérisé en ce que** le site de liaison est principalement situé
- sur la surface d'hélice de l'Alphabody,
- dans une fente de l'Alphabody, en impliquant au moins un résidu e ou g de l'hélice de l'Alphabody
- dans la région de boucle ou de lieur de l'Alphabody.

3. Polypeptide d'Alphabody selon la revendication 1, dans lequel le site de liaison est principalement formé :
(i) par des positions d'heptade e dans une première hélice alpha de l'Alphabody et par des positions d'heptade g dans une deuxième hélice alpha, et facultativement par des positions d'heptade b dans ladite première hélice alpha de l'Alphabody et/ou par des positions d'heptade c dans ladite deuxième hélice alpha de l'Alphabody, ou
(ii) par des positions d'heptade b, c et f dans une hélice alpha de l'Alphabody, ou
(iii) par des positions dans un fragment de lieur reliant deux hélices alpha consécutives de l'Alphabody.

4. Polypeptide d'Alphabody selon l'une quelconque des revendications 1 à 3, qui se lie spécifiquement à une cytokine ou un facteur de croissance.

5. Polypeptide d'Alphabody selon la revendication 4, qui se lie spécifiquement aux sites de liaison de récepteurs sur ladite cytokine ou ledit facteur de croissance.

6. Polypeptide d'Alphabody selon l'une quelconque des revendications 1 à 3, qui se lie spécifiquement à un récepteur de cytokine ou un récepteur de facteur de croissance.

7. Polypeptide d'Alphabody selon la revendication 6, **caractérisé en ce que** ledit polypeptide d'Alphabody se lie spécifiquement aux sites de liaison de cytokine ou de facteur de croissance sur ledit récepteur de cytokine ou récepteur de facteur de croissance.

8. Polypeptide d'Alphabody selon l'une quelconque des revendications 1 à 6, **caractérisé en ce que** ledit polypeptide d'Alphabody inhibe l'interaction entre ladite cytokine ou ledit facteur de croissance et ledit récepteur de cytokine ou récepteur de facteur de croissance.

9. Polypeptide d'Alphabody selon l'une quelconque des revendications 1 à 8, **caractérisé en ce que** ledit polypeptide d'Alphabody se lie spécifiquement à ladite cytokine ou audit facteur de croissance et/ou audit récepteur de cytokine ou récepteur de facteur de croissance avec une constante de dissociation (KD) d'au moins 1 micromolaire.

10. Polypeptide d'Alphabody selon l'une quelconque des revendications 1, 2, 3, 4, 5, 8 ou 9, **caractérisé en ce que** ladite cytokine ou ledit facteur de croissance est choisi dans le groupe constitué des cardiotrophine 1, facteur de cytokine de type cardiotrophine 1, facteur de cytokine de type cardiotrophine 1, facteur neurotrophique ciliaire, interleukine 11, interleukine 6 (interféron, bêta 2), facteur inhibiteur de leucémie (facteur de différenciation cholinergique), oncostatine M, isoforme 1 d'interleukine 4, interleukine 13, interleukine 12A, sous-unité alpha p19 d'interleukine 23, facteur stimulant les colonies 2, interleukine 3, interleukine 5, interleukine 2, isoforme 1 d'interleukine 4, interleukine 7, interleukine 9, préproprotéine d'interleukine 15, interleukine 21, isoforme a de facteur stimulant les colonies 3, érythropoïétine, isoforme 1 de lymphopoïétine stromale thymique, proprotéine alpha d'interleukine 1 et proprotéine bêta d'interleukine 1, interleukine 10, isoforme 2 d'interleukine 19, interleukine 20, interleukine 22, isoforme 1 d'interleukine 24, interleukine 28A, interleukine 28B et interleukine 29, interleukine 17, interleukine 17B et isoforme 1 d'interleukine 17E, interféron alpha 1, interféron bêta 1, interféron kappa, interféron epsilon 1, interféron oméga 1, interféron gamma, membre 7 de la superfamille des ligands du facteur de nécrose tumorale, précurseur d'isoforme 1 du membre 14 de la superfamille des ligands du facteur de nécrose tumorale, alphaproprotéine d'isoforme du membre 13 de la superfamille des ligands du facteur de nécrose tumorale, isoforme 1 du membre 11 de la superfamille des ligands du facteur de nécrose tumorale, facteur de nécrose tumorale alpha, membre 9 de la superfamille (des ligands) du facteur de nécrose tumorale, membre 8 de la superfamille (des ligands) du facteur de nécrose tumorale, membre 4 de la superfamille (des ligands) du facteur de nécrose tumorale, membre 18 de la superfamille (des ligands) du facteur de nécrose tumorale, membre 13b de la superfamille (des ligands) du facteur de nécrose tumorale, précurseur d'isoforme 1 du membre 12 de la superfamille (des ligands) du facteur de nécrose tumorale, membre 10 de la superfamille (des ligands) du facteur de nécrose tumorale, isoforme a de lymphotoxine-bêta, lymphotoxine alpha, ligand de fas, isoforme EDA-A2 d'ectodysplasine A, facteur de croissance nerveuse, ligand CD27, ligand de CD30, ligand de CD40, isoforme a de facteur stimulant les colonies 1, facteur de croissance épidermique (bêta-urogastrone), ligand de tyrosine kinase 3 fms-like, préproprotéine d'isoforme 1 de facteur de croissance des hépatocytes, isoforme b de ligand de KIT, protéine contenant un domaine PH, préproprotéine d'isoforme 1 de facteur de croissance dérivé des plaquettes bêta, facteur de croissance dérivé des plaquettes C, facteur de croissance endothéliale vasculaire B, préproprotéine de facteur de croissance endothéliale vasculaire C, isoforme a de facteur de croissance endothéliale vasculaire, facteur de croissance transformant bêta 3, facteur de croissance transformant bêta 2, facteur de croissance transformant bêta 1, préproprotéine de chaîne C d'inhibine bêta, sous-unité B d'inhibine bêta, inhibine bêta A, préproprotéine de facteur de différenciation de croissance 5, protéine morphogénétique osseuse 7, protéine morphogénétique osseuse 2, activine bêta E, ligand de chimiokine 1 (motif C), ligand de chimiokine 2 (motif C), isoforme 1 de ligand de chimiokine 14 (motif C-C), ligand de chimiokine 15 (motif C-C), ligand de chimiokine 20 (motif C-C), ligand de chimiokine 26 (motif C-C), ligand de chimiokine 3 (motif C-C), ligand de chimiokine 4 (motif C-C), ligand de chimiokine 7 (motif C-C), ligand de chimiokine 1 (motif C-C), ligand de chimiokine 11 (motif C-C), ligand de chimiokine 13 (motif C-C), ligand de chimiokine 16 (motif C-C), ligand de chimiokine 17 (motif C-C), ligand de chimiokine 19 (motif C-C), ligand de chimiokine 2 (motif C-C), ligand de chimiokine 21 (motif C-C), ligand de chimiokine 22 (motif C-C), isoforme CKbêta8-1 de ligand de chimiokine 23 (motif C-C), ligand de chimiokine 24 (motif C-C), isoforme 1 de ligand de chimiokine 25 (motif C-C), ligand de chimiokine 27 (motif C-C), ligand de chimiokine 28 (motif C-C), ligand de chimiokine 5 (motif C-C), ligand de chimiokine 8 (motif C-C), ligand de chimiokine 1 (motif C-X-C), isoforme bêta de ligand de chimiokine 12 (motif C-X-C) (facteur dérivé des cellules stromales 1), ligand de chimiokine 13 (motif C-X-C) (agent chimioattractif des lymphocytes B), ligand de chimiokine 16 (motif C-X-C), ligand de chimiokine 2 (motif C-X-C), ligand de chimiokine 3 (motif C-X-C), ligand de chimiokine 5 (motif C-X-C), ligand de chimiokine 6 (motif C-X-C) (protéine chimiotactique des granulocytes 2), interleukine 8, pro-protéine basique plaquettaire, facteur plaquettaire (PF)4, groa, MIG, ENA-78, protéine inflammatoire des macrophages (MIP) I a, protéine chimioattractive des monocytes MIP I (MCP)-1, 1-3 09, HC 14, C 10, régulé par activation, exprimé et sécrété par les lymphocytes T normaux (RANTES), ligand de chimiokine 10 (motif C-X-C), ligand de chimiokine 11 (motif C-X-C), ligand 9 de chimiokine (motif C-X-C) et ligand 1 de chimiokine (motif C-X3-C).

11. Polypeptide d'Alphabody selon les revendications 6 à 9, **caractérisé en ce que** ladite cytokine ou ledit récepteur de facteur de croissance est choisi dans le groupe constitué des récepteur d'interleukine type 1, récepteur d'érythropoiétine, récepteur de GM-CSF, récepteur de G-CSF, récepteur d'oncostatine M, récepteur de facteur inhibiteur de leucémie, récepteurs d'interleukine de type II, récepteur d'interféron-alpha/bêta, récepteur d'interféron-gamma, récepteur d'interleukine-1, CSF1, récepteur de C-kit, récepteur d'interleukine-18, CD27, CD30, CD120, CD40, récepteur de lymphotoxine bêta, récepteur d'interleukine-8, CCR1, CCR5, CXCR4, CXCR7, récepteur de MCAF, récepteur de NAP-2, récepteurs de chimiokine CC, récepteurs de chimiokine CXC, récepteurs de chimiokine CX3C, récepteur de chimiokine XC (XCR1), récepteur 1 de TGF-bêta et récepteur 2 de TGF-bêta.

12. Polypeptide d'Alphabody selon l'une quelconque des revendications 1 à 11, **caractérisé en ce que** ladite cytokine ou ledit facteur de croissance est une cytokine ou un facteur de croissance hétérodimère et/ou ladite cytokine ou ledit récepteur de facteur de croissance est un récepteur pour une cytokine ou un facteur de croissance hétérodimère.

13. Polypeptide d'Alphabody selon la revendication 12, **caractérisé en ce que** ladite cytokine hétérodimère est choisie dans le groupe constitué de IL-12, IL-23, IL-27 et IL-35 et/ou ledit récepteur pour une cytokine hétérodimère est choisi dans le groupe constitué des récepteur de IL-12, récepteur de IL-23, récepteur de IL-27 et récepteur de IL-35.

14. Polypeptide d'Alphabody selon l'une quelconque des revendications 12 ou 13, **caractérisé en ce que** ladite cytokine hétérodimère est IL-23.

15. Polypeptide d'Alphabody selon la revendication 14, **caractérisé en ce que** ledit polypeptide d'Alphabody se lie à la sous-unité p19 de IL-23.

16. Polypeptide d'Alphabody selon l'une quelconque des revendications 13 à 15, **caractérisé en ce que** ledit polypeptide d'Alphabody se lie à la sous-unité p19 et non à la sous-unité p40 de IL-23.

17. Polypeptide d'Alphabody selon l'une quelconque des revendications 1 à 16 ayant au moins 80 % d'identité de séquence avec au moins une des séquences d'acides aminés correspondant à l'un quelconque de SEQ ID NO: 1 to SEQ ID NO: 127 et SEQ ID NO: 134 et SEQ ID NO: 135.

18. Polypeptide d'Alphabody selon la revendication 10 ou 11, **caractérisé en ce que** ledit polypeptide d'Alphabody se lie au ligand de Flt3 ou au récepteur de Flt3.

19. Polypeptide d'Alphabody selon l'une quelconque des revendications 1 à 18, comprenant facultativement, en plus dudit Alphabody, un ou plusieurs groupes supplémentaires, facultativement liés par l'intermédiaire d'un ou plusieurs lieurs.

20. Séquence d'acide nucléique codant pour un polypeptide d'Alphabody selon l'une quelconque des revendications 1 à 19.

21. Composition pharmaceutique comprenant au moins un polypeptide d'Alphabody selon l'une quelconque des revendications 1 à 19 ou une séquence d'acide nucléique selon la revendication 20 et facultativement au moins un véhicule pharmaceutiquement acceptable.

22. Procédé de production d'au moins un polypeptide d'Alphabody selon l'une quelconque des revendications 1 à 19, ayant une affinité de liaison détectable pour une cytokine ou un facteur de croissance et/ou pour un récepteur de ladite cytokine ou dudit facteur de croissance, ledit procédé comprenant au moins les étapes de :
a) production d'une banque d'Alphabody monocaténaires comprenant au moins 100 polypeptides d'Alphabody monocaténaires de séquences différentes, lesdits polypeptides d'Alphabody différant les uns des autres à au moins une d'un ensemble défini de 5 à 20 positions de résidu d'acide aminé variées, et au moins 70 % desdites positions de résidu d'acide aminé variées étant situées soit :
(i) aux positions d'heptade e dans une première hélice alpha des polypeptides d'Alphabody et aux positions d'heptade g dans une deuxième hélice alpha, et facultativement aux positions d'heptade b dans ladite première hélice alpha des polypeptides d'Alphabody et/ou aux positions d'heptade c dans ladite deuxième hélice alpha des polypeptides d'Alphabody, ou
(ii) aux positions d'heptade b, c et f dans une hélice alpha des polypeptides d'Alphabody, ou
(iii) aux positions dans un fragment de lieur reliant deux hélices alpha consécutives des polypeptides d'Alphabody, et
b) sélection dans ladite banque d'Alphabody monocaténaires d'au moins un polypeptide d'Alphabody monocaténaire ayant une affinité de liaison détectable pour ladite cytokine ou ledit facteur de croissance et/ou pour un récepteur de ladite cytokine ou dudit facteur de croissance.

23. Polypeptide d'Alphabody selon l'une quelconque des revendications 1 à 19, se liant spécifiquement à une cytokine ou un facteur de croissance et/ou un récepteur de cytokine ou un récepteur de facteur de croissance pour utilisation dans la prévention et/ou le traitement d'une maladie inflammatoire et/ou auto-immune, un rejet de greffe, la mucoviscidose, l'asthme, la bronchopneumopathie chronique obstructive, le cancer, une infection virale ou une immunodéficience variable commune.
